(19) Europäisches Patentamt
European Patent Office
Office européen des brevets

(11) Publication number: **0 640 594 A1**

# (12) EUROPEAN PATENT APPLICATION

(21) Application number: **94113137.7**

(22) Date of filing: **23.08.94**

(51) Int. Cl.6: **C07D 233/72**, A61K 31/415, A61K 31/505, A61K 31/41, C07D 233/76, C07D 233/78, C07D 233/80, C07D 233/90, C07D 235/02, C07D 249/12, C07D 239/22, C07D 403/06

(30) Priority: **23.08.93 JP 227834/93**

(43) Date of publication of application:
**01.03.95 Bulletin 95/09**

(84) Designated Contracting States:
**AT BE CH DE DK ES FR GB GR IE IT LI LU MC NL PT SE**

(71) Applicant: **FUJIREBIO INC.**
**Shinjuku Daiichi Seimei Building,**
**7-1, Nishi-Shinjuku 2-chome**
**Shinjuku-ku,**
**Tokyo 163 (JP)**

(72) Inventor: **Kobayashi, Nobuo, c/o Fujirebio Inc.**
**Shinjuku**
**Daiichi Seimei B.,**
**7-1, Nishi-Shinjuku 2-chome**
**Shinjuku-ku,**
**Tokyo 163 (JP)**
Inventor: **Nakayama, Yukihide, c/o Fujirebio Inc., Shinjuku**
**Daiichi Seimei Bldg.,**
**7-1, Nishi-Shinjuku2-chome**
**Shinjuku-ku,**
**Tokyo 163 (JP)**
Inventor: **Kojima, Kazuhiro, c/o Fujirebio Inc.**
**Shinjuku**
**Daiichi Seimei B.,**
**7-1, Nishi-Shinjuku 2-chome**
**Shinjuku-ku,**
**Tokyo 163 (JP)**
Inventor: **Yamaura, Tetsuaki, c/o Fujirebio Inc.**
**Shinjuku**
**Daiichi Seimei B.,**
**7-1, Nishi-Shinjuku 2-chome**
**Shinjuku-ku,**
**Tokyo 163 (JP)**
Inventor: **Ikawa, Hiroshi, c/o Fujirebio Inc.**
**Shinjuku**
**Daiichi Seimei B.,**
**7-1, Nishi-Shinjuku 2-chome**
**Shinjuku-ku,**
**Tokyo 163 (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Hydantoin derivative as metalloprotease inhibitor.**

(57) A hydantoin derivative represented by formula (I):

EP 0 640 594 A1

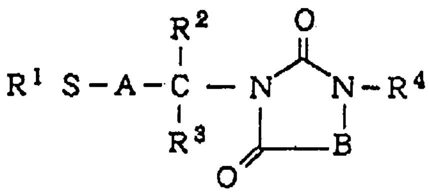

(I)

wherein the all symbols are defined in the disclosure. The hydantoin derivative has an inhibitory activity on metalloprotease and hence is useful as analgesic and cardiovascular drug.

## FIELD OF THE INVENTION

The present invention relates to a novel hydantoin derivative having an inhibitory activity on metalloprotease.

## BACKGROUND OF THE INVENTION

In general, a series of proteases of which a metal such as Zn, Ca or Co plays an important role in expression of their enzymatic activities or maintenance of their structures are classified as metalloprotease.

There are various kinds of metalloproteases in mammalia and each of them plays its respective important role. For example, carboxypeptidase A (which takes part in digestion and absorption of dietary protein in mammals), angiotensin converting enzyme (ACE; EC 3.4.15.1) (which takes part in the biosynthesis of angiotensin II which is a potent vasoconstrictor), neutral metalloendopeptidase (NEP; EC 3.4.24.11) (which takes part in the metabolic degradation of *in vivo* peripheral hormones or neuropeptides) and collagenase (which takes part in the metabolic degradation of collagen which is an important constituent of cartilaginous matrix) are known as a metalloprotease. In addition, it has recently been revealed that the endothelin converting enzyme (ECE) (which takes part in the biosynthesis of endothelin I which is a potent vasoconstrictor) is also one kind of metalloproteases.

As described above, it is considered that metalloprotease contributes to the biosynthesis and metabolic degradation of various hormones, neurotransmitters and the like which posses a potent physiological functions, and hence plays an important role to keep homeostasis in mammalia.

Accordingly, there is a possibility that inhibitors of these metalloproteases are useful as treating drugs for diseases induced by anomalies of hormones or neurotransmitters.

Among these metalloproteases, NEP is an enzyme which is widely distributed in mammalia and known that it metabolically degrades circulatory peptide hormones such as atrial natriuretic peptide (ANP) and endogenous opioid peptides (e.g., endorphins, enkephalins), thereby inactivating these peptide hormones.

Endorphins and enkephalins are polypeptides and pentapeptides, respectively, and both of them bind with the opioid receptor in the brain, thereby raising the threshold of pain and expressing an analgesic effect. ANP is a peptide composed of 28 amino acids which is excreted from the atrium. Its physiological action includes vasodilation, natriuresis, and diuretic action, which are important for the physiological control of blood pressure, body fluid, and electrolytes.

Consequently, it is expected that an inhibitor of NEP is useful as a potential drug to sustain and enhance the analgesic effect of endogenous opioid peptides and as a treating drug for a cardiovascular disease (circulatory diseases) (such as hypertension and heart failure) due to its ability to sustain and enhance the natriuresis and diuretic action possessed by ANP.

Like NEP, ACE is also a membrane-bound metalloprotease which is widely present in the lung, kidney, and brain. It is known to take part in the biosynthesis of angiotensin II (which is a potent vasoconstrictor) through the cleavage of the C terminal dipeptide (His-Leu) of angiotensin I.

Angiotensin II directly constricts blood vessels to increases the blood pressure and also promotes the secretion of aldosterone and vasopressin (which influence sodium and water absorption) to increase the amount of body fluid.

Therefore, inhibitors of ACE are useful as an effective treating drug for various diseases due to the anomalous level of angiotensin II (such as hypertension and congestive heart failure).

A dipeptide-mimic derivative, which has a similarity with the substrate of metalloprotease and has a functional groups capable of binding to the metal and base moiety of the enzyme, has been known as the inhibitor on metalloprotease. (For example, the NEP inhibitors are described in Life Sci. 31, 2947-2954 (1982), Eur. J. Biochem. 139, 267-274 (1984), J. Med. Chem. 28, 1158-1169 (1985), J. Med. Chem. 29, 751-757 (1986), JP-A-56-158746, JP-A-62-270555, and JP-A-2-209861 (the term "JP-A" as used herein means "unexamined published Japanese Patent Application"); the ACE inhibitors are described in J. Med. Chem. 24, 355-361 (1981), J. Med. Chem. 25, 250-258 (1982), J. Med. Chem. 26, 1267-1277 (1983), and JP-A-55-9060, JP-A-55-31022, JP-A-55-147257, JP-A-55-27199, JP-A-57-88165, and JP-A-57-163390.)

## SUMMARY OF THE INVENTION

In an effort to develop a new metalloprotease inhibitor differing from the conventional peptide-mimic derivative, the present inventors found a metalloprotease inhibitor of new type having the hydantoin skeleton, thus completing the present invention.

It is an object of the present invention to provide a hydantoin derivative represented by the following formula (I) which is a novel type of metalloprotease inhibitor having an inhibitory activity on NEP or ACE and is useful as a treating drug for cardiovascular diseases such as hypertension and heart failure, and an analgesic drug:

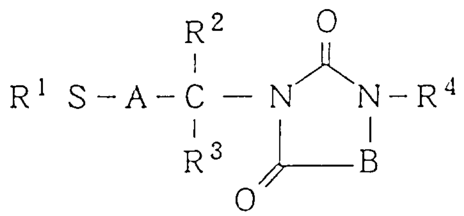

(I)

wherein A represents a $C_{1-10}$ divalent aliphatic hydrocarbon group which may be substituted by an aromatic hydrocarbon group or an aromatic heterocyclic group; B represents $-(CH_2)_n-N(R^5)-$ or $-(CH_2)_n-CR^6-(R^7)-$ (in which $R^5$, $R^6$, and $R^7$ each represents a hydrogen atom, an aromatic hydrocarbon group, an aromatic heterocyclic group or a $C_{1-16}$ substituted or unsubstituted monovalent aliphatic hydrocarbon group ($R^6$ and $R^7$ may be combined with each other to form a 4- to 7-membered carbon ring)); n represents 0 or 1; $R^1$ represents a hydrogen atom or a protecting group of the mercapto group; $R^2$ and $R^3$ each represents a hydrogen atom, an aromatic hydrocarbon group, an aromatic heterocyclic group, or a $C_{1-10}$ substituted or unsubstituted monovalent aliphatic hydrocarbon group ($R^2$ and $R^3$ are not hydrogen atoms at the same time and may be combined with each other to form a 4- to 7-membered carbon ring); and $R^4$ represents an aromatic hydrocarbon group, an aromatic heterocyclic group, a $-NR^8R^9$ group (wherein $R^8$ and $R^9$ each represents a hydrogen atom, an aromatic hydrocarbon group, an aromatic heterocyclic group, or a $C_{1-16}$ substituted or unsubstituted monovalent aliphatic hydrocarbon group, or $R^8$ and $R^9$ may be combined with each other to form a 4- to 7-membered ring), or a $C_{1-16}$ substituted or unsubstituted monovalent aliphatic hydrocarbon group, or a pharmaceutical acceptable salt thereof.

It is another object of the present invention to provide a treating drug for cardiovascular diseases and an analgesic drug each comprising the hydantoin derivative of formula (I) and a pharmaceutical acceptable carrier or diluent.

## DETAILED DESCRIPTION OF THE INVENTION

Since the hydantoin derivative represented by formula (I) may have a chiral carbon atoms, it may be obtained as an individual enantiomer or a mixture thereof, or an individual diastereomer or a mixture thereof. The present invention embraces these mixtures and the individual enantiomer and diastereomer.

The hydantoin derivative represented by formula (I) according to the present invention may be prepared by the reaction scheme shown below.

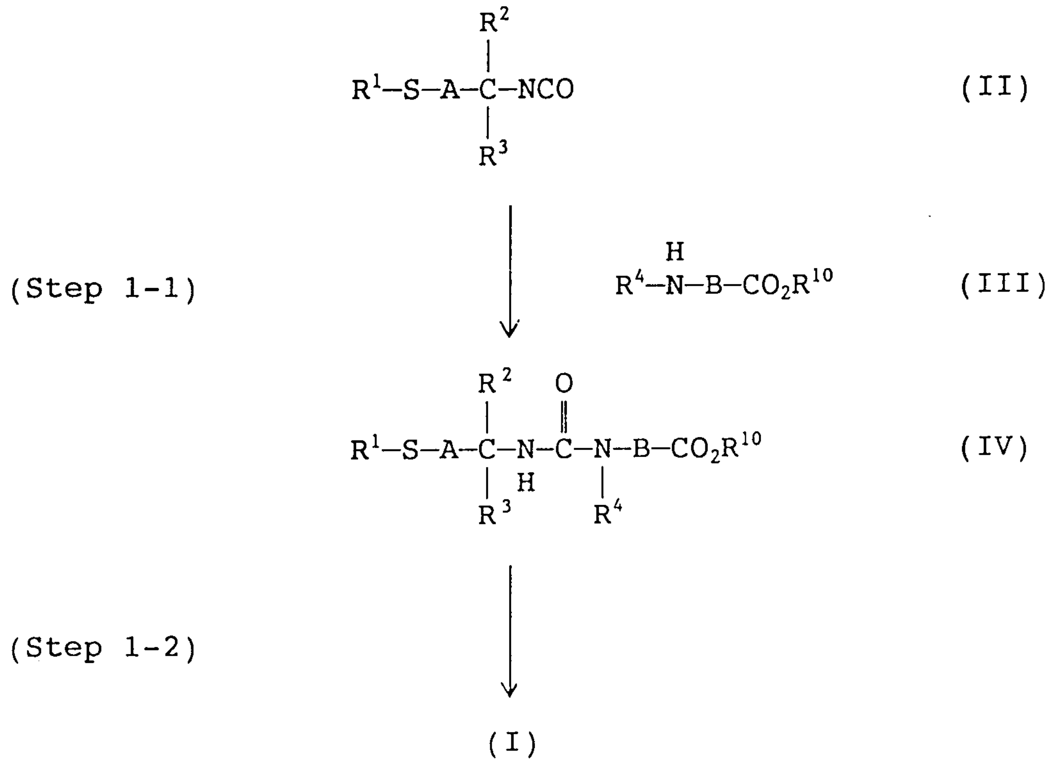

(II)
(III)
(IV)
(I)

wherein A, B, $R^1$, $R^2$, $R^3$ and $R^4$ are the same meanings as defined above, and $R^{10}$ represents a $C_{1-4}$ alkyl group (e.g., methyl group, ethyl group, butyl group, propyl group).

The term "protecting group of the mercapto group" as used herein means thioether-type, thioester-type, thiocarbonate-type, thiocarbamate-type, disulfide-type, sulfenyl-type, dithioacetal-type, or ketal-type protecting groups. Among them, the thioether-type protecting group is preferred.

Examples of the thioether-type protecting group include benzyl group, p-methoxybenzyl group, p-nitrobenzyl group, 4-picolyl group, diphenylmethyl group, bis(4-methoxyphenyl)methyl group, triphenylmethyl group, phenyl group, t-butyl group, 1-adamantyl group, methoxymethyl group, isobutoxymethyl group, tetrahydropyranyl group, benzylthiomethyl group, phenylthiomethyl group, acetoamidomethyl group, trimethylacetoamidomethyl group, benzamidomethyl group, acetylmethyl group, carboxymethyl group, cyanomethyl group, 2-nitro-1-phenylethyl group, 2-(4-pyridyl)ethyl group, 2,2-bis(carboethoxy)ethyl group and phenylsulfonylethyl group.

Examples of thioester-type protecting group include acetyl group, benzoyl group and 2,4,6-trimethylbenzoyl group.

Examples of the thiocarbonate-type protecting group include 2,2,2-trichloroethoxycarbonyl group, t-butoxycarbonyl group, benzyloxycarbonyl group and p-methoxybenzyloxycarbonyl group.

Examples of the thiocarbamate-type protecting group include ethylaminocarbonyl group, diethylaminocarbonyl group and methoxymethylaminocarbonyl group.

Examples of the disulfide-type protecting group include ethylthio group and t-butylthio group.

Examples of the sulfenyl-type protecting group include sulfonate group, benzyloxycarbonylthio group, t-butoxycarbonylthio group and 3-nitro-2-pyridinesulfenyl group.

Examples of the dithioacetal-type and ketal-type protecting groups include S,S'-methylene group, S,S'-isopropylidene group and S,S'-benzylidene group.

The term "aliphatic hydrocarbon group" as used herein means a saturated or unsaturated straight-chain or branched or cyclic aliphatic hydrocarbon group.

The divalent aliphatic hydrocarbon group represented by A in formula (I) has 1 to 10 carbon atoms, preferably from 1 to 6 carbon atoms. Specific examples thereof include methylene group, ethylene group, trimethylene group, tetramethylene group, pentamethylene group, hexamethylene group, methylmethylene group, ethylmethylene group, isopropylmethylene group, propylmethylene group, butylmethylene group, isobutylmethylene group, cyclopentylmethylene group, cyclohexylmethylene group, methylethylene group, ethylethylene group, isopropylethylene group, propylethylene group, butylethylene group, isobutylethylene group, cyclopentylethylene group, cyclohexylethylene group, dimethylmethylene group, 1,1-dimethylethylene group, 1,1-cyclopentylene group, 1,2-cyclopentylene group, 1,3-cyclopentylene group, 1,1-cyclohexylene group, 1,2-cyclohexylene group, 1,3-cyclohexylene group, 1,4-cyclohexylene group, 2-butenylene group, 1-methyl-2-butenylene group, 2-methyl-2-butenylene group and 2-butynylene group.

Examples of the substituents for the "substituted or unsubstituted monovalent aliphatic hydrocarbon group" include an aromatic hydrocarbon group, an aromatic heterocyclic group, hydroxyl group, a $C_{1-4}$ alkoxy group, mercapto group, a $C_{1-4}$ alkylthio group, carboxyl group, a $C_{2-5}$ alkoxycarbonyl group, sulfo group, cyano group, $CONR^{11}R^{12}$, and $SO_2NR^{13}N^{14}$ (wherein $R^{11}$, $R^{12}$, $R^{13}$, and $R^{14}$ each represents a hydrogen atom, a $C_{1-4}$ alkyl group, or an aromatic hydrocarbon group).

The term "aromatic hydrocarbon group" as used herein means phenyl group or naphthyl group, and the term "aromatic heterocyclic group" as used herein means furyl group, thienyl group, pyridyl group, indolyl group, quinolyl group, isoquinolyl group, or imidazolyl group. They may have a substituent selected from a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ are as defined as above).

Examples of the $C_{1-4}$ alkyl group include methyl group, ethyl group, propyl group, 2-propyl group, butyl group, 2-butyl group and t-butyl group.

Examples of the $C_{1-4}$ alkoxy group include methoxy group, ethoxy group, propyloxy group, 2-propyloxy group, butyloxy group, 2-butyloxy group and t-butyloxy group.

Examples of the $C_{1-4}$ alkylthio group include methylthio group, ethylthio group, propylthio group, 2-propylthio group, butylthio group, 2-butylthio group and t-butylthio group.

Examples of the $C_{2-8}$ alkoxyalkyl group include those comprising a $C_{1-4}$ alkoxy moiety and a $C_{1-4}$ alkyl moiety, such as methoxymethyl group, ethoxymethyl group, propyloxymethyl group, methoxymethyl group, 2-propyloxyethyl group, and t-butyloxyethyl group.

Examples of the $C_{2-5}$ alkoxycarbonyl group include those comprising a $C_{1-4}$ alkoxy moiety and a carbonyl moiety, such as methoxycarbonyl group, ethoxycarbonyl group, propyloxycarbonyl group and t-butyloxycarbonyl group.

Examples of the halogen atom include fluorine atom, chlorine atom, bromine atom and iodine atom.

Examples of the 4- to 7-membered carbon ring which is formed by $R^2$ with $R^3$ or $R^6$ with $R^7$ include cyclobutane ring, cyclopentane ring, cyclohexane ring, cycloheptane ring, indane ring, tetrahydronaphthalene ring, fluorene ring and dihydrophenalene ring.

Examples of the 4- to 7-membered ring which is formed by $R^8$ with $R^9$ include pyrrolidine ring, piperidine ring, morpholine ring and piperazine ring.

The monovalent hydrocarbon group represented by $R^4$, $R^5$, $R^6$ or $R^7$ in formula (I) has from 1 to 16 carbon atoms, preferably from 1 to 6 carbon atoms. Specific examples thereof include methyl group, ethyl group, propyl group, butyl group, pentyl group, hexyl group, heptyl group, octyl group, nonyl group, decyl group, isopropyl group, isobutyl group, 2-butyl group, t-butyl group, 2-pentyl group, 3-pentyl group, neopentyl group, 3-methylbutyl group, 3-methyl-2-butyl group, 2-hexyl group, 3-hexyl group, 4-methylpentyl group, 4-methyl-2-pentyl group, 2-propenyl group, 2-butenyl group, 3-butenyl group, 2-pentenyl group, 3-pentenyl group, 4-pentenyl group, 2,4-hexadienyl group, 3-methyl-2-butenyl group, 2-propynyl group, 2-butynyl group, 3-butynyl group, cyclobutyl group, cyclopentyl group, cyclohexyl group, cycloheptyl group, 1-adamantyl group, 2-adamantyl group, cyclobutylmethyl group, cyclopentylmethyl, cyclohexylmethyl group, cycloheptylmethyl group, cycloheptyl group, 1-adamantylmethyl group, 2-adamantylmethyl group, 2-cyclobutylethyl group, 2-cyclopentylethyl group, 2-cyclohexylethyl group, 2-cycloheptylethyl group, 2-(1-adamantyl)ethyl group, 2-(2-adamantyl)ethyl group, 2-indanyl group, 9-fluorenyl group and 9-fluorenylmethyl group.

The monovalent hydrocarbon group represented by $R^2$ or $R^3$ has from 1 to 10 carbon atoms, preferably from 1 to 6 carbon atoms.

Preferred examples of the group represented by $R^4$ include methyl group, ethyl group, propyl group, butyl group, pentyl group, hexyl group, heptyl group, octyl group, nonyl group, decyl group, isopropyl group, isobutyl group, 2-butyl group, t-butyl group, 2-pentyl group, 3-pentyl group, neopentyl group, 3-methylbutyl group, 3-methyl-2-butyl group, 2-hexyl group, 3-hexyl group, 4-methylpentyl group, 4-methyl-2-pentyl group, 2-propenyl group, 2-butenyl group, 3-butenyl group, 2-pentenyl group, 3-pentenyl group, 4-pentenyl group, 2,4-hexadienyl group, 3-methyl-2-butenyl group, 2-propynyl group, 2-butynyl group, 3-butynyl group, cyclobutyl group, cyclopentyl group, cyclohexyl group, cycloheptyl group, 1-adamantyl group, 2-adamantyl group, cyclobutylmethyl group, cyclopentylmethyl, cyclohexylmethyl group, cycloheptylmethyl group, cycloheptyl group, 1-adamantylmethyl group, 2-adamantylmethyl group, 2-cyclobutylethyl group, 2-cyclopentylethyl group, 2-cyclohexylethyl group, 2-cycloheptylethyl group, 2-(1-adamantyl)ethyl group, 2-(2-adamantyl)ethyl group, phenyl group, 2-pyridyl group, 3-pyridyl group, 4-pyridyl group, 5-indoryl

group, 3-quinolyl group, 5-quinolyl group, 6-quinolyl group, 8-quinolyl group, phenylmethyl group, 2-phenylethyl group, 3-phenylpropyl group, 1-furylmethyl group, 2-furylmethyl group, 1-thienylmethyl group, 2-thienylmethyl group, 1-naphtylmethyl group, 2-naphtylmethyl group, 3-indolylmethyl group, 2-indanyl group, 9-fluorenyl group, 9-fluorenylmethyl group, diphenylmethyl group, 2-ethylphenyl group, 4-propylphenyl group, 2-carboxyphenyl group, 3-carboxyphenyl group, 4-carboxyphenyl group, 2-methoxycarbonylphenyl, 3-methoxycarbonylphenyl group, 2-chlorophenyl group, 2-cyanophenyl group, 2-nitrophenyl group, 2-(2-carboxyphenyl)ethyl group, 2-(2-ethoxyphenyl)ethyl group, carboxymethyl group, 2-carboxyethyl group, 3-carboxypropyl group, ethoxymethyl group, t-butoxymethyl group, cyanomethyl group, 2-hydroxyethyl group, 2-methoxyethyl group, 2-phenoxyethyl group, 2-benzyloxy group, 2-mercaptoethyl group, 2-phenylthioethyl group, and amidomethyl group.

(Step 1-1)

In Step 1-1, an isocyanate derivative represented by formula (II) above is reacted with an amino acid derivative represented by formula (III) above to give a urea derivative represented by formula (IV) above.

The isocyanate derivative represented by formula (II), which is a starting material to be used in this step, can be prepared by Curtius rearrangement from a carboxylic acid aside derived from a carboxylic acid. The carboxylic acid derivative may be prepared by the processes described, for example, in JP-A-1-149763 and JP-A-2-157260, WO 9109840, and J. Org. Chem. 50 1830-1835 (1985), and examples thereof include:

2-(acetylthiomethyl)propanoic acid,
2-(acetylthiomethyl)butanoic acid,
2-(acetylthiomethyl)pentanoic acid,
2-(acetylthiomethyl)-3-methylbutanoic acid,
2-(acetylthiomethyl)-4-methylpentanoic acid,
2-(acetylthiomethyl)-3-phenylpropanoic acid,
2-(acetylthiomethyl)-4-phenylbutanoic acid,
2-(acetylthiomethyl)-3-cyclohexylpropanoic acid,
2-(acetylthiomethyl)-3-(2-pyridyl)propanoic acid,
2-(acetylthiomethyl)-3-(3-pyridyl)propanoic acid,
2-(acetylthiomethyl)-3-(4-pyridyl)propanoic acid,
2-(acetylthiomethyl)-3-(2-furyl)propanoic acid,
2-(acetylthiomethyl)-3-(3-furyl)propanoic acid,
2-(acetylthiomethyl)-3-(2-thienyl)propanoic acid,
2-(acetylthiomethyl)-3-(3-thienyl)propanoic acid,
2-(acetylthiomethyl)3-(3-indolyl)propanoic acid,
2-(acetylthiomethyl)-3-(4-methylphenyl)propanoic acid,
2-(acetylthiomethyl)-3-(4-methoxyphenyl)propanoic acid,
2-(acetylthiomethyl)-3-(4-benzyloxyphenyl)propanoic acid,
2-(acetylthiomethyl)-3-(4-fluorophenyl)propanoic acid,
2-(acetylthiomethyl)-3-(4-nitrophenyl)propanoic acid,
2-(acetylthiomethyl)-3-(4-cyanophenyl)propanoic acid,
2-(acetylthiomethyl)-3-(4-dimethylaminophenyl)propanoic acid,
2-(acetylthiomethyl)-3-(2,3-dimethoxyphenyl)propanoic acid,
2- (acetylthiomethyl)-3-(2,3-methylenedioxyphenyl)propanoic acid,
2-(acetylthiomethyl)-2-methylpropanoic acid,
3-(acetylthio)-2,2-diphenylpropanoic acid,
4-(acetylthio)-2,2-diphenylbutanoic acid,
5-(acetylthio)-2,2-diphenylpentanoic acid,
1-(acetylthiomethyl)cyclopentanecarboxylic acid,
1-(acetylthiomethyl)cyclohexanecarboxylic acid,
2-(acetylthiomethyl)indane-2-carboxylic acid,
2-(benzoylthiomethyl)-3-phenylpropanoic acid,
2-[(2,4,6-trimethylbenzoyl)thiomethyl]-3-phenylpropanoic acid,
2-(t-butoxycarbonylthiomethyl)-3-phenylpropanoic acid,
2-(phenylmethoxycarbonylthiomethyl)-3-phenylpropanoic acid,
2-(ethylaminocarbonylthiomethyl)-3-phenylpropanoic acid,
2-(ethyldithiomethyl)-3-phenylpropanoic acid,

2-(t-butoxycarbonyldithiomethyl)-3-phenylpropanoic acid,
2-(3-nitro-2-pyridylthiomethyl)-3-phenylpropanoic acid,
2-(phenylmethylthiomethyl)-3-phenylpropanoic acid,
2-(4-nitrophenylmethylthiomethyl)-3-phenylpropanoic acid,
2-(diphenylmethylthiomethyl)-3-phenylpropanoic acid,
2-(methoxymethylthiomethyl)-3-phenylpropanoic acid,
2-(2-tetrahydropyranylthiomethyl)-3-phenylpropanoic acid,
2-(2-cyanoethylthiomethyl)-3-phenylpropanoic acid, and
2-[2,2-bis(ethoxycarbonyl)ethylthiomethyl]-3-phenylpropanoic acid.

The amino acid derivative of formula (III) may be prepared by the process described in Helv. Chim. Acta. 51 78-85 (1968), Eur. J. Med. Chem. 23 523-531 (1988), and JP-A-58-150562, and examples thereof include:

N-methylglycine methyl ester,
N-methylglycine ethyl ester,
N-ethylglycine methyl ester,
N-propylglycine methyl ester,
N-(2-propyl)glycine methyl ester,
N-(t-butyl)glycine methyl ester,
N-(3-pentyl)glycine methyl ester,
N-(2,2-dimethylpropyl)glycine methyl ester,
N-cyclobutylglycine methyl ester,
N-cyclopentylglycine methyl ester,
N-cyclohexylglycine methyl ester,
N-(1-adamantyl)glycine methyl ester,
N-(2-adamantyl)glycine methyl ester,
N-phenylglycine methyl ester,
N-(phenylmethyl)glycine methyl ester,
N-(2,4-dimethoxyphenylmethyl)glycine methyl ester,
N-(2-phenylethyl)glycine methyl ester,
N-(1-naphthylmethyl)glycine methyl ester,
N-(2-naphthylmethyl)glycine methyl ester,
N-(2-pyridyl)glycine methyl ester,
N-(3-pyridyl)glycine methyl ester,
N-(4-pyridyl)glycine methyl ester,
N-(5-indolyl)glycine methyl ester,
N-(2-indanyl)glycine methyl ester,
N-(9-fluorenyl)glycine methyl ester,
N-(diphenylmethyl)glycine methyl ester,
N-(2-ethylphenyl)glycine methyl ester,
N-(4-propylphenyl)glycine methyl ester,
N-(2-methoxycarbonylphenyl)glycine methyl ester,
N-(3-methoxycarbonylphenyl)glycine methyl ester,
N-(2-chlorophenyl)glycine methyl ester,
N-(2-cyanophenyl)glycine methyl ester,
N-(2-nitrophenyl)glycine methyl ester,
N-cyclopentylaspartic acid dimethyl ester,
N-cyclopentylglutamic acid diethyl ester,
cyclopentylaminomalonic acid diethyl ester,
cyclohexylaminomalonic acid diethyl ester,
N-(ethoxycarbonylmethyl)glycine t-butyl ester,
N-(ethoxycarbonylmethyl)valine t-butyl ester,
N-(1-ethoxycarbonyl-1-methylethyl)glycine ethyl ester,
N-(1-methoxycarbonylcyclopentyl)glycine ethyl ester,
N-(1-methoxycarbonylcyclohexyl)glycine ethyl ester,
N-(ethoxycarbonylmethyl)valine methyl ester,
N-(ethoxycarbonylmethyl)phenylalanine t-butyl ester,
N-(ethoxycarbonylmethyl)leucine t-butyl ester,
N-(ethoxycarbonylmethyl)alanine t-butyl ester,

N-(ethoxycarbonylmethyl)isoleucine t-butyl ester,
N-(ethoxycarbonylmethyl)tryptophan t-butyl ester,
$N^2$-(ethoxycarbonylmethyl)-$N^6$-(t-butoxycarbonyl)lysine t-butyl ester,
N-(t-butoxycarbonylmethyl)valine methyl ester,
N-(t-butoxycarbonylmethyl)phenylalanine ethyl ester,
N-(1-methoxycarbonylcyclopentyl)glycine t-butyl ester,
N-(t-butoxycarbonylmethyl)-o-benzyltyrosine methyl ester,
N-(t-butoxycarbonylmethyl)tryptophan methyl ester,
N-(1-methoxycarbonaylcyclopentyl)alanine t-butyl ester,
N-(t-butoxycarbonylmethyl)valine methyl ester,
N-(t-butoxycarbonylmethyl)isoleucine methyl ester,
N-(t-butoxycarbonylmethyl)threonine methyl ester,
N-(1-methoxycarbonyl-1-methylethyl)glycine t-butyl ester,
N-(1-methoxycarbonylcyclopentyl)glycine t-butyl ester,
N-(2-ethoxycarbonyl-2-indanyl)glycine t-butyl ester,
N-(ethoxycarbonylmethyl)-$\beta$-alanine ethyl ester,
N-(3-t-butoxycarbonylpropyl)glycine ethyl ester,
N-(cyanomethyl)glycine ethyl ester,
N-(hydroxyethyl)glycine ethyl ester,
3-(phenylmethylamino)-3-methylbutanoic acid ethyl ester,
3-(1-amadantylmethylamino)-3-methylbutanoic acid ethyl ester,
3-(2-pyridylmethylamino)-3-methylbutanoic acid ethyl ester,
3-(t-butoxycarbonylmethylamino)-3-methylbutanoic acid ethyl ester,
1-(phenylmethylamino)cyclopentylacetic acid ethyl ester,
1-(ethoxycarbonylmethylamino)pyrrolidine,
1-(ethoxycarbonylmethylamino)piperidine,
4-(ethoxycarbonylmethylamino)morpholine,
N-ethoxycarbonyl-N'-methylhydrazine,
N-methoxycarbonyl-N'-cyclopentylhydrazine,
N-ethoxycarbonyl-N'-phenylhydrazine,
N-ethoxycarbonyl-N'-phenylmethylhydrazine,
N-ethoxycarbonyl-N'-(4-fluorophenylmethyl)hydrazine,
N-ethoxycarbonyl-N'-(2-methoxycarbonylphenylmethyl)hydrazine,
N-ethoxycarbonyl-N'-(2,4-dimethoxyphenylmethyl)hydrazine,
N-ethoxycarbonyl-N-cyclohexyl-N'-cyclohexylhydrazine,
N-ethoxycarbonyl-N'-(ethoxycarbonylmethyl)hydrazine,
N-ethoxycarbonyl-N-methyl-N'-methylhydrazine,
N-(ethoxycarbonylmethyl)-N-methyl-N'-methylhydrazine,
N-(ethoxycarbonylmethyl)-N-phenylmethyl-N'-phenylmethylhydrazine, and
N,N-bis(ethoxycarbonylmethyl)-N'-cyclopentylhydrazine.

The production of the isocyanate derivative of formula (II) from the carboxylic acid derivative is preferably carried out in a solvent such as benzene, toluene and diphenylether. The isocyanate derivative of formula (II) may be produced by heating the carboxylic acid derivative at a reaction temperature of generally from 20 to 200°C, preferably from 40 to 120°C, in the presence of diphenylphosphorylazide (DPPA) and triethylamine.

The reaction of Step 1-1 is preferably carried out in a solvent such as benzene, toluene, diphenylether, methylenechloride and tetrahydrofuran. The isocyanate derivative of formula (II) is reacted with the amino acid derivative of formula (III) at a reaction temperature of generally from 0 to 200°C, preferably from 20 to 120°C, and if desired, in the presence of an amine compound such as triethylamine, thus the urea derivative of formula (IV) is obtained.

(Step 1-2)

In Step 1-2, the urea derivative of formula (IV) is subjected to cyclization reaction, thereby the hydantoin derivative of formula (I) is produced.

The cyclization reaction may be carried out in either (a) a method in which the reaction is carried out under an alkaline condition, (b) a method in which the reaction is carried out under an acidic condition, or (c) a method in which the reaction is carried out while heating.

Examples of the alkali to be used in the method (a) include sodium hydroxide, potassium hydroxide, and potassium tert-butoxide. The cyclization reaction under an alkaline condition is preferably carried out in a solvent such as methanol, ethanol and tetrahydrofuran. The reaction proceeds smoothly at from 0 to 100 ° C.

Examples of the acid to be used in the method (b) include p-toluenesulfonic acid, methanesulfonic acid, and trifluoroacetic acid. The cyclization under an acidic condition is preferably carried out in a solvent such as methanol and ethanol. The reaction proceeds smoothly at from 0 to 150 ° C.

In the method (c), the cyclization reaction is proceeded by simply heating the reactants at, generally from 80 to 300 ° C, preferably from 120 to 180 ° C.

In the production of the hydantoin derivative of formula (I), it is possible to start Step 1-2 without isolating the urea derivative of formula (IV) obtained in the Step 1-1.

Alternatively, the hydantoin derivative represented by formula (I) according to the present invention may also be prepared by the reaction scheme shown below.

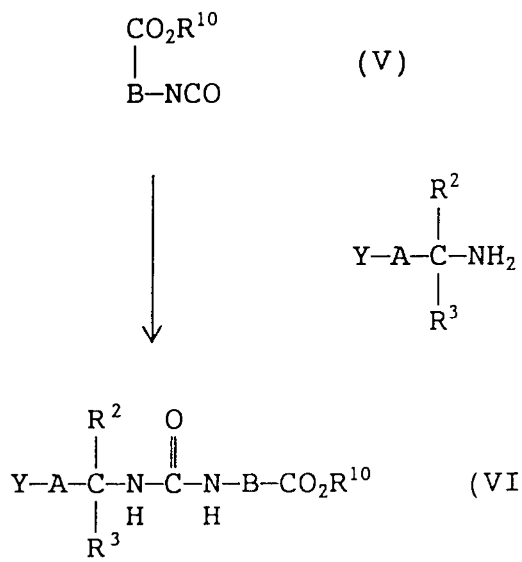

(V)

(Step 2-1)

(VI)

(VII)

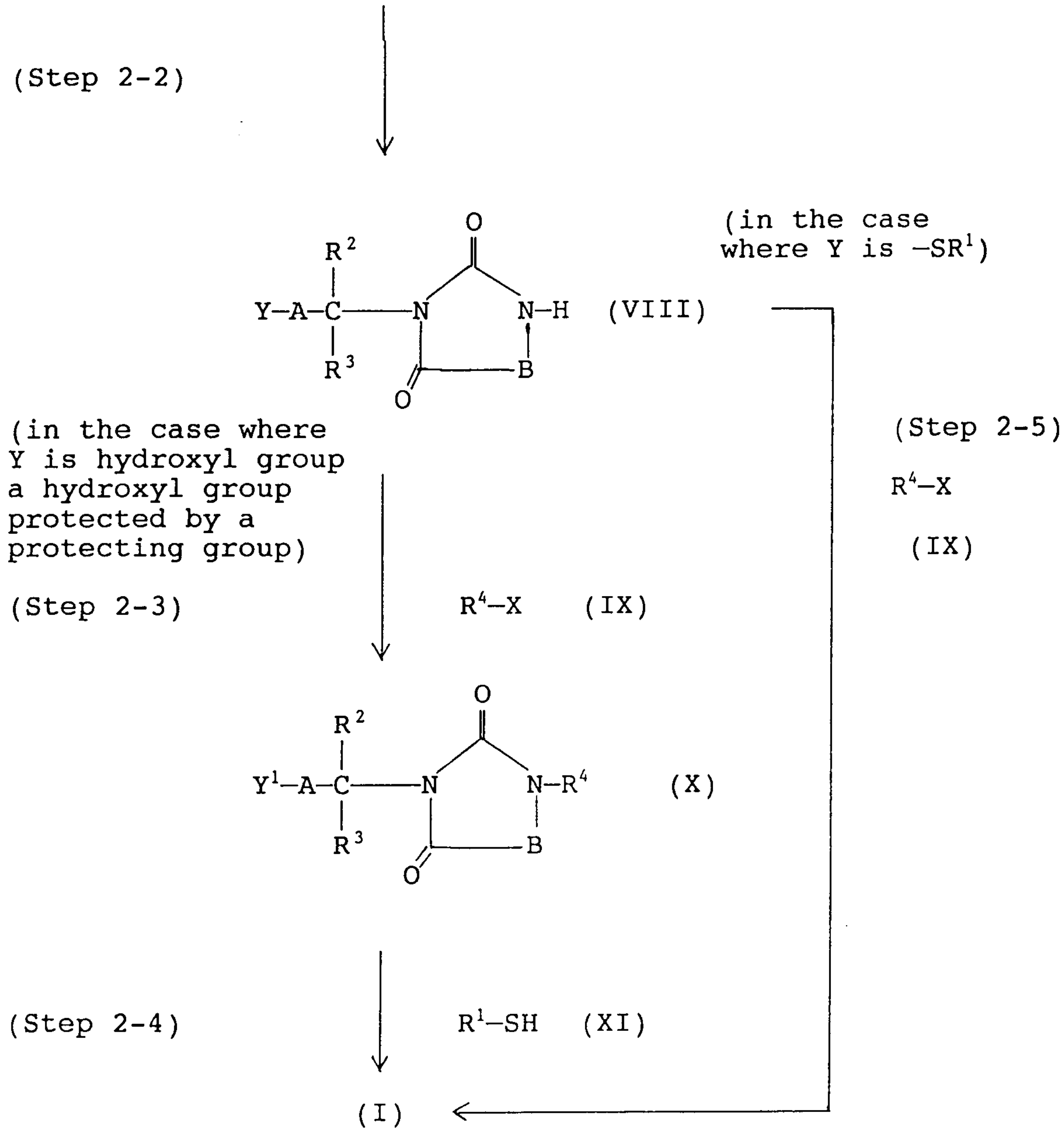


wherein A, B, $R^1$, $R^2$, $R^3$ and $R^{10}$ are the same meanings as defined above; Y represents hydroxyl group, a hydroxyl group protected by a protecting group, or $-SR^1$ group; $Y^1$ represents hydroxyl group or a hydroxyl group protected by a protecting group; and X represents a halogen atom.

Examples of the protecting group for the hydroxyl group represented by Y or $Y^1$ include t-butyl group, acetyl group, benzyl group, methoxymethyl group, benzoyl group and tetrahydropyranyl group.

Examples of the halogen atom include fluorine atom, chlorine atom, bromine atom and iodine atom.

(Step 2-1)

In Step 2-1, an isocyanate derivative represented by formula (V) above is reacted with an amine derivative represented by formula (VI) above to give a urea derivative represented by formula (VII) above.

As in Step 1-1, the isocyanate derivative represented by formula (V), which is a starting material to be used in this step, can be prepared by Curtius rearrangement from a carboxylic acid azide derived from a corresponding carboxylic acid derivative. In view of the stability of the isocyanate derivative of formula (V), it is preferred that the carboxylic acid derivative is subjected to the above-mentioned reaction to give the isocyanate derivative of formula (V), and, without isolation, the resulting isocyanate derivative is then reacted with the amine derivative of formula (VII). Accordingly, the carboxylic acid derivative can be

exemplified by way of examples of the isocyanate derivative. Examples of the carboxylic acid derivative include 2-ethoxycarbonyl-2-methylpropanonic acid, 1-ethoxy-carbonyl-1-cyclobutanecarboxylic acid, 1-methoxycarbonyl-1-cycloheptanecarboxylic acid, 1-methoxycarbonyl-1-cyclohexanecarboxylic acid, 2-methoxycarbonylpropanonic acid, 2-methoxycarbonyl-2-phenylacetic acid, 2-ethoxycarbonyl-2-(phenylmethyl)-3-phenylpropanoic acid, 2-ethoxycarbonyl-3-phenylpropanoic acid, 1-(ethoxycarbonyl)indan-1-carboxylic acid and 2-(methoxycarbonyl)indan-2-carboxylic acid.

Examples of the amine derivative represented by formula (VI) include 2-aminoethanol, 3-amino-1-propanol, 1-amino-2-propanol, 2-amino-1-propanol, 1-amino-2-butanol, 2-amino-1-butanol, 4-amino-1-butanol, 2-amino-2-methyl-1-propanol, 2-amino-3-methyl-1-butanol, 2-amino-1-phenylethanol, 2-amino-2-phenylethanol, 2-amino-3-phenyl-1-propanol, 1-amino-1-cyclopentanemethanol, 1-amino-1-cyclohexanemethanol and 2-amino-2-indanemethanol. The hydroxyl group of these compounds may be protected by a protecting group. Preferred examples of the protecting group include t-butyl group, acetyl group, benzyl group, methoxymethyl group, benzoyl group and tetrahydropyranyl group. Further examples of the amine derivative represented by formula (VI) include 1-(acetylthio)-2-propylamine, 1-(acetylthio)-3-phenyl-2-propylamine, 1-(actylthio)-3-methyl-2-butylamine, 1-(benzoylthio)-2-methyl-2-propylamine, 2-(benzoylthio)-1-phenylethylamine, 1-(acetylthiomethyl)-1-cyclopentylamine, 1-(benzoylthiomethyl)-1-cyclohexylamine, 4-(acetylthio)-2-butylamine and 1-(acetylthio)-4-methyl-2-butylamine.

The reaction of Step 2-1 may be carried out in the same condition as in Step 1-1.

(Step 2-2)

In Step 2-2, the urea derivative of formula (VII) obtained in Step 2-1 is subjected to cyclization reaction, thereby a hydantoin derivative of formula (VIII) is produced.

The reaction of Step 2-2 may be carried out in the same condition as in Step 1-2.

(Step 2-3)

In Step 2-3, the hydantoin derivative of formula (VIII) obtained in Step 2-2 wherein Y is hydroxyl group or a hydroxyl group protected by a protecting group is reacted with an alkyl halide of formula (IX), thereby a hydantoin derivative of formula (X) is produced.

Examples of the alkyl halide of formula (IX) include methyl iodide, bromoethan, 3-bromopropane, benzyl bromide, (1-bromoethyl)benzene, (2-bromoethyl)benzene, bromocyclopentane, bromocyclohexane, ethyl bromoacetate, methyl 2-bromopropanoate, methyl 3-bromopropanoate, bromomethylcyclohexane, methyl 2-bromo-3-phenylacetate, bromoacetonitrile and chlorodiphenylmethane.

The reaction of Step 2-3 may preferably be carried out in the presence of a base such as sodium hydroxide, potassium hydroxide, sodium methoxide, sodium ethoxide, sodium hydride, potassium t-butoxide, sodium carbonate and potassium carbonate. This reaction is preferably carried out in a solvent such as tetrahydrofuran, acetonitrile and acetone. The reaction proceeds smoothly at from -20 to 100 °C.

(Step 2-4)

In Step 2-4, the hydantoin derivative of formula (X) obtained in Step 2-3 is reacted with the thiol compound of formula (XI) in the presence of triphenylphosphine and a dialkylazodicarboxylate, thereby the hydantoin derivative of formula (I) is produced.

The thiol compound of formula (XI) is a compound consisting of the group represented by $R^1$ defined above and thiol group and it is commercially available.

The reaction of Step 2-3 is preferably carried out in a solvent such as tetrahydrofuran, diethylether, benzene and toluene. The reaction proceeds smoothly at -30 to 100 °C.

(Step 2-5)

In Step 2-5, the hydantoin derivative of formula (VIII) obtained in Step 2-2 wherein Y is $-SR^1$ is reacted with the alkyl halide of formula (VIII), thereby the hydantoin derivative of formula (I) is produced.

The reaction of Step 2-5 may be carried out in the same manner as in Step 2-3.

The production methods mentioned above permit the efficient production of the hydantoin derivative of formula (I) of the present invention.

The pharmaceutically acceptable salt of the hydantoin derivative of formula (I) include metal a salt such as sodium salt, potassium salt, calcium salt and magnesium salt and an acid addition salt such as

hydrochloride, sulfate, phosphate, acetate, citrate, fumarate, maleate and succinate.

The hydantoin derivative represented of formula (I) of the present invention or a pharmaceutically acceptable salt thereof has an inhibitory activity on metalloprotease, and thus is useful as a treating drug for cardiovascular diseases and an analgesic drug.

For the above mentioned purpose, the hydantoin derivative of formula (I) or a salt thereof according to the present invention may generally be administrated to a patient in a dose of from about 0.1 to 500 mg, preferably from about 1 to 50 mg per kg of body weight per day at once or by several times (e.g., 1 to 4 times) per day by oral or parenteral administration (e.g., hypodermically, intermuscularly, or intra-abdominally), preferably oral administration.

The hydantoin derivative of formula (I) or a salt thereof according to the present invention may be formulated into tablets, powders, pills, capsules, granules or emulsions for oral administration or an injection, an external drug or a suppository for parenteral administration, together with a carrier (e.g., starch, lactose), a vehicle (e.g., dicalcium phosphate), a binder (corn starch, gum arabic), a lubricant (magnesium stearate), etc., in a conventional manner.

## EXAMPLE 1

1-cyclopentyl-3-(1-mercapto-3-phenyl-2-propyl)hydantoin

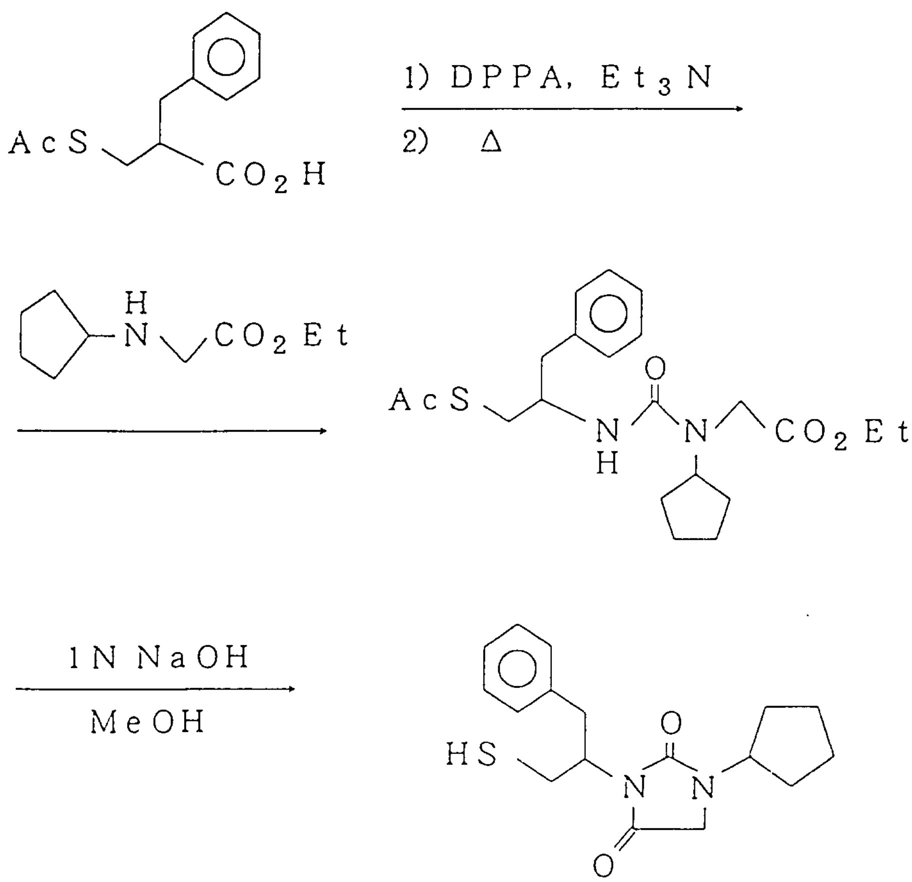


(1) N-(1-acetylthio-3-phenyl-2-propyl)-N'-cyclopentyl-N'-(ethoxycarbonylmethyl)urea:

To a solution of 1.19 g (5 mmol) of 3-acetylthio-2-benzylpropionic acid and 1.38 g (5 mmol) of diphenylphosphorylazide (DPPA) in 30 ml of dry toluene was added 0.7 ml (5 mmol) of triethylamine under nitrogen atmosphere, followed by stirring at room temperature for 30 minutes and then at 80 °C for 2 hours. The mixture was cooled to room temperature and a solution of 0.86 g (5 mmol) of N-cyclopentylglycine ethyl ester in 5 ml of dry toluene was added thereto, followed by stirring for 18 hours. To the reaction mixture was added ethyl acetate, and the resulting mixture was washed with saturated aqueous solution of

sodium hydrogen carbonate and then with saturated aqueous solution of sodium chloride. The organic layer was dried over anhydrous sodium sulfate, and the solvent was removed under reduced pressure. The residue was purified by silica gel chromatography to give the title compound in a yield of 1.17 g (58%).
NMR Spectrum (δ, $CDCl_3$):
1.27 (3H, t, J = 7Hz), 1.31-1.47 (2H, m), 1.50-1.71 (4H, m), 1.77-1.91 (2H, m), 2.36 (3H, s), 2.76 (1H, dd, J = 14Hz, 8Hz), 3.01 (2H, d, J = 6Hz), 3.08 (1H, dd, J = 14Hz, 5Hz), 3.85 (1H, d, J = 16Hz), 3.89 (1H, d, J = 16Hz), 3.79-4.08 (1H, m), 4.09-4.20 (1H, m), 4.19 (2H, q, J = 7Hz), 4.97 (1H, d, J = 7Hz), 7.20-7.33 (5H, m)

(2) 1-cyclopentyl-3-(1-mercapto-3-phenyl-2-propyl)hydantoin:

To a solution of 1.02 g (2.5 mmol) of N-(1-acetylthio-3-phenyl-2-propyl)-N'-cyclopentyl-N'-(ethoxycarbonylmethyl)urea in 30 ml of methanol was added 7.5 ml of 1N NaOH while cooling with ice, followed by stirring at room temperature for 2 hours. After removing the methanol under reduced pressure, 30 ml of water and 9 ml of 1N HCl were added to the residue, followed by extraction with ethyl acetate. The organic layer was washed with a saturated solution of sodium chloride and dried over anhydrous sodium sulfate, and the solvent was removed under reduced pressure. The residue was purified by silica gel chromatography to give the title compound in a yield of 0.64 g (81%).

| Elemental analysis for $C_{17}H_{22}N_2O_2S$: | | | | |
|---|---|---|---|---|
| | C % | H % | N % | S % |
| Calcd. | 64.12 | 6.96 | 8.80 | 10.07 |
| Found | 64.23 | 7.01 | 8.71 | 10.16 |

Property: oily
IR Spectrum (νKBr, $cm^{-1}$): 2560, 1768, 1712
NMR Spectrum (δ, $CDCl_3$):
1.32-1.48 (3H, m), 1.52-1.96 (6H, m), 2.86-2.96 (1H, m), 3.11-3.26 (3H, m), 3.59 (1H, d, J = 17Hz), 3.67 (1H, d, J = 17Hz), 4.26-4.42 (2H, m), 7.13-7.29 (5H, m)

EXAMPLE 2

1-(2,2-dimethylpropyl)-3-(1-mercapto-3-phenyl-2-propyl)-hydantoin

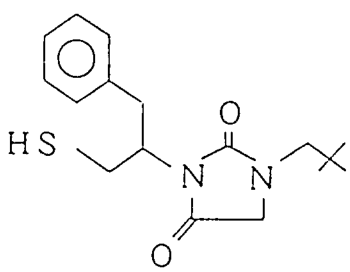

The same procedure as in Example 1 was repeated except that N-(2,2-dimethylpropyl)glycine ethyl ester was used as the amino acid derivative, thereby the title compound was obtained.

| Elemental analysis for $C_{17}H_{24}N_2O_2S$: | | | | |
|---|---|---|---|---|
| | C % | H % | N % | S % |
| Calcd. | 63.72 | 7.55 | 8.74 | 10.00 |
| Found | 63.85 | 7.42 | 8.69 | 10.11 |

Property: oily
IR Spectrum (νKBr, $cm^{-1}$): 2576, 1760, 1700
MNR Spectrum (δ, $CDCl_3$):
0.89 (9H, s), 1.37 (1H, dd, J = 10Hz, 7Hz), 2.89-2.99 (1H, m), 3.04 (2H, s), 3.12-3.28 (3H, m), 3.78 (1H, d, J = 17Hz), 3.82 (1H, d, J = 17Hz), 4.27-4.39 (1H, m), 7.15-7.30 (5H, m)

EXAMPLE 3

1-cyclohexyl-3-(1-mercapto-3-phenyl-2-propyl)hydantoin

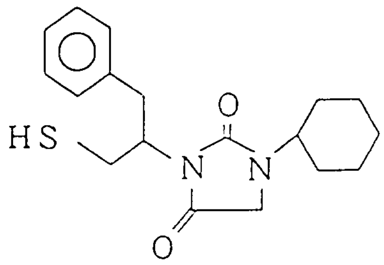

The same procedure as in Example 1 was repeated except that N-cyclohexylglycine ethyl ester was used as the amino acid derivative, thereby the title compound was obtained.

| Elemental analysis for $C_{18}H_{24}N_2O_2S$: | | | | |
|---|---|---|---|---|
| | C % | H % | N % | S % |
| Calcd. | 65.03 | 7.28 | 8.43 | 9.64 |
| Found | 65.11 | 7.19 | 8.51 | 9.59 |

Property: oily
IR Spectrum (νKBr, $cm^{-1}$): 2580, 1754, 1696
MNR Spectrum (δ, $CDCl_3$):
1.01-1.43 (7H, m), 1.59-1.87 (4H, m), 2.87-2.97 (1H, m), 3.11-3.26 (3H, m), 3.58 (1H, d, J = 17Hz), 3.66 (1H, d, J = 17Hz), 3.78-3.88 (1H, m), 4.26-4.37 (1H, m), 7.13-7.29 (5H, m)

EXAMPLE 4

3-(1-mercapto-3-phenyl-2-propyl)-1-(phenylmethyl)hydantoin

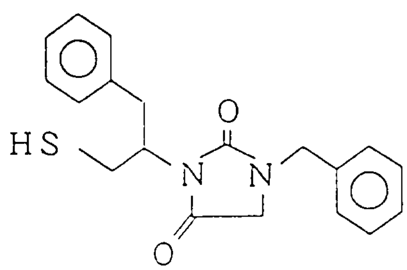

The same procedure as in Example 1 was repeated except that N-(phenylmethyl)glycine ethyl ester was used as the amino acid derivative, thereby the title compound was obtained.

| Elemental analysis for $C_{19}H_{20}N_2O_2S$: | | | | |
|---|---|---|---|---|
| | C % | H % | N % | S % |
| Calcd. | 67.03 | 5.92 | 8.23 | 9.42 |
| Found | 67.21 | 5.90 | 8.19 | 9.36 |

Property: oily
IR Spectrum ($\nu$KBr, $cm^{-1}$): 2560, 1770, 1710
NMR Spectrum ($\delta$, $CDCl_3$):
1.40 (1H, dd, J = 10Hz, 7Hz), 2.91-3.02 (1H, m), 3.13-3.31 (3H, m), 3.53 (1H, d, J = 18Hz), 3.59 (1H, d, J = 18Hz), 4.31-4.42 (1H, m), 4.44 (1H, d, J = 15Hz), 4.52 (1H, d, J = 15Hz), 7.02-7.08 (1H, m), 7.15-7.37 (9H, m)

EXAMPLE 5

3-(1-mercapto-3-phenyl-2-propyl)-1-(1-naphthylmethyl)-hydantoin

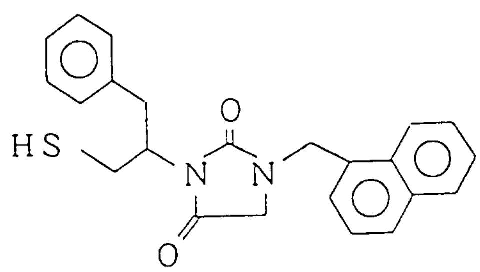

The same procedure as in Example 1 was repeated except that N-(1-naphthylmethyl)glycine ethyl ester was used as the amino acid derivative, thereby the title compound was obtained.

| Elemental analysis for $C_{23}H_{22}N_2O_2S$: | | | | |
|---|---|---|---|---|
| | C % | H % | N % | S % |
| Calcd. | 70.74 | 5.68 | 7.17 | 8.21 |
| Found | 70.69 | 5.72 | 7.20 | 8.19 |

Property: oily
IR Spectrum ($\nu$KBr, $cm^{-1}$): 2560, 1770, 1710
NMR Spectrum ($\delta$, $CDCl_3$):
1.37 (1H, dd, J = 10Hz, 8Hz), 2.88-2.99 (1H, m), 3.11-3.31 (3H, m), 3.41 (1H, d, J = 18Hz), 3.51 (1H, d, J = 18Hz), 4.29-4.41 (1H, m), 4.89 (1H, d, J = 15Hz), 5.02 (1H, d, J = 15Hz), 7.11-7.59 (9H, m), 7.81-8.06 (3H, m)

EXAMPLE 6

1-(2-indanyl)-3-(1-mercapto-3-phenyl-2-propyl)hydantoin

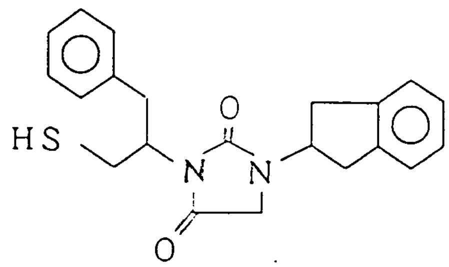

The same procedure as in Example 1 was repeated except that N-(2-indanyl)glycine ethyl ester was used as the amino acid derivative, thereby the title compound was obtained.

| Elemental analysis for $C_{21}H_{22}N_2O_2S$: | | | | |
|---|---|---|---|---|
| | C % | H % | N % | S % |
| Calcd. | 68.83 | 6.05 | 7.64 | 8.75 |
| Found | 69.01 | 6.18 | 7.59 | 8.66 |

Property: oily
IR Spectrum ($\nu$KBr, $cm^{-1}$): 2560, 1770, 1708
NMR Spectrum ($\delta$, $CDCl_3$):
1.38 (1H, dd, J=10Hz, 7Hz), 2.68-2.97 (3H, m), 3.12-3.29 (5H, m), 3.39 (1H, d, J=17Hz), 3.43 (1H, d, J=17Hz), 4.25-4.37 (1H, m), 4.88-4.99 (1H, m), 7.15-7.33 (9H, m)

EXAMPLE 7

3-(1-mercapto-3-phenyl-2-propyl)-1-(3-pentyl)hydantoin

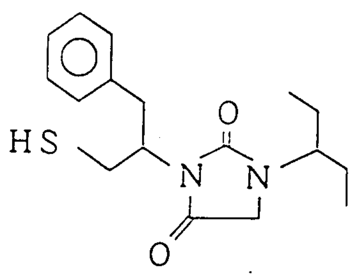

The same procedure as in Example 1 was repeated except that N-(3-pentyl)glycine ethyl ester was used as the amino acid derivative, thereby the tile compound was obtained.

| Elemental analysis for $C_{17}H_{24}N_2O_2S$: | | | | |
|---|---|---|---|---|
| | C % | H % | N % | S % |
| Calcd. | 63.72 | 7.55 | 8.74 | 10.00 |
| Found | 63.69 | 7.61 | 8.69 | 10.11 |

Property: oily
IR Spectrum ($\nu$KBr, $cm^{-1}$): 2570, 1760, 1702

NMR Spectrum (δ, $CDCl_3$):
0.72 (3H, t, J = 7Hz), 0.83 (3H, t, J = 7Hz), 1.21-1.59 (5H, m), 2.88-2.98 (1H, m), 3.09-3.30 (3H, m), 3.48 (1H, d, J = 17Hz), 3.55 (1H, d, J = 17Hz), 3.72-3.84 (1H, m), 4.27-4.40 (1H, m), 7.14-7.38 (5H, m)

## EXAMPLE 8

1-(2-adamantyl)-3-(1-mercapto-3-phenyl-2-propyl)hydantoin

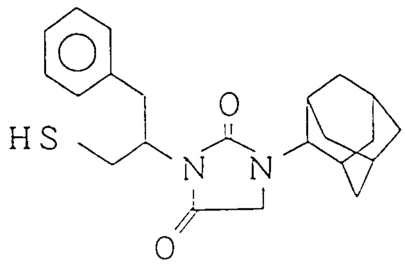

The same procedure as in Example 1 was repeated except that N-(2-adamantyl)glycine ethyl ester was used as the amino acid derivative, thereby the title compound was obtained.

| Elemental analysis for $C_{22}H_{28}N_2O_2S$: | | | | |
|---|---|---|---|---|
| | C % | H % | N % | S % |
| Calcd. | 68.72 | 7.34 | 7.29 | 8.34 |
| Found | 68.76 | 7.29 | 7.31 | 8.40 |

Melting point: 140.3-141.2 °C
IR Spectrum (νKBr, $cm^{-1}$): 2552, 1752, 1702
NMR Spectrum (δ, $CDCl_3$):
1.38 (1H, dd, J = 10Hz, 7Hz), 1.58-1.92 (12H, m), 2.20-2.33 (2H, m), 2.86-2.98 (1H, m), 3.11-3.28 (3H, m), 3.82 (1H, d, J = 17Hz), 3.83 (1H, br-s), 3.91 (1H, d, J = 17Hz), 4.26-4.38 (1H, m), 7.15-7.31 (5H, m)

## EXAMPLE 9

1-(9-fluorenyl)-3-(1-mercapto-3-phenyl-2-propyl)hydantoin

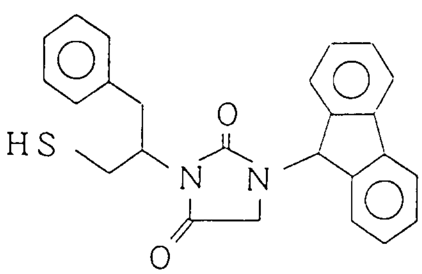

The same procedure as in Example 1 was repeated except that N-(9-fluorenyl)glycine ethyl ester was used as the amino acid derivative, thereby the title compound was obtained.

| Elemental analysis for $C_{25}H_{22}N_2O_2S$: | | | | |
|---|---|---|---|---|
| | C % | H % | N % | S % |
| Calcd. | 72.44 | 5.35 | 6.76 | 7.73 |
| Found | 72.41 | 5.27 | 6.66 | 7.68 |

Melting point: 102.4-103.0 °C
IR Spectrum ($\nu$KBr, $cm^{-1}$): 2555, 1768, 1710
NMR Spectrum ($\delta$, $CDCl_3$):
1.47 (1H, dd, J = 10Hz, 7Hz), 2.97-3.11 (3H, m), 3.14-3.38 (3H, m), 4.39-4.51 (1H, m), 6.21 (1H, s), 7.09 (1H, d, J = 7Hz), 7.22-7.46 (10H, m), 7.70 (2H, dd, J = 7Hz, 3Hz)

EXAMPLE 10

1-(diphenylmethyl)-3-(1-mercapto-3-phenyl-2-propyl)hydantoin

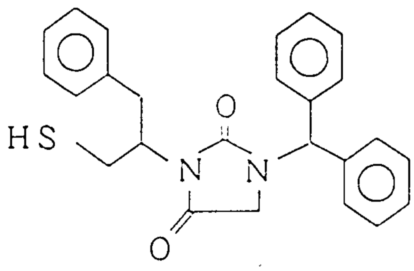

The same procedure as in Example 1 was repeated except that N-(diphenylmethyl)glycine ethyl ester was used as the amino acid derivative, thereby the title compound was obtained.

| Elemental analysis for $C_{25}H_{24}N_2O_2S$: | | | | |
|---|---|---|---|---|
| | C % | H % | N % | S % |
| Calcd. | 72.09 | 5.81 | 6.73 | 7.70 |
| Found | 72.11 | 5.90 | 6.68 | 7.69 |

Property: oily
IR Spectrum ($\nu$KBr, $cm^{-1}$): 2560, 1770, 1708
NMR Spectrum ($\delta$, $CDCl_3$):
1.41 (1H, dd, J = 10Hz, 7Hz), 2.93-3.03 (1H, m), 3.14-3.32 (3H, m), 3.46 (1H, d, J = 18Hz), 3.54 (1H, d, J = 18Hz), 4.32-4.45 (1H, m), 6.48 (1H, s), 6.93-7.00 (2H, m), 7.06-7.13 (2H, m), 7.16-7.40 (11H, m)

EXAMPLE 11

5,6-dihydro-6,6-dimethyl-3-(1-mercapto-3-phenyl-2-propyl)-1-(phenylmethyl)uracil

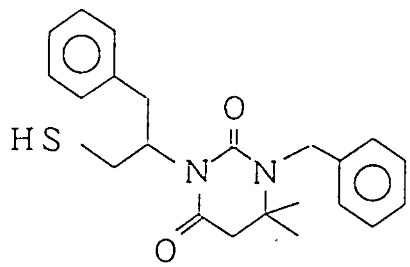

The same procedure as in Example 1 was repeated except that N-(phenylmethyl)-β,β-dimethyl-β-alanine ethyl ester was used as the amino acid derivative, thereby the title compound was obtained.

| Elemental analysis for $C_{22}H_{26}N_2O_2S$: | | | | |
|---|---|---|---|---|
| | C % | H % | N % | S % |
| Calcd. | 69.08 | 6.85 | 7.32 | 8.38 |
| Found | 69.09 | 6.90 | 7.29 | 8.41 |

Property: oily
IR Spectrum (νKBr, $cm^{-1}$): 2570, 1714, 1662
NMR Spectrum (δ, $CDCl_3$):
0.97 (3H, s), 1.06 (3H, s), 1.35 (1H, dd, J = 10Hz, 7Hz), 2.37 (1H, d, J = 16Hz), 2.46 (1H, d, J = 16Hz), 2.93-3.04 (1H, m), 3.17 (1H, dd, J = 14Hz, 6Hz), 3.26-3.38 (2H, m), 4.54 (1H, d, J = 16Hz), 4.66 (1H, d, J = 16Hz), 5.14-5.26 (1H, m), 7.13-7.33 (10H, m)

EXAMPLE 12

1-(t-butoxycarbonylmethyl)-3-(1-mercapto-3-phenyl-2-propyl)hydantoin

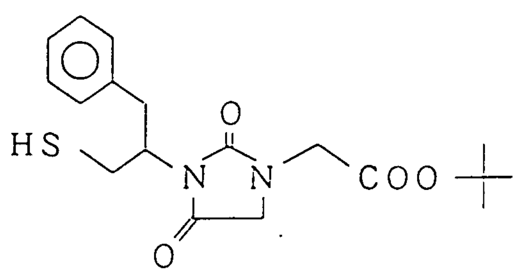

The same procedure as in Example 1 was repeated except that N-(ethoxycarbonylmethyl)glycine t-butyl ester was used as the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.64 | 7.69 | 8.80 |
| Found | 59.35 | 6.60 | 7.71 | 8.83 |

Property: oily
IR Spectrum (νKBr, $cm^{-1}$): 2575, 1776, 1740, 1712
NMR Spectrum (δ, $CDCl_3$):
1.40 (1H, dd, J = 10Hz, J = 9Hz), 1.46 (9H, s), 2.82-2.92 (1H, m), 3.11-3.29 (3H, m), 3.85 (1H, d, J = 19Hz), 3.92 (1H, d, J = 19Hz), 3.99 (2H, s), 4.28-4.38 (1H, m), 7.18-7.29 (5H, m)

EXAMPLE 13

3-(1-mercapto-3-phenyl-2-propyl)-1-(1-methoxycarbonyl-2-methylpropyl)hydantoin

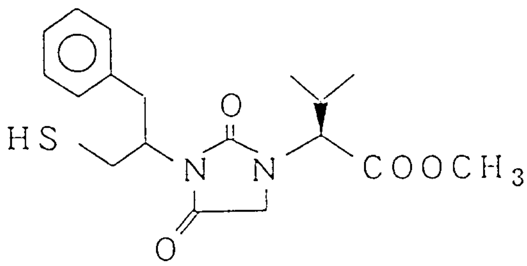

The same procedure as in Example 1 was repeated except that N-(ethoxycarbonylmethyl)valine methyl ester was used the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.64 | 7.69 | 8.80 |
| Found | 59.37 | 6.61 | 7.73 | 8.82 |

Property: oily
IR Spectrum (νKBr, $cm^{-1}$): 2560, 1772, 1744, 1712
NMR Spectrum (δ, $CDCl_3$):
0.73 (3/2H, d, J = 7Hz), 0.87 (3/2H, d, J = 7Hz), 0.92 (3/2H, d, J = 7Hz), 0.97 (3/2H, d, J = 7Hz), 1.36 (1H, dd, J = 10Hz, J = 8Hz), 1.97-2.16 (1H, m), 2.87-2.99 (1H, m), 3.11-3.28 (3H, m), 3.64-3.74 (1H, m), 3.71 (3/2H, d, J = 5Hz), 3.73 (3/2 H, d, J = 5Hz), 4.07-4.18 (1H, m), 4.28-4.40 (1H, m), 4.35-4.42 (1H, m), 7.14-7.27 (5H, m)

EXAMPLE 14

3-(1-acetylthio-3-phenyl-2-propyl)-3-cyclopentyl-5-(ethoxycarbonyl)hydantoin

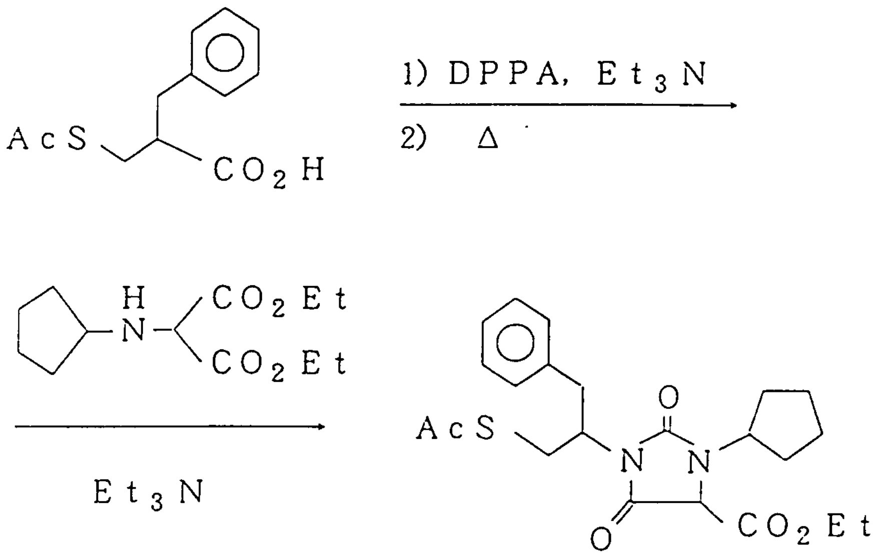


To a solution of 1.19 g (5 mmol) of 3-acetylthio-2-benzylpropionic acid and 1.38 g (5 mmol) of diphenylphosphorylazide (DPPA) in 30 ml of dry toluene was added 0.7 ml (5 mmol) of triethylamine under nitrogen atmosphere, followed by stirring at room temperature for 30 minutes and then at 80 ° C for 2 hours. The mixture was cooled to room temperature and a solution of 1.22 g (5 mmol) of diethylcyclopentyl malonate in 5 ml of dry toluene and 1 ml of triethylamine were added thereto, followed by stirring at 80 ° C for 18 hours. To the reaction mixture was added ethyl acetate, and the mixture was washed with saturated aqueous solution of sodium hydrogen carbonate and then with saturated aqueous solution of sodium chloride. The organic layer as dried over anhydrous sodium sulfate and then the solvent was removed under reduced pressure. The residues were purified by silica gel chromatography to give the title compound in a yield of 0.62 g (29%).

| Elemental analysis for $C_{22}H_{28}N_2O_5S$: | | | | |
|---|---|---|---|---|
| | C % | H % | N % | S % |
| Calcd. | 61.09 | 6.53 | 6.48 | 7.41 |
| Found | 61.30 | 6.43 | 6.51 | 7.23 |

Property: oily
IR Spectrum ($\nu_{neat}$, $cm^{-1}$): 1778, 1746, 1720
NMR Spectrum (δ, $CDCl_3$):
1.27 (3/2H, t, J = 7Hz), 1.29 (3/2H, t, J = 7Hz), 1.31-1.98 (8H, m), 2.30 (3/2H, s), 2.32 (3/2H, s), 3.12 (1H, dd, J = 14Hz, 7Hz), 3.22-3.37 (2H, m), 3.44-3.56 (1H, m), 4.00-4.45 (5H, m), 7.12-7.29 (5H, m)

EXAMPLE 15

1-cyclopentyl-5-ethoxycarbonyl-3-(1-mercapto-3-phenyl-2-propyl)hydantoin

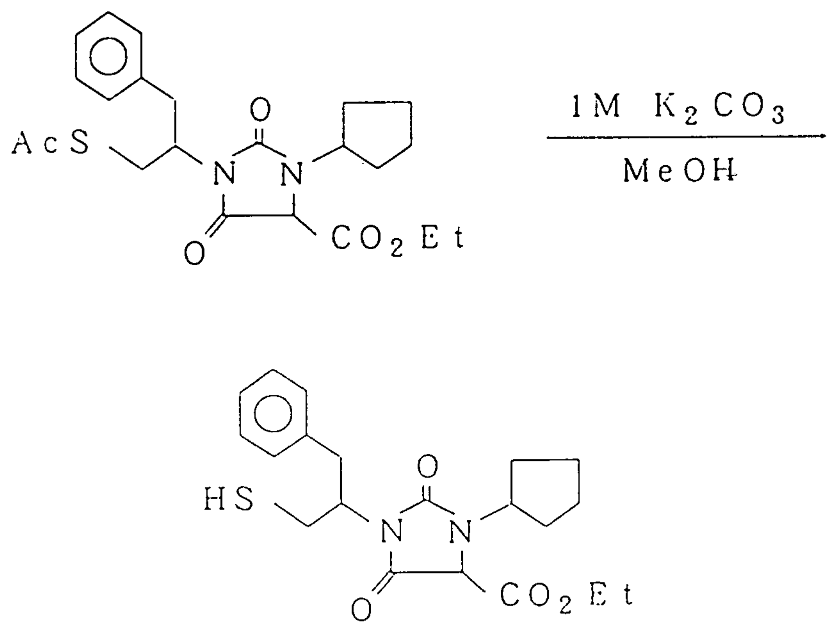


To a solution of 588 mg (1.36 mmol) of 3-(1-acetylthio-3-phenyl-2-propyl)-3-cyclopentyl-5-(ethoxycarbonyl)-hydantoin in 20 ml of methanol was added 2 ml of a 1M aqueous solution of $K_2CO_3$ while cooling with ice, followed by stirring at room temperature for 2 hours. After removing the methanol under reduced pressure, 20 ml of water and 5 ml of 1N HCl was added to the residue, followed by extraction with ethyl acetate. The organic layer was washed with a saturated solution of sodium chloride and dried over anhydrous sodium sulfate, and the solvent was removing under reduced pressure. The residue was purified by silica gel chromatography to give the title compound in a yield of 399 mg (75%).

| Elemental analysis for $C_{20}H_{26}N_2O_4S$: | | | | |
|---|---|---|---|---|
| | C % | H % | N % | S % |
| Calcd. | 61.52 | 6.71 | 7.17 | 8.21 |
| Found | 61.49 | 6.80 | 7.23 | 8.17 |

Property: oily
IR Spectrum ($\nu$KBr, $cm^{-1}$): 2560, 1780, 1748, 1716
NMR Spectrum ($\delta$, $CDCl_3$):
1.23-2.01 (12H, m), 2.78-2.94 (1H, m), 3.08-3.29 (3H, m), 3.77 (1/2H, s), 3.79 (1/2H, s), 4.02-4.45 (4H, m), 7.12-7.35 (5H, m)

EXAMPLE 16

3-(1-acetylthio-3-phenyl-2-propyl)-1-(1-adamantyl)hydantoin

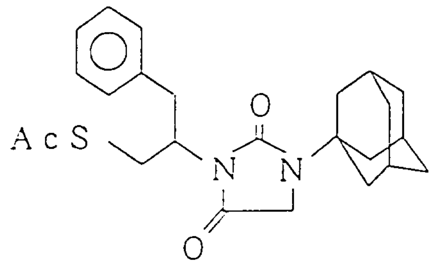

The same procedure as in Example 14 was repeated except that N-(1-adamantyl)glycine ethyl ester was used as the amino acid derivative, thereby the title compound was obtained.

| Elemental analysis for $C_{24}H_{30}N_2O_3S$: | | | | |
|---|---|---|---|---|
| | C % | H % | N % | S % |
| Calcd. | 67.58 | 6.62 | 5.78 | 6.61 |
| Found | 67.49 | 6.59 | 5.81 | 6.59 |

Melting point: 99.7-100.5 °C
IR Spectrum ($\nu$KBr, $cm^{-1}$): 1756, 1696
NMR Spectrum ($\delta$, $CDCl_3$):
1.63-1.70 (6H, m), 1.95-2.02 (6H, m), 2.10 (3H, brs), 2.32 (3H, s), 3.13 (1H, dd, J = 14Hz, 8Hz), 3.22-3.38 (2H, m), 3.51 (1H, dd, J = 14Hz, 10Hz), 3.61 (1H, d, J = 17Hz), 3.78 (1H, d, J = 17Hz), 4.31-4.43 (1H, m), 7.15-7.31 (5H, m)

EXAMPLE 17

1-(1-adamantyl)-3-(1-mercapto-3-phenyl-2-propyl)hydantoin

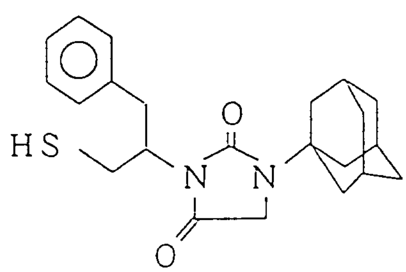

The same procedure as in Example 15 was repeated using 3-(1-acetylthio-3-phenyl-2-propyl)-1-(1-adamantyl)-hydantoin was used, thereby the title compound was obtained.

| Elemental analysis for $C_{22}N_{28}N_2O_2S$: | | | | |
|---|---|---|---|---|
| | C % | H % | N % | S % |
| Calcd. | 68.72 | 7.34 | 7.29 | 8.34 |
| Found | 68.90 | 7.41 | 7.36 | 8.49 |

Property: oily
IR Spectrum (νKBr, $cm^{-1}$): 2572, 1750, 1696
NMR Spectrum (δ, $CDCl_3$):
1.36 (1H, dd, J = 10Hz, 8Hz), 1.64-1.72 (6H, m), 1.97-2.04 (6H, m), 2.08-2.16 (3H, m), 2.83-2.96 (1H, m), 3.10-3.27 (3H, m), 3.67 (1H, d, J = 18Hz), 3.80 (1H, d, J = 18Hz), 4.21-4.33 (1H, m), 7.14-7.31 (5H, m)

EXAMPLE 18

5-(carboxymethyl)-1-cyclopentyl-3-(1-mercapto-3-phenyl-2-propyl)hydantoin

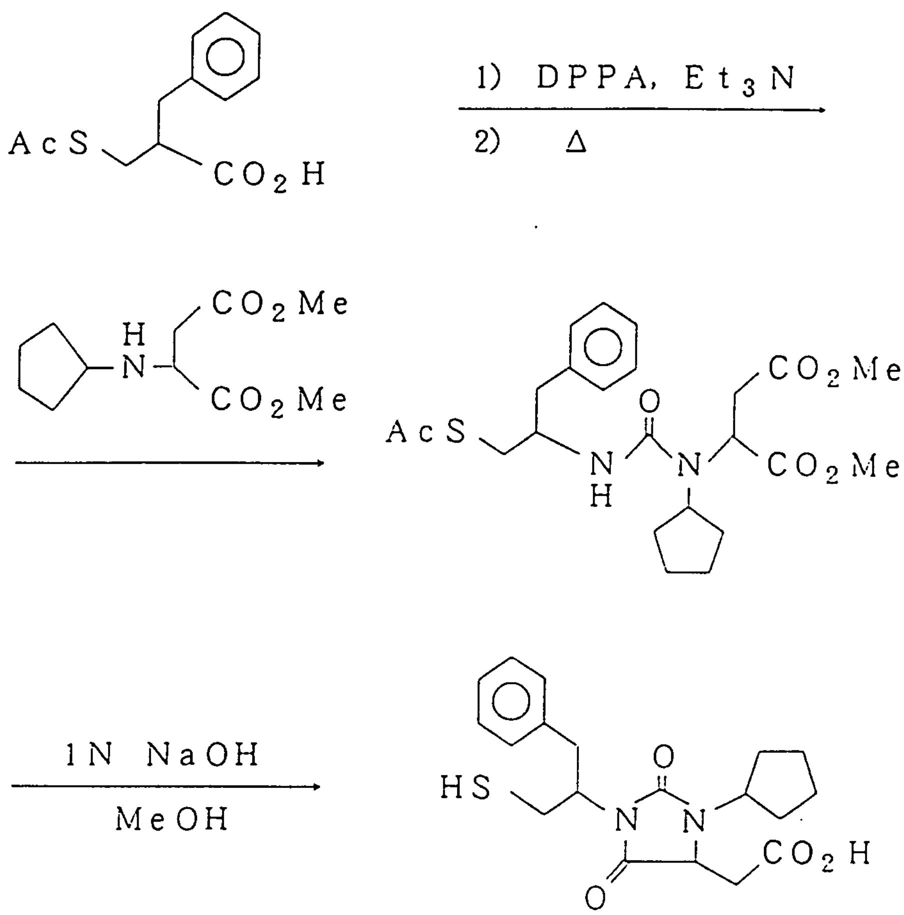


(1) N-(1-acetylthio-3-phenyl-2-propyl)-N'-cyclopentyl-N'-[1,2-bis(methoxycarbonyl)ethyl]urea:

The same procedure as in Example 1 was repeated except that 1.15 g (5 mmol) of N-cyclopentylaspartic acid dimethyl ester was used as the amino acid derivative, thereby the title compound was obtained in a yield of 1.58 g (68%).
NMR Spectrum (δ, $CDCl_3$):
1.41-1.89 (8H, m), 2.35 (3/2H, s), 2.37 (3/2H, s), 2.41-2.52 (1H, m), 2.73-2.87 (1H, m), 2.90-3.09 (3H, m), 3.44-3.58 (1H, m), 3.65-3.70 (1H, m), 3.68 (3/2H, s), 3.70 (3/2H, s), 3.71 (3H, s), 4.08-4.20 (1H, m), 4.23-4.30 (1H, m), 4.75-4.83 (1H, m), 7.15-7.34 (5H, m)

(2) 5-(carboxymethyl)-1-cyclopentyl-3-(1-mercapto-3-phenyl-2-propyl)hydantoin:

To a solution of 1.31 g (2.82 mmol) of N-(1-acetylthio-3-phenyl-2-propyl)-N'-cyclopentyl-N'-[1,2-bis-(methoxycarbonyl)ethyl]urea in 30 ml of methanol was added 10 ml of 1N NaOH while cooling with ice, followed by stirring at room temperature for 2 hours. After removing the methanol under reduced pressure, 30 ml of water and 15 ml of 1N HCl were added to the residue, followed by extraction with ethyl acetate.

The organic layer was washed with a saturated solution of sodium chloride and dried over anhydrous sodium sulfate, and the solvent was removed under reduced pressure. The residue was purified by silica gel chromatography to give the title compound in a yield of 0.85 g (80%).

| Elemental analysis for $C_{19}H_{24}N_2O_4S$: | | | | |
|---|---|---|---|---|
| | C % | H % | N % | S % |
| Calcd. | 60.62 | 6.43 | 7.44 | 8.52 |
| Found | 60.59 | 6.31 | 7.40 | 8.55 |

Property: oily
IR Spectrum ($\nu$KBr, $cm^{-1}$): 2575, 1766, 1710
NMR Spectrum ($\delta$, $CDCl_3$):
1.42-1.97 (9H, m), 2.56-2.92 (3H, m), 3.14-3.33 (3H, m), 3.81-4.06 (2H, m), 4.27-4.39 (1H, m), 7.14-7.30 (5H, m)

EXAMPLE 19

5-(2-carboxyethyl)-1-cyclopentyl-3-(1-mercapto-3-phenyl-2-propyl)hydantoin

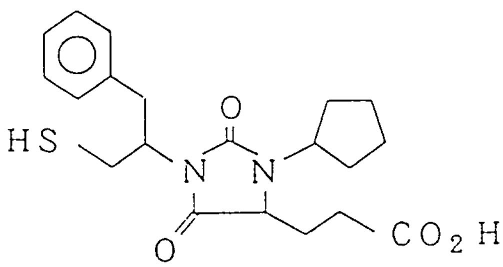

The same procedure as in Example 18 was repeated except that N-cyclopentylglutamic acid diethyl ester was used as the amino acid derivative, thereby the title compound was obtained.

| Elemental analysis for $C_{20}H_{26}N_2O_4S$: | | | | |
|---|---|---|---|---|
| | C % | H % | N % | S % |
| Calcd. | 61.52 | 6.71 | 7.17 | 8.21 |
| Found | 61.39 | 6.84 | 7.29 | 8.17 |

Property: oily
IR Spectrum ($\nu$KBr, $cm^{-1}$): 2568, 1764, 1706
NMR Spectrum ($\delta$, $CDCl_3$):
1.33-1.42 (1H, m), 1.45-2.33 (12H, m), 2.86-3.00 (1H, m), 3.06-3.28 (3H, m), 3.77-3.93 (2H, m), 4.27-4.39 (1H, m), 7.13-7.30 (5H, m)

EXAMPLE 20

1-(carboxymethyl)-3-(1-mercapto-3-phenyl-2-propyl)hydantoin

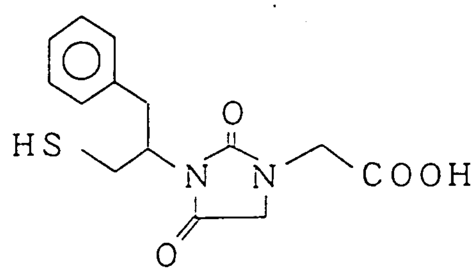

The same procedure as in Example 18 was repeated except that N-(ethoxycarbonylmethyl)glycine ethyl ester was used as the amino acid derivative, thereby the title compound was obtained.

| Elemental analysis for $C_{14}H_{16}N_2O_4S$: | | | | |
|---|---|---|---|---|
| | C % | H % | N % | S % |
| Calcd. | 54.53 | 5.23 | 9.09 | 10.40 |
| Found | 54.58 | 5.19 | 9.14 | 10.56 |

Property: oily
IR Spectrum ($\nu$KBr, $cm^{-1}$): 2575, 1772, 1712
NMR Spectrum ($\delta$, $CDCl_3$):
1.40 (1H, dd, J=10Hz, J=2Hz), 2.82-2.94 (1H, m), 3.10-3.27 (3H, m), 3.85 (1H, d, J=17Hz), 3.92 (1H, d, J=17Hz), 4.12 (1H, d, J=18Hz), 4.18 (1H, d, J=18Hz), 4.27-4.38 (1H, m), 7.15-7.29 (5H, m)

EXAMPLE 21

1-[(1S)-1-carboxy-2-methylpropyl)-3-(1-mercapto-3-phenyl-2-propyl)hydantoin

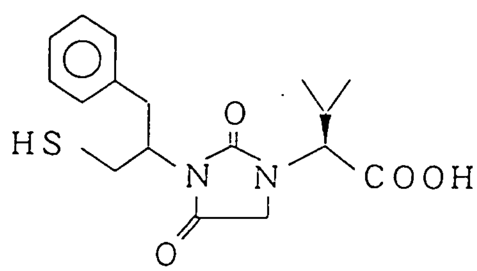

The same procedure as in Example 18 was repeated except that N-(ethoxycarbonylmethyl)valine methyl ester was used as the amino acid derivative, thereby the title compound was obtained.

| Elemental analysis for $C_{17}H_{22}N_2O_4S$: | | | | |
|---|---|---|---|---|
| | C % | H % | N % | S % |
| Calcd. | 58.27 | 6.33 | 7.99 | 9.15 |
| Found | 58.38 | 6.40 | 7.90 | 9.21 |

Property: oily
IR Spectrum ($\nu$KBr, $cm^{-1}$): 2560, 1770, 1710

NMR Spectrum (δ, $CDCl_3$):
0.79 (3/2H, d, J = 7Hz), 0.91 (3/2H, d, J = 7Hz), 0.99 (3/2H, d, J = 7Hz), 1.05 (3/2H, d, J = 7Hz), 1.37 (1H, dd, J = 10Hz, J = 8Hz), 2.07-2.20 (1H, m), 2.86-2.98 (1H, m), 3.12-3.28 (3H, m), 3.69 (1/2H, d, J = 17Hz), 3.73 (1/2H, d, J = 17Hz), 4.09 (1/2H, d, J = 17Hz), 4.13 (1/2H, d, J = 17Hz), 4.28-4.41 (1H, m), 4.43 (1H, t, J = 9Hz), 7.14-7.26 (5H, m)

EXAMPLE 22

1-(1-carboxy-1-methylethyl)-3-(1-mercapto-3-phenyl-2-propyl)hydantoin

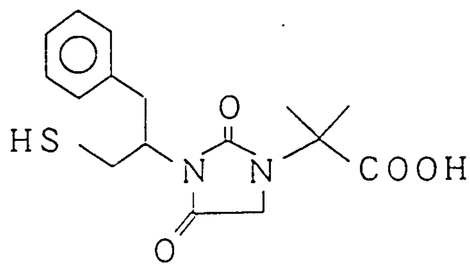

The same procedure as in Example 18 was repeated except that N-(1-methoxycarbonyl-1-methylethyl)-glycine ethyl ester was used as the amino acid derivative, thereby the title compound was obtained.

| Elemental analysis for $C_{16}H_{20}N_2O_4S$: | | | | |
|---|---|---|---|---|
| | C % | H % | N % | S % |
| Calcd. | 57.13 | 5.99 | 8.33 | 9.53 |
| Found | 57.08 | 5.92 | 8.40 | 9.60 |

Property: oily
IR Spectrum (νKBr, $cm^{-1}$): 2560, 1764, 1706
NMR Spectrum (δ, $CDCl_3$):
1.39 (1H, dd, J = 9Hz, J = 8Hz), 1.53 (3H, s), 1.59 (3H, s), 2.82-2.93 (1H, m), 3.10-3.25 (3H, m), 3.80 (1H, d, J = 17Hz), 3.87 (1H, d, J = 17Hz), 4.22-4.36 (1H, m), 7.15-7.29 (5H, m)

EXAMPLE 23

1-(1-carboxycyclopentyl)-3-(1-mercapto-3-phenyl-2-propyl)hydantoin

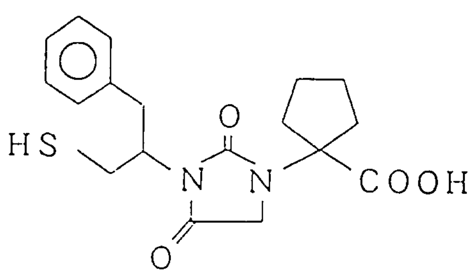

The same procedure as in Example 18 was repeated except that N-(1-methoxycarbonylcyclopentyl)-glycine methyl ester was used as the amino acid derivative, thereby the title compound was obtained.

| Elemental analysis for $C_{18}H_{22}N_2O_4S$: | | | | |
|---|---|---|---|---|
| | C % | H % | N % | S % |
| Calcd. | 59.65 | 6.12 | 7.73 | 8.85 |
| Found | 59.59 | 6.02 | 7.80 | 8.94 |

Property: oily
IR Spectrum ($\nu$KBr, $cm^{-1}$): 2570, 1770, 1708
NMR Spectrum ($\delta$, $CDCl_3$):
1.39 (1H, dd, J=9Hz, J=8Hz), 1.62-1.98 (4H, m), 2.12-2.38 (4H, m), 2.81-2.91 (1H, m), 3.10-3.28 (3H, m), 3.78 (1H, d, J=17Hz), 3.85 (1H, d, J=17Hz), 4.25-4.38 (1H, m), 7.15-7.29 (5H, m)

EXAMPLE 24

1-(1-carboxycyclohexyl)-3-(1-mercapto-3-phenyl-2-propyl) hydantoin

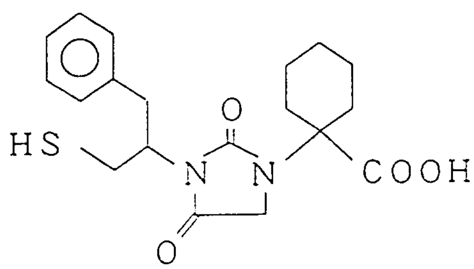

The same procedure as in Example 18 was repeated except that N-(1-methoxycarbonylcyclohexyl)-glycine ethyl ester was used as the amino acid derivative, thereby the title compound was obtained.

| Elemental analysis for $C_{19}H_{24}N_2O_4S$: | | | | |
|---|---|---|---|---|
| | C % | H % | N % | S % |
| Calcd. | 60.62 | 6.43 | 7.44 | 8.52 |
| Found | 60.51 | 6.38 | 7.51 | 8.59 |

Property: oily
IR Spectrum ($\nu$KBr, $cm^{-1}$): 2560, 1770, 1708
NMR Spectrum ($\delta$, $CDCl_3$):
1.20-1.45 (3H, m), 1.39 (1H, dd, J=6Hz, J=5Hz), 1.58-1.78 (3H, m), 1.86-2.00 (2H, m), 2.21-2.34 (2H, m), 3.82-3.94 (1H, m), 3.11-3.28 (3H, m), 3.78 (1H, d, J=18Hz), 3.86 (1H, d, J=18Hz), 4.25-4.38 (1H, m), 7.16-7.28 (5H,m)

EXAMPLE 25

1-[(1R)-1-carboxy-2-methylpropyl]-3-(1-mercapto-3-phenyl-2-propyl)hydantoin

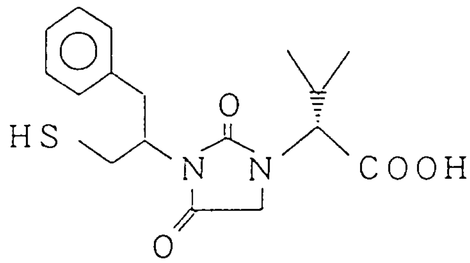

The same procedure as in Example 18 was repeated except that D-N-(ethoxycarbonylmethyl)valine methyl ester was used as the amino acid derivative, thereby the title compound was obtained.

| Elemental analysis for $C_{17}H_{22}N_2O_4S$: | | | | |
|---|---|---|---|---|
| | C % | H % | N % | S % |
| Calcd. | 58.27 | 6.33 | 7.99 | 9.15 |
| Found | 58.19 | 6.39 | 8.06 | 9.24 |

Property: oily
IR Spectrum ($\nu$KBr, $cm^{-1}$): 2560, 1772, 1710
NMR Spectrum ($\delta$, $CDCl_3$):
0.78 (3/2H, d, J = 7Hz), 0.90 (3/2H, d, J = 7Hz), 0.99 (3/2H, d, J = 7Hz), 1.05 (3/2H, d, J = Hz), 1.37 (1H, dd, J = 10Hz, J = 8Hz), 2.06-2.22 (1H, m), 2.85-2.98 (1H, m), 3.11-3.26 (3H, m), 3.69 (1/2H, d, J = 17Hz), 3.73 (1/2H, d, J = 17Hz), 4.15 (1/2H, d, J = 17Hz), 4.10 (1/2H, d, J = 17Hz), 4.28-4.38 (1H, m), 4.43 (1H, t, J = 10Hz), 7.13-7.26 (5H, m)

EXAMPLE 26

1-[(1S)-1-carboxymethyl]-3-(1-mercapto-3-phenyl-2-propyl) hydantoin

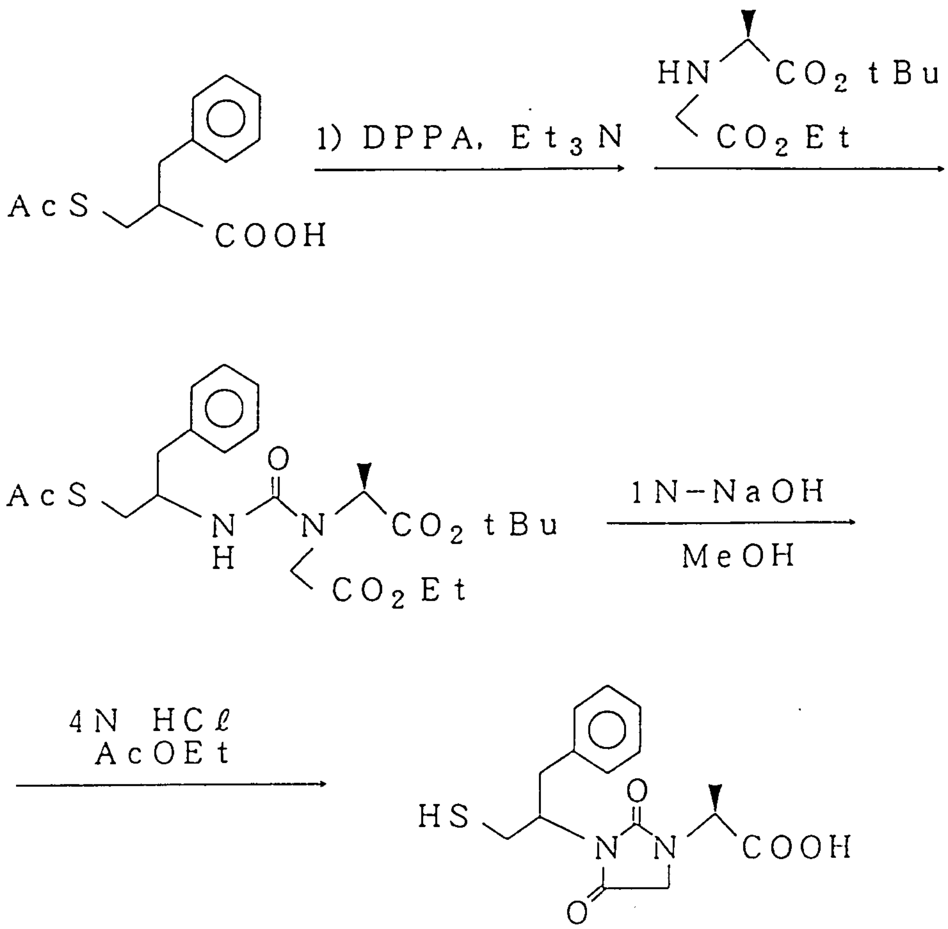


(1) N-(1-acetylthio-3-phenyl-2-propyl)N'-[(1S)-1-t-butoxycarbonylethyl]-N'-(ethoxycarbonylmethyl)urea:

The same procedure as in Example 1(1) was repeated using 1.16 g (5 mmol) of N-(ethoxycarbonylmethyl)alanine t-butyl ester as the amino acid derivative, thereby the title compound was obtained in a yield of 1.76 g (75%).
NMR Spectrum (δ, $CDCl_3$):
1.24-1.36 (6H, m), 1.43 (9/2H, s), 1.45 (9/2H, s), 2.35 (3/2H, s), 2.36 (3/2H, s), 2.68-2.81 (1H, m), 2.95-3.12 (3H, m), 3.79-4.00 (2H, m), 4.10-4.23 (3H, m), 4.54-4.68 (1H, m), 5.33 (1H, t, J=6Hz), 7.20-7.32 (5H, m)

(2) 1-[(1S)-1-carboxyethyl]-3-(1-mercapto-3-phenyl-2-propyl)hydantoin:

To a solution of 1.67 g (3.6 mmol) of N-(1-acetylthio-3-phenyl-2-propyl)N'-[(1S)-1-t-butoxycarbonylethyl]-N'-(ethoxycarbonylmethyl)urea in 30 ml of methanol was added 10 ml of an aqueous solution containing 2.9 g (7 mmol) of NaOH while cooling with ice, followed by stirring at room temperature for 2 hours. After removing the methanol under reduced pressure, 20 ml of water and 20 ml of 1N HCl was added to the residue, followed by extraction with chloroform. The organic layer was dried over anhydrous sodium sulfate, and the solvent was removing under reduced pressure. To the residues was added a mixture of 4N HCl/ethyl acetate while cooling with ice, followed by stirring at the same temperature for 3 hours. After removing the solvent under reduced pressure, residue was purified by silica gel chromatography to give the title compound in a yield of 0.79 g (68%).

| Elemental analysis for $C_{15}H_{18}N_2O_4S$: | | | | |
|---|---|---|---|---|
| | C % | H % | N % | S % |
| Calcd. | 55.89 | 5.63 | 8.69 | 9.94 |
| Found | 55.96 | 5.60 | 8.73 | 9.88 |

Property: oily
IR Spectrum ($\nu$KBr, $cm^{-1}$): 2575, 1770, 1712
NMR Spectrum ($\delta$, $CDCl_3$):
1.35-1.43 (1H, m), 1.47 (3H, t, J = 8Hz), 2.82-2.96 (1H, m), 3.10-3.28 (3H, m), 3.71 (1/2H, d, J = 12Hz), 3.76 (1/2H, d, J = 12Hz), 3.90 (1/2H, d, J = 15Hz), 3.96 (1/2H, d, J = 15Hz), 4.25-4.39 (1H, m), 4.82 (1H, q, J = 8Hz), 7.15-7.28 (5H, m)

EXAMPLE 27

1-[(1S)-1-carboxy-2-phenylethyl]-3-(1-mercapto-3-phenyl-2-propyl)hydantoin

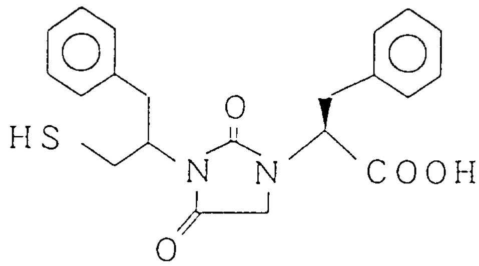

The same procedure as in Example 26 was repeated except that N-(ethoxycarbonylmethyl)-phenylalanine t-butyl ester was used as the amino acid derivative, thereby the title compound was obtained.

| Elemental analysis for $C_{21}H_{22}N_2O_4S$: | | | | |
|---|---|---|---|---|
| | C % | H % | N % | S % |
| Calcd. | 63.30 | 5.57 | 7.03 | 8.05 |
| Found | 63.41 | 5.50 | 6.99 | 8.09 |

Property: oily
IR Spectrum ($\nu$KBr, $cm^{-1}$): 2560, 1770, 1710
NMR Spectrum ($\delta$, $CDCl_3$):
1.14 (1/2H, t, J = 6Hz), 1.23 (1/2H, t, J = 6Hz), 2.83-2.95 (1H, m), 2.97-3.20 (4H, m), 3.39-3.49 (1H, m), 3.58 (1/2H, d, J = 16Hz), 3.58 (1/2H, d, J = 16Hz), 3.65 (1/2H, d, J = 16Hz), 3.82 (1/2H, d, J = 16Hz), 3.87 (1/2H, d, J = 16Hz), 4.17-4.28 (1H, m), 5.00-5.08 (1H, m), 7.00-7.33 (10H, m)

EXAMPLE 28

1-[(1S)-1-carboxy-3-methylbutyl]-3-(1-mercapto-3-phenyl-2-propyl)hydantoin

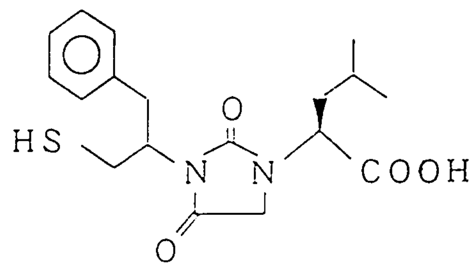

The same procedure as in Example 26 was repeated except that N-(ethoxycarbonylmethyl)leucine t-butyl ester was used as the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.63 | 7.69 | 8.80 |
| Found | 59.38 | 6.57 | 7.62 | 8.71 |

Property: oily
IR Spectrum ($\nu$KBr, $cm^{-1}$): 2570, 1768, 1710
NMR Spectrum ($\delta$, $CDCl_3$):
0.84-0.99 (6H, m), 1.35-1.48 (2H, m), 1.56-1.80 (2H, m), 2.86-2.98 (1H, m), 3.11-3.26 (3H, m), 3.60 (1/2H, d, J = 17Hz), 3.62 (1/2H, d, J = 17Hz), 3.96 (1/2H, d, J = 17Hz), 4.00 (1/2H, d, J = 17Hz), 4.26-4.40 (1H, m), 4.72-4.85 (1H, m), 7.14-7.26 (5H, m)

EXAMPLE 29

1-[(1S)-1-carboxy-2-methylbutyl]-3-(1-mercapto-3-phenyl-2-propyl)hydantoin

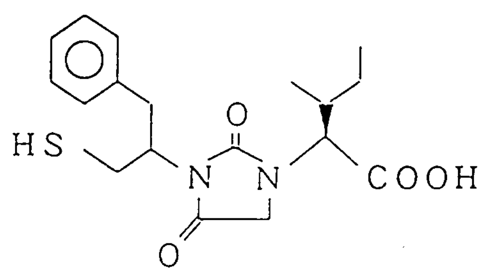

The same procedure as in Example 26 was repeated except that N-(ethoxycarbonylmethyl)isoleucine t-butyl ester was used as the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.63 | 7.69 | 8.80 |
| Found | 59.24 | 6.57 | 7.76 | 8.72 |

Property: oily
IR Spectrum ($\nu$KBr, $cm^{-1}$): 2560, 1770, 1710
NMR Spectrum ($\delta$, $CDCl_3$):
0.80-1.02 (6H, m), 1.05-1.41 (3H, m), 1.84-1.95 (1H, m), 2.85-2.99 (1H, m), 3.11-3.28 (3H, m), 3.64 (1/2H, d, J=14Hz), 3.69 (1/2H, d, J=14Hz), 4.09 (1/2H, d, J=14Hz), 4.15 (1/2H, d, J=14Hz), 4.28-4.34 (1H, m), 4.49 (1/2H, d, J=9Hz), 4.53 (1/2H, d, J=9Hz), 7.14-7.28 (5H, m)

EXAMPLE 30

1-[2-carboxyethyl]-3-(1-mercapto-3-phenyl-2-propyl)hydantoin

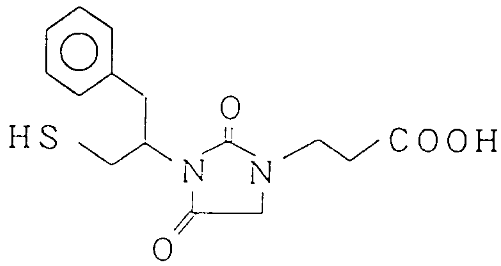

The same procedure as in Example 26 was repeated except that N-(2-t-butoxycarbonylmethyl)glycine ethyl ester was used as the amino acid derivative, thereby the title compound was obtained.

| Elemental analysis for $C_{15}H_{18}N_2O_4S$: | | | | |
|---|---|---|---|---|
| | C % | H % | N % | S % |
| Calcd. | 55.89 | 5.63 | 8.69 | 9.94 |
| Found | 55.94 | 5.67 | 8.72 | 9.99 |

Property: oily
IR Spectrum ($\nu$KBr, $cm^{-1}$): 2560, 1770, 1704
NMR Spectrum ($\delta$, $CDCl_3$):
1.36 (1/2H, d, J=7Hz), 1.39 (1/2H, d, J=7Hz), 2.58-2.62 (2H, m), 2.86-2.95 (1H, m), 3.10-3.24 (3H, m), 3.59 (2H, t, J=6Hz), 3.78 (1H, d, J=18Hz), 3.86 (1H, d, J=18Hz), 4.25-4.35 (1H, m), 7.14-7.29 (5H, m)

EXAMPLE 31

1-[(1S)-1-carboxy-2-(3-indolyl)ethyl]-3-(1-mercapto-3-phenyl-2-propyl)hydantoin

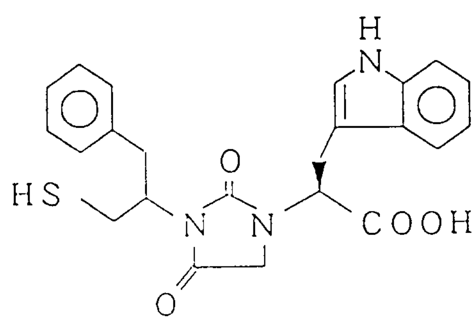

The same procedure as in Example 26 was repeated except that N-(ethoxycarbonylmethyl)tryptophan t-butyl ester was used as the amino acid derivative, thereby the title compound was obtained.

| Elemental analysis for $C_{23}H_{23}N_3O_4S$: | | | | |
|---|---|---|---|---|
| | C % | H % | N % | S % |
| Calcd. | 63.14 | 5.30 | 9.60 | 7.33 |
| Found | 63.08 | 5.32 | 9.59 | 7.36 |

Property: oily

IR Spectrum ($\nu$KBr, $cm^{-1}$): 2560, 1768, 1704

NMR Spectrum ($\delta$, $CDCl_3$):

1.15-1.27 (1H, m), 2.75-2.88 (1H, m), 3.04-3.27 (3H, m), 3.46-3.54 (1H, m), 3.61 (1/2H, d, J = 17Hz), 3.65 (1/2H, d, J = 17Hz), 3.85 (1/2H, d, J = 17Hz), 3.87 (1/2H, d, J = 17Hz), 4.20-4.31 (1H, m), 5.10-5.17 (1H, m), 5.65-6.10 (1H, m), 6.85-6.93 (1H, m), 7.04-7.25 (7H, m), 7.36 (1H, d, J = 8Hz), 7.57 (1H, dd, J = 11Hz, J = 8Hz), 8.00-8.05 (1H, m)

EXAMPLE 32

1-[(1S)-5-amino-1-carboxypentyl]-3-(1-mercapto-3-phenyl-2-propyl)hydantoin hydrochloride:

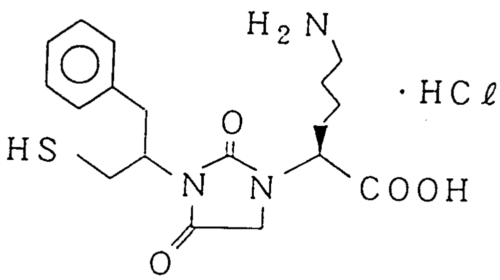

The same procedure as in Example 26 was repeated except that $N^2$-(ethoxycarbonylmethyl)-$N^6$-(t-butoxycarbonyl)lysine t-butyl ester was used as the amino acid derivative, thereby the title compound was obtained.

| Elemental analysis for $C_{18}H_{26}ClN_3O_4S$: | | | | |
|---|---|---|---|---|
| | C % | H % | N % | S % |
| Calcd. | 51.98 | 6.30 | 6.74 | 7.71 |
| Found | 52.04 | 6.40 | 6.69 | 7.74 |

Property: oily

IR Spectrum ($\nu$KBr, $cm^{-1}$): 2560, 1766, 1704

NMR Spectrum ($\delta$, $CDCl_3$):

1.22-1.42 (2H, m), 1.58-1.88 (3H, m), 1.92-2.40 (1H, m), 2.85-2.98 (3H, m), 3.08-3.25 (3H, m), 3.75-4.03 (2H, m), 4.23-4.35 (1H, m), 4.52-4.58 (1H, m), 7.15-7.27 (5H, m)

EXAMPLE 33

1-(3-carboxypropyl)-3-(1-mercapto-3-phenyl-2-propyl)hydantoin

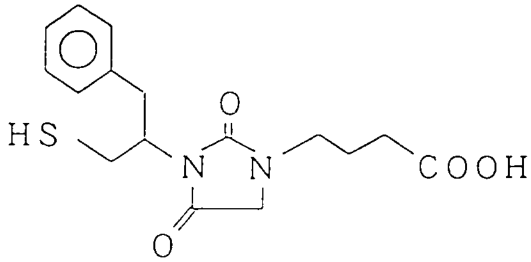

The same procedure as in Example 26 was repeated except that N-(3-t-butoxycarbonylpropyl)glycine ethyl ester was used as the amino acid derivative, thereby the title compound was obtained.

| Elemental analysis for $C_{16}H_{20}N_2O_4S$: | | | | |
|---|---|---|---|---|
| | C % | H % | N % | S % |
| Calcd. | 57.13 | 5.99 | 8.33 | 9.53 |
| Found | 57.20 | 6.04 | 8.38 | 9.49 |

Property: oily
IR Spectrum ($\nu$KBr, $cm^{-1}$): 2560, 1768, 1708
NMR Spectrum ($\delta$, $CDCl_3$):
1.39 (1H, dd, J=18Hz, J=7Hz), 1.76-1.88 (2H, m), 2.29 (2H, t, J=7Hz), 2.87-2.96 (1H, m), 3.10-3.28 (3H, m), 3.31-3.42 (2H, m), 3.68 (1H, d, J=17Hz), 3.75 (1H, d, J=17Hz), 4.26-4.38 (1H, m), 7.15-7.30 (5H, m)

EXAMPLE 34

1-(carboxymethyl-3-(1-mercapto-3-phenyl-2-propyl)hydantoin-5-spirocyclohexane

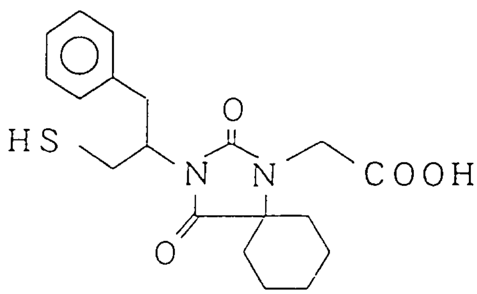

The same procedure as in Example 26 was repeated except that N-(1-methoxycarbonylcyclohexyl)-glycine t-butyl ester was used as the amino acid derivative, thereby the title compound was obtained.

| Elemental analysis for $C_{19}H_{24}N_2O_4S$: | | | | |
|---|---|---|---|---|
| | C % | H % | N % | S % |
| Calcd. | 60.62 | 6.43 | 7.44 | 8.52 |
| Found | 60.57 | 6.44 | 7.39 | 8.43 |

Property: oily
IR Spectrum (νKBr, $cm^{-1}$): 2560, 1770, 1708
NMR Spectrum (δ, $CDCl_3$):
1.00-1.43 (4H, m), 1.40 (1H, d, J=10Hz, J=8Hz), 1.07-2.04 (6H, m), 2.90-2.97 (1H, m), 3.11-3.30 (3H, m), 3.94 (1H, d, J=18Hz), 4.01 (1H, d, J=18Hz), 4.28-4.40 (1H, m), 7.16-7.26 (5H, m)

EXAMPLE 35

1-[(1S)-1-carboxyethyl]-3-(1-mercapto-3-phenyl-2-propyl) hydantoin-5-spirocyclohexane

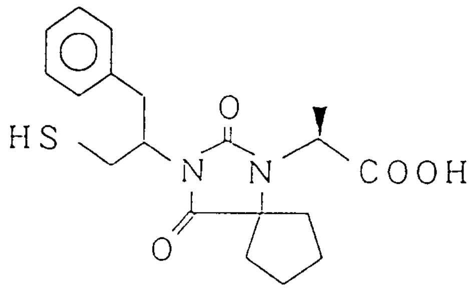

The same procedure as in Example 26 was repeated except that N-(1-methoxycarbonyl-1-cyclopentyl)-alanine t-butyl ester was used as the amino acid derivative, thereby the title compound was obtained.

| Elemental analysis for $C_{19}H_{24}N_2O_4S$: | | | | |
|---|---|---|---|---|
| | C % | H % | N % | S % |
| Calcd. | 60.62 | 6.43 | 7.44 | 8.52 |
| Found | 60.69 | 6.47 | 7.41 | 8.57 |

Property: oily
IR Spectrum (νKBr, $cm^{-1}$): 2560, 1764, 1706
NMR Spectrum (δ, $CDCl_3$):
1.34-1.43 (1H, m), 1.45-2.05 (8H, m), 1.57 (3/2H, d, J=6Hz), 1.67 (3/2H, d, J=6Hz), 2.85-3.00 (1H, m), 3.08-3.28 (3H, m), 3.72-3.81 (1H, m), 4.21-4.37 (1H, m), 7.14-7.26 (5H, m)

EXAMPLE 36

1-(carboxymethyl)-5-(1-methylethyl)-3-(1-mercapto-3-phenyl-2-propyl)hydantoin

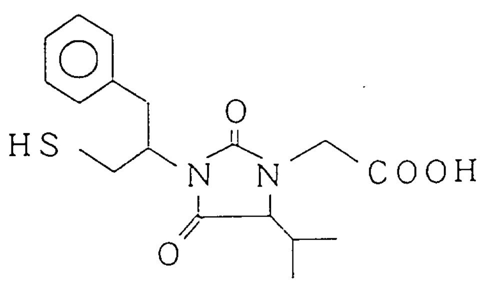

The same procedure as in Example 26 was repeated except that N-(t-butoxycarbonylmethyl)valine methyl ester was used as the amino acid derivative, thereby the title compound was obtained.

| Elemental analysis for $C_{17}H_{22}N_2O_4S$: | | | | |
|---|---|---|---|---|
| | C % | H % | N % | S % |
| Calcd: | 58.27 | 6.33 | 7.99 | 9.15 |
| Found | 58.31 | 6.29 | 8.03 | 9.19 |

Property: oily
IR Spectrum ($\nu$KBr, $cm^{-1}$): 2560, 1770, 1710
NMR Spectrum ($\delta$, $CDCl_3$):
0.64 (3/2H, d, J=7Hz), 0.85 (3/2H, d, J=7Hz), 0.92 (3/2H, d, J=7Hz), 1.04 (3/2H, d, J=7Hz), 1.35 (1/2H, dd, J=9Hz, J=8Hz), 1.46 (1/2H, dd, J=9Hz, J=8Hz), 1.99-2.12 (1H, m), 2.83-2.92 (1H, m), 3.08-3.28 (3H, m), 3.71 (1/2H, d, J=18Hz), 3.74 (1/2H, d, J=18Hz), 3.87 (1/2H, d, J=20Hz), 3.88 (1/2H, d, J=20Hz), 4.30-4.45 (1H, m), 4.56 (1H, dd, J=21Hz, J=19Hz), 7.16-7.26 (5H, m)

EXAMPLE 37

1-(carboxymethyl)-3-(1-mercapto-3-phenyl-2-propyl)-5-(phenylmethyl)hydantoin

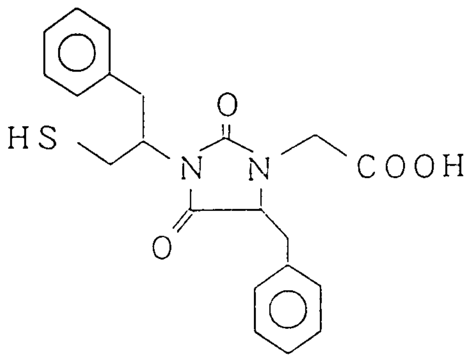

The same procedure as in Example 26 was repeated except that N-(t-butoxycarbonylmethyl)-phenylalanine ethyl ester was used as the amino acid derivative, thereby the title compound was obtained.

| Elemental analysis for $C_{21}H_{22}N_2O_4S$: | | | | |
|---|---|---|---|---|
| | C % | H % | N % | S % |
| Calcd. | 63.30 | 5.57 | 7.03 | 8.05 |
| Found | 63.37 | 5.67 | 7.04 | 8.11 |

Property: oily
IR Spectrum ($\nu$KBr, $cm^{-1}$): 2560, 1770, 1712
NMR Spectrum ($\delta$, $CDCl_3$):
1.02 (1/2H, t, J=9Hz), 1.15 (1/2H, t, J=9Hz), 2.61-2.95 (2H, m), 3.03-3.17 (4H, m), 3.35 (1/2H, d, J=18Hz), 3.45 (1/2H, d, J=18Hz), 4.20-4.28 (2H, m), 4.39 (1H, t, J=16Hz), 7.09-7.31 (10H, m)

EXAMPLE 38

1-(carboxymethyl)-3-(1-mercapto-3-phenyl-2-propyl)-5-[[4-(phenylmethoxy)phenyl]methyl]hydantoin

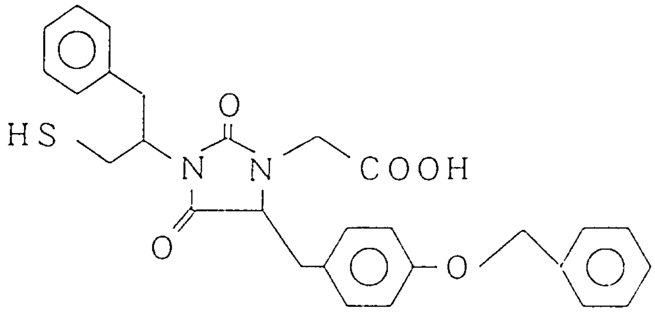

The same procedure as in Example 26 was repeated except that O-benzyl-N-(ethoxycarbonylmethyl)-tyrosine t-butyl ester was used as the amino acid derivative, thereby the title compound was obtained.

| Elemental analysis for $C_{28}H_{28}N_2O_5S$: | | | | |
|---|---|---|---|---|
| | C % | H % | N % | S % |
| Calcd. | 66.65 | 5.59 | 5.55 | 6.35 |
| Found | 66.68 | 5.61 | 5.51 | 6.40 |

Property: oily
IR Spectrum ($\nu$KBr, $cm^{-1}$): 2560, 1772, 1714
NMR Spectrum ($\delta$, $CDCl_3$):
1.04 (1/2H, t, J=9Hz), 1.16 (1/2H, t, J=9Hz), 2.60 (1/2H, d, J=7Hz), 2.65 (1/2H, d, J=7Hz), 2.99-3.08 (1H, m), 2.82 (1/2H, d, J=7Hz), 2.87 (1/2H, d, J=7Hz), 3.02-3.18 (3H, m), 3.38 (1/2H, d, J=18Hz), 3.47 (1/2H, d, J=18Hz), 4.16-4.35 (2H, m), 4.33 (1/2H, d, J=18Hz), 4.44 (1/2H, d, J=18Hz), 5.01 (2H, d, J=3Hz), 6.85-6.90 (2H, m), 7.00 (2H, d, J=8Hz), 7.11-7.40 (10H, m)

EXAMPLE 39

1-(carboxymethyl)-5-[2-(3-indolyl)ethyl]-3-(1-mercapto-3-phenyl-2-propyl)hydantoin

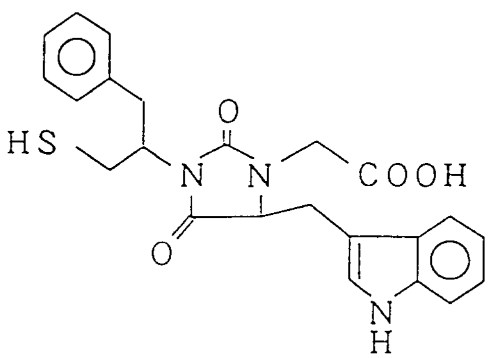

The same procedure as in Example 26 was repeated except that N-(t-butoycarbonylmethyl)tryptophan methyl ester was used as the amino acid derivative, thereby the title compound was obtained.

| Elemental analysis for $C_{23}H_{23}N_3O_4S$: | | | | |
|---|---|---|---|---|
| | C % | H % | N % | S % |
| Calcd. | 63.14 | 5.30 | 9.60 | 7.33 |
| Found | 63.19 | 5.38 | 9.66 | 7.29 |

Property: oily
IR Spectrum ($\nu$KBr, $cm^{-1}$): 2650, 1770, 1706
NMR Spectrum ($\delta$, $CDCl_3$):
0.97 (1/2H, t, J=9Hz), 1.08 (1/2H, t, J=9Hz), 2.67-2.77 (1H, m), 2.91-3.91 (3H, m), 3.23-3.33 (1H, m), 3.53 (1/2H, d, J=16Hz), 3.59 (1/2H, d, J=16Hz), 4.18-4.35 (3H, m), 6.88-6.94 (1H, m), 7.09-7.35 (8H, m), 7.52 (1H, t, J=9Hz), 8.15 (1H, brs)

EXAMPLE 40

1-(carboxymethyl)-5,5-dimethyl-3-(1-mercapto-3-phenyl-2-propyl)hydantoin

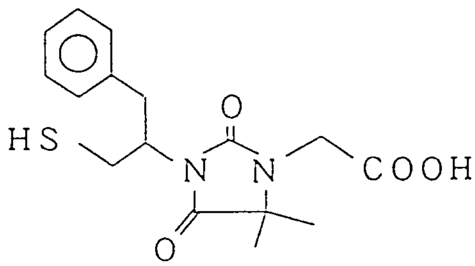

The same procedure as in Example 26 was repeated except that N-(1-methoxycarbonyl-1-methylethyl)-glycine t-butyl ester was used as the amino acid derivative, thereby the title compound was obtained.

| Elemental analysis for $C_{16}H_{20}N_2O_4S$: | | | | |
|---|---|---|---|---|
| | C % | H % | N % | S % |
| Calcd. | 57.13 | 5.99 | 8.33 | 9.53 |
| Found | 57.16 | 6.03 | 8.37 | 9.44 |

Property: oily
IR Spectrum ($\nu$KBr, $cm^{-1}$): 2775, 1774, 1710
NMR Spectrum ($\delta$, $CDCl_3$):
1.11 (3H, s), 1.26 (3H, s), 1.40 (1H, dd, J=10Hz, 8Hz), 2.89-3.00 (1H, m), 3.05-3.29 (3H, m), 3.94 (1H, d, J=18Hz), 4.02 (1H, d, J=18Hz), 4.27-4.40 (1H, m), 7.14-7.29 (5H, m)

EXAMPLE 41

1-(carboxymethyl)-3-(1-mercapto-3-phenyl-2-propyl)hydantoin-5-spirocyclopentane

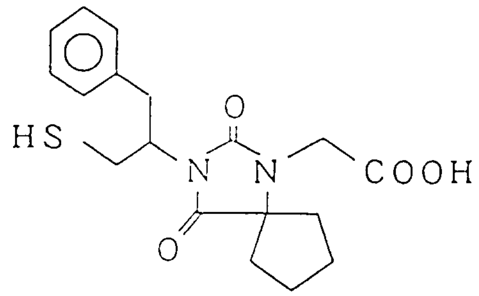

The same procedure as in Example 26 was repeated except that N-(1-methoxycarbonylcyclopentyl)-glycine t-butyl ester was used as the amino acid derivative, thereby the title compound was obtained.

| Elemental analysis for $C_{18}H_{22}N_2O_4S$: | | | | |
|---|---|---|---|---|
| | C % | H % | N % | S % |
| Calcd. | 59.65 | 6.12 | 7.73 | 8.85 |
| Found | 59.71 | 6.16 | 7.70 | 8.88 |

Property: oily
IR Spectrum ($\nu$KBr, $cm^{-1}$): 2570, 1770, 1704
NMR Spectrum ($\delta$, $CDCl_3$):
1.39 (1H, dd, J = 9Hz, 8Hz), 1.56-2.07 (8H, m), 2.87-2.99 (1H, m), 3.04-3.30 (3H, m), 3.95 (1H, d, J = 18Hz), 4.02 (1H, d, J = 18Hz), 4.26-4.38 (1H, m), 7.14-7.30 (5H, m)

EXAMPLE 42

5-(2-butyl)-1-(carboxymethyl)-3-(1-mercapto-3-phenyl-2-propyl)hydantoin

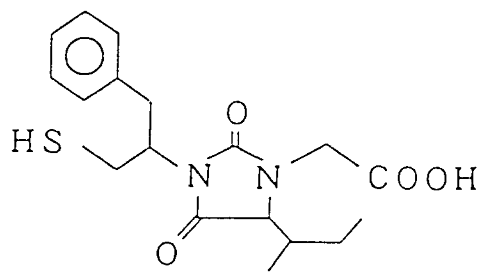

The same procedure as in Example 26 was repeated except that N-(t-butoxycarbonylmethyl)isoleucine methyl ester was used as the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.64 | 7.69 | 8.80 |
| Found | 59.34 | 6.68 | 7.65 | 8.84 |

Property: oily
IR Spectrum (νKBr, $cm^{-1}$): 2575, 1770, 1708
NMR Spectrum (δ, $CDCl_3$):
0.54 (3/2H, d, J=7Hz), 0.73-0.97 (9/2H, m), 1.21-1.80 (4H, m), 2.77-3.28 (4H, m), 3.64-4.01 (2H, m), 4.29-4.65 (2H, m), 7.13-7.32 (5H, m)

EXAMPLE 43

1-(carboxymethyl)-5-(1-hydroxyethyl)-3-(1-mercapto-3-phenyl-2-propyl)hydantoin

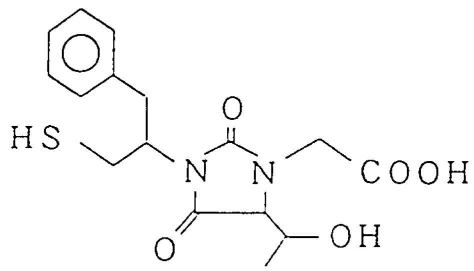

The same procedure as in Example 26 was repeated except that N-(t-butoxycarbonylmethyl)threonine methyl ester was used as the amino acid derivative, thereby the title compound was obtained.

| Elemental analysis for $C_{16}H_{20}N_2O_5S$: | | | | |
|---|---|---|---|---|
| | C % | H % | N % | S % |
| Calcd. | 54.53 | 5.72 | 7.95 | 9.10 |
| Found | 54.55 | 5.70 | 7.96 | 9.13 |

Property: oily
IR Spectrum (νKBr, $cm^{-1}$): 2575, 1772, 1708
NMR Spectrum (δ, $CDCl_3$):
1.04 (3/2H, d, J=7Hz), 1.24 (3/2H, d, J=7Hz), 1.42 (1/2H, t, J=8Hz), 1.51 (1/2H, t, J=8Hz), 2.78-2.91 (1H, m), 3.09-3.28 (3H, m), 3.69-4.44 (5H, m), 7.13-7.35 (5H, m)

EXAMPLE 44

1-(carboxymethyl)-3-(1-mercapto-3-phenyl-2-propyl)hydantoin-5-spiro-2'-indane

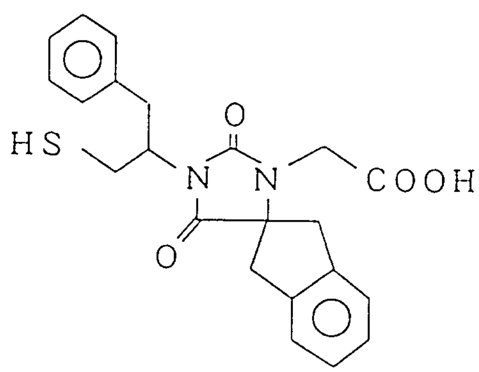

The same procedure as in Example 26 was repeated except that N-(2-ethoxycarbonyl-2-indanyl)glycine t-butyl ester was used as the amino acid derivative, thereby the title compound was obtained.

| Elemental analysis for $C_{22}H_{22}N_2O_4S$: | | | | |
|---|---|---|---|---|
| | C % | H % | N % | S % |
| Calcd. | 64.37 | 5.46 | 6.82 | 7.81 |
| Found | 64.34 | 5.41 | 6.85 | 7.79 |

Property: oily
IR Spectrum ($\nu$KBr, $cm^{-1}$): 2565, 1776, 1710
NMR Spectrum ($\delta$, $CDCl_3$):
1.42 (1H, dd, J=10Hz, J=8Hz), 2.92-3.32 (7H, m), 3.51 (1H, d, 17Hz), 3.71 (1H, d, J=18Hz), 3.84 (1H, d, J=18Hz), 4.32-4.44 (1H, m), 7.12-7.33 (9H, m)

EXAMPLE 45

3-(1-mercapto-3-phenyl-2-propyl)-1-piperidinylhydantoin

The same procedure as in Example 1 was repeated except that (1-ethoxycarbonylmethyl)amino piperidine was used as the amino acid derivative, thereby the title compound was obtained.

| Elemental analysis for $C_{17}H_{23}N_3O_2S$: | | | | |
|---|---|---|---|---|
| | C % | H % | N % | S % |
| Calcd. | 61.23 | 6.95 | 12.60 | 9.61 |
| Found | 61.49 | 7.04 | 12.87 | 9.37 |

Melting point: 91.3-93.0 ° C
IR Spectrum ($\nu$KBr, $cm^{-1}$): 2552, 1768, 1706
$^1$H-NMR Spectrum ($\delta$, $CDCl_3$):
1.34-1.40 (3H, m), 1.62-1.70 (4H, m), 2.80-2.97 (5H, m), 3.11-3.24 (3H, m), 3.71 (1H, d, J=17Hz), 3.79 (1H, d, J=17Hz), 4.26-4.37 (1H, m), 7.16-7.29 (5H, m)

EXAMPLE 46

3-(1-mercapto-3-phenyl-2-propyl)-1-(4-morpholinyl)hydantoin

The same procedure as in Example 1 was repeated except that 4-(ethoxycarbonylmethyl)-aminomorpholine was used as the amino acid derivative, thereby the title compound was obtained.

| Elemental analysis for $C_{16}H_{21}N_3O_3S$: | | | | |
|---|---|---|---|---|
| | C % | H % | N % | S % |
| Calcd. | 57.29 | 6.31 | 12.53 | 9.56 |
| Found | 57.44 | 6.53 | 12.77 | 9.29 |

Property: oily
IR Spectrum ($\nu$KBr, $cm^{-1}$): 2560, 1774, 1714
$^1$H-NMR Spectrum ($\delta$, $CDCl_3$):
1.38 (1H, dd, J=10Hz, 7Hz), 2.87-2.98 (5H, m), 3.11-3.25 (3H, m), 3.74-3.75 (4H, m), 3.73 (1H, d, J=17Hz), 3.80 (1H, d, J=17Hz), 4.27-4.37 (1H, m), 7.15-7.27 (5H, m)

EXAMPLE 47

1-(diethylamino)-3-(1-mercapto-3-phenyl-2-propyl)-hydantoin

The same procedure as in Example 1 was repeated except that N-(diethylamino)glycine ethyl ester was used as the amino acid derivative, thereby the title compound was obtained.

| Elemental analysis for $C_{16}H_{23}N_3O_2S$: | | | | |
|---|---|---|---|---|
| | C % | H % | N % | S % |
| Calcd. | 59.79 | 7.21 | 13.07 | 9.97 |
| Found | 59.99 | 7.11 | 12.91 | 10.13 |

Property: oily
IR Spectrum ($\nu$KBr, $cm^{-1}$): 2564, 1774, 1714
$^1$H-NMR Spectrum ($\delta$, $CDCl_3$):
0.94 (6H, t, J=7Hz), 1.37 (1H, dd, J=10Hz, 7Hz), 2.80 (4H, q, J=7Hz), 2.95 (1H, ddd, J=14Hz, 7Hz, 5Hz), 3.10-3.29 (3H, m), 3.61 (1H, d, J=7Hz), 3.67 (1H, d, J=7Hz), 4.30-4.41 (1H, m), 7.17-7.28 (5H, m)

EXAMPLE 48

1-[bis(phenylmethyl)]amino-3-(1-mercapto-3-phenyl-2-propyl)-hydantoin

The same procedure as in Example 1 was repeated except that N-[bis(phenylmethyl)]aminoglycine ethyl ester was used as the amino acid derivative, thereby the title compound was obtained.

| Elemental analysis for $C_{26}H_{27}N_3O_2S$: | | | | |
|---|---|---|---|---|
| | C % | H % | N % | S % |
| Calcd. | 70.09 | 6.11 | 9.43 | 7.20 |
| Found | 69.89 | 6.24 | 9.58 | 7.01 |

Property: oily
IR Spectrum ($\nu$KBr, $cm^{-1}$): 2568, 1770, 1712
$^1$H-NMR Spectrum ($\delta$, $CDCl_3$):
1.17 (1H, dd, J=9Hz, 8Hz), 2.79 (1H, ddd, J=14Hz, 8Hz, 5Hz), 2.95-3.19 (5H, m), 4.14-4.28 (5H, m), 7.08-7.30 (15H, m)

EXAMPLE 49

1-(carboxymethyl)-5,5-diethyl-3-(1-mercapto-3-phenyl-2-propyl)hydantoin

The same procedure as in Example 1 was repeated except that N-(3-ethoxycarbonyl-3-pentyl)glycine t-butyl ester was used as the amino acid derivative, thereby the title compound was obtained.

| Elemental analysis for $C_{18}H_{24}N_2O_4S$: | | | | |
|---|---|---|---|---|
| | C % | H % | N % | S % |
| Calcd. | 59.32 | 6.64 | 7.69 | 8.80 |
| Found | 59.09 | 6.79 | 7.43 | 8.99 |

Property: oily
IR Spectrum ($\nu$KBr, $cm^{-1}$): 2568, 1770, 1706
NMR Spectrum ($\delta$, $CDCl_3$):
0.43 (3H, t, J=7Hz), 0.74 (3H, t, J=7Hz), 1.35-1.55 (3H, m), 1.64-1.86 (2H, m), 2.86-2.97 (1H, m), 3.11-3.30

(3H, m), 3.86 (1H, d, J = 17Hz), 3.96 (1H, d, J = 17Hz), 4.40-4.51 (1H, m), 7.15-7.24 (5H, m)

EXAMPLE 50

1-(carboxymethyl)-3-(1-mercapto-3-phenyl-2-propyl)-hydantoin-5-spirocyclobutane

The same procedure as in Example 26 was repeated except that N-(1-ethoxycarbonylcyclobutyl)glycine t-butyl ester was used as the amino acid derivative, thereby the title compound was obtained.

| Elemental analysis for $C_{17}H_{20}N_2O_4S$: | | | | |
|---|---|---|---|---|
| | C % | H % | N % | S % |
| Calcd. | 58.60 | 5.79 | 8.04 | 9.20 |
| Found | 58.44 | 5.95 | 7.89 | 9.11 |

Property: oily
IR Spectrum ($\nu$KBr, $cm^{-1}$): 2568, 1772, 1712
NMR Spectrum ($\delta$, $CDCl_3$):
1.39 (1H, dd, J = 9Hz, J = 8Hz), 1.67-1.80 (1H, m), 2.05-2.40 (5H, m), 2.87-2.96 (1H, m), 3.09-3.28 (3H, m), 4.12 (1H, d, J = 12Hz), 4.17 (1H, d, J = 12Hz), 4.27-4.38 (1H, m), 7.14-7.23 (5H, m)

EXAMPLE 51

1-(carboxymethyl)-5-cyclopentyl-3-(1-mercapto-3-phenyl-2-propyl)hydantoin

The same procedure as in Example 26 was repeated except that N-(1-cyclohexyl-1-ethoxycarbonylmethyl)glycine t-butyl ester was used as the amino acid derivative, thereby the title compound was obtained.

| Elemental analysis for $C_{19}H_{24}N_2O_4S$: | | | | |
|---|---|---|---|---|
| | C % | H % | N % | S % |
| Calcd. | 60.62 | 6.43 | 7.44 | 8.52 |
| Found | 60.85 | 6.64 | 7.29 | 8.49 |

Property: oily
IR Spectrum ($\nu$KBr, $cm^{-1}$): 2568, 1770, 1710
NMR Spectrum ($\delta$, $CDCl_3$):
1.34-1.68 (9H, m), 2.02-2.25 (1H, m), 2.83-2.92 (1H, m), 3.09-3.27 (3H, m), 3.71 (1/2H, d, J = 7Hz), 3.77 (1/2H, d, J = 7Hz), 3.99 (1/2H, d, J = 4Hz), 4.05 (1/2H, d, J = 4Hz), 4.30-4.47 (1H, m), 4.47 (1/2H, d, J = 18Hz), 4.59 (1/2H, d, J = 18Hz), 7.14-7.24 (5H, m)

EXAMPLE 52

1-(carboxymethyl)-5-phenyl-3-(1-mercapto-3-phenyl-2-propyl)hydantoin

The same procedure as in Example 26 was repeated except that N-(t-butoxycarbonylmethyl)-phenylglycine ethyl ester was used as the amino acid derivative, thereby the title compound was obtained.

| Elemental analysis for $C_{20}H_{20}N_2O_4S$: | | | | |
|---|---|---|---|---|
| | C % | H % | N % | S % |
| Calcd. | 62.48 | 5.24 | 7.29 | 8.34 |
| Found | 62.19 | 5.03 | 7.45 | 8.15 |

Property: oily
IR Spectrum (νKBr, $cm^{-1}$): 2568, 1774, 1714
NMR Spectrum (δ, $CDCl_3$):
1.40-1.48 (1H, m), 2.88-3.01 (1H, m), 3.10-3.43 (3H, m), 3.33 (1/2H, d, J=18Hz), 3.40 (1/2H, d, J=18Hz), 4.34-4.52 (1H, m), 4.49 (1/2H, d, J=11Hz), 4.56 (1/2H, d, J=11Hz), 4.94 (1/2H, s), 4.99 (1/2H, s), 6.85-6.87 (1H, m), 7.01-7.04 (1H, m), 7.12-7.20 (1H, m), 7.20-7.38 (7H, m)

EXAMPLE 53

1-(carboxymethyl)-5-(diphenylmethyl)-3-(1-mercapto-3-phenyl-2-propyl)hydantoin

The same procedure as in Example 26 was repeated except that N-(2,2-diphenyl-1-ethoxycarbonylethyl)glycine t-butyl ester was used as the amino acid derivative, thereby the title compound was obtained.

| Elemental analysis for $C_{27}H_{26}N_2O_4S$: | | | | |
|---|---|---|---|---|
| | C % | H % | N % | S % |
| Calcd. | 68.33 | 5.52 | 5.90 | 6.76 |
| Found | 68.09 | 5.73 | 5.76 | 6.95 |

Property: oily
IR Spectrum (νKBr, $cm^{-1}$): 2568, 1774, 1716
NMR Spectrum (δ, $CDCl_3$):
0.78-0.90 (1H, m), 2.46-2.61 (1H, m), 2.81-3.03 (3H, m), 3.12 (1/2H, d, J=18Hz), 3.21 (1/2H, d, J=18Hz), 4.06-4.19 (1H, m), 4.37-4.55 (2H, m), 4.84 (1/2H, d, J=5Hz), 4.87 (1/2H, d, J=5Hz), 7.06-7.40 (15H, m)

EXAMPLE 54

1-(carboxymethyl)-3(1-mercapto-3-phenyl-2-propyl)-5-(3-methylthio-1-propyl)hydantoin

The same procedure as in Example 26 was repeated except that N-(t-butoxycarbonylmethyl)methionine methyl ester was used as the amino acid derivative, thereby the title compound was obtained.

| Elemental analysis for $C_{17}H_{22}N_2O_4S$: | | | | |
|---|---|---|---|---|
| | C % | H % | N % | S % |
| Calcd. | 53.38 | 5.80 | 7.32 | 16.76 |
| Found | 53.12 | 6.01 | 7.14 | 16.98 |

Property: oily
IR Spectrum (νKBr, $cm^{-1}$): 2568, 1772, 1712
NMR Spectrum (δ, $CDCl_3$):
1.41-1.48 (1H, m), 1.60-2.50 (7H, m), 2.82-2.95 (1H, m), 3.07-3.28 (3H, m), 3.74 (1H, d, J=17Hz), 4.04-4.17 (1H, m), 4.29-4.57 (2H, m), 7.14-7.30 (5H, m)

EXAMPLE 55

1-(carboxymethyl)-5-(4-hydroxyphenyl)-3-(1-mercapto-3-phenyl-2-propyl)hydantoin

The same procedure as in Example 26 was repeated except that N-[1-ethoxycarbonyl-1-(4-hydroxyphenyl)methyl]-glycine t-butyl ester was used as the amino acid derivative, thereby the title compound was obtained.

| Elemental analysis for $C_{20}H_{20}N_2O_5S$: | | | | |
|---|---|---|---|---|
| | C % | H % | N % | S % |
| Calcd. | 59.99 | 5.03 | 7.00 | 8.01 |
| Found | 60.11 | 5.25 | 7.18 | 7.84 |

Property: oily
IR Spectrum ($\nu$KBr, $cm^{-1}$): 2552, 1626
NMR Spectrum ($\delta$, $CDCl_3$):
1.39-1.50 (1H, m), 2.90-3.11 (1H, m), 3.12-3.33 (3H, m), 3.43 (1/2H, d, J=4Hz), 3.49 (1/2H, d, J=4Hz), 4.31-4.51 (2H, m), 4.86 (1/2H, s), 4.91 (1/2H, s), 5.10-5.50 (1H, m), 6.57 (2H, s), 6.62 (1H, d, J=9Hz), 6.79 (1H, d, J=9Hz), 7.14-7.24 (5H, m)

EXAMPLE 56

1-(carboxymethyl)-3-(1-mercapto-3-phenyl-2-propyl)-5-(4-methoxyphenyl)hydantoin

The same procedure as in Example 26 was repeated except that N-[1-ethoxycarbonyl-1-(4-methoxyphenyl)methyl]-glycine t-butyl ester was used as the amino acid derivative, thereby the title compound was obtained.

| Elemental analysis for $C_{21}H_{22}N_2O_5S$: | | | | |
|---|---|---|---|---|
| | C % | H % | N % | S % |
| Calcd. | 60.86 | 5.35 | 6.76 | 7.74 |
| Found | 60.59 | 5.43 | 6.93 | 7.65 |

Property: oily
IR Spectrum ($\nu$KBr, $cm^{-1}$): 2568, 1776, 1714
NMR Spectrum ($\delta$, $CDCl_3$):
1.40-1.49 (1H, m), 2.88-3.02 (1H, m), 3.10-3.42 (3H, m), 3.39 (1H, d, J=18Hz), 3.79 (3/2H, s), 3.81 (3/2H, s), 4.32-4.52 (1H, m), 4.47 (1/2H, d, J=18Hz), 4.51 (1/2H, d, J=18Hz), 4.89 (1/2H, s), 4.94 (1/2H, s), 6.72-7.30 (9H, m)

EXAMPLE 57

1-(1-carboxy-2-phenylethyl)-3-(1-mercapto-3-phenyl-2-propyl)-5-phenylhydantoin

The same procedure as in Example 26 was repeated except that N-(1-ethoxycarbonyl-1-phenylmethyl)-phenylalanine t-butyl ester was used as the amino acid derivative, thereby the title compound was obtained.

| Elemental analysis for $C_{27}H_{26}N_2O_4S$: | | | | |
|---|---|---|---|---|
| | C % | H % | N % | S % |
| Calcd. | 68.33 | 5.52 | 5.90 | 6.76 |
| Found | 68.08 | 5.75 | 5.81 | 6.75 |

Property: oily
IR Spectrum ($\nu$KBr, $cm^{-1}$): 2568, 1772, 1714
NMR Spectrum ($\delta$, $CDCl_3$):
1.46 (1H, dd, J=10Hz, J=7Hz), 2.86-3.09 (2H, m), 3.10-3.37 (3H, m), 3.67 (1H, s), 3.73 (1H, s), 3.93-4.04 (1H, m), 4.38-4.52 (1H, m), 6.58 (1H, d, J=7Hz), 6.82 (1H, d, J=7Hz), 7.00-7.09 (3H, m), 7.17-7.33 (10H, m)

EXAMPLE 58

1-(carboxymethyl)-3-(1-mercapto-3-phenyl-2-propyl)-5-[4-(2-propyloxy)phenyl]hydantoin

The same procedure as in Example 26 was repeated except that N-[1-ethoxycarbonyl-1-[4-(2-propyloxy)phenyl]-methyl]glycine t-butyl ester was used as the amino acid derivative, thereby the title compound was obtained.

| Elemental analysis for $C_{23}H_{26}N_2O_5S$: | | | | |
|---|---|---|---|---|
| | C % | H % | N % | S % |
| Calcd. | 62.43 | 5.47 | 6.33 | 7.24 |
| Found | 62.54 | 5.33 | 6.45 | 7.51 |

Property: oily
IR Spectrum ($\nu$KBr, $cm^{-1}$): 2568, 1776, 1716
NMR Spectrum ($\delta$, $CDCl_3$):
1.32 (3H, d, J=6Hz), 1.34 (3H, d, J=6Hz), 1.40-1.50 (1H, m), 2.90-3.01 (1H, m), 3.11-3.35 (3H, m), 3.40 (1H, d, J=18Hz), 4.32-4.57 (3H, m), 4.87 (1/2H, s), 4.92 (1/2H, s), 6.69-6.95 (4H, m), 7.12-7.34 (5H, m)

EXAMPLE 59

1-(1-carboxyethyl)-3-(1-mercapto-3-phenyl-2-propyl)-5-phenylhydantoin

The same procedure as in Example 26 was repeated except that N-(1-ethoxycarbonyl-1-phenylmethyl)-alanine t-butyl ester was used as the amino acid derivative, thereby the title compound was obtained.

| Elemental analysis for $C_{21}H_{22}N_2O_4S$: | | | | |
|---|---|---|---|---|
| | C % | H % | N % | S % |
| Calcd. | 63.30 | 5.57 | 7.03 | 8.05 |
| Found | 63.44 | 5.81 | 7.22 | 8.15 |

Property: oily
IR Spectrum ($\nu$KBr, $cm^{-1}$): 2568, 1772, 1712
NMR Spectrum ($\delta$, $CDCl_3$):
1.09 (3/2H, d, J=7Hz), 1.11 (3/2H, d, J=7Hz), 1.40-1.58 (1H, m), 2.86-3.00 (1H, m), 3.10-3.35 (3H, m), 4.32-4.50 (1H, m), 4.70-4.97 (2H, m), 6.85-7.38 (10H, m)

EXAMPLE 60

1-(carboxymethyl)-5-(9-fluorenyl)-3-(1-mercapto-3-phenyl-2-propyl)hydantoin

(1) 5-(9-fluorenyl)-3-(1-mercapto-3-phenyl-2-propyl)-hydantoin:

The same procedure as in Example 14 was repeated using 1.96 g (7.3 mmol) of N-(9-fluorenyl)glycine methyl ester as the amino acid derivative, thereby the title compound was obtained in a yield of 547 mg (17 %).
NMR Spectrum ($\delta$, $CDCl_3$):
2.27 (1H, s), 2.38 (3H, s), 2.77-2.93 (2H, m), 2.91-3.09 (3H, m), 4.05-4.17 (1H, m), 5.22-5.30 (1H, m), 7.17-7.35 (13H, m)

(2) 1-(carboxymethyl)-5-(9-fluorenyl)-3-(1-mercapto-3-phenyl-2-propyl)hydantoin:

To a solution of 540 mg (1.2 mmol) of 5-(9-fluorenyl)-3-(1-mercapto-3-phenyl-2-propyl)hydantoin in 10 ml of anhydrous acetonitrile was added 200 mg (1.4 mmol) of anhydrous potassium carbonate. To this

mixture was added a solution of 240 mg (1.4 mmol) of t-butyl bromoacetate in 5 ml of anhydrous acetonitrile while cooling with ice, followed by stirring at room temperature for 18 hours. Water was added to the reaction mixture, followed by extraction with ethyl acetate. The organic layer was dried over anhydrous sodium sulfate, and the solvent was removed under reduced pressure. To 15 ml of methanol solution containing 580 mg (1.1 mmol) of the residue thus obtained was added 5 ml of an aqueous solution containing 130 mg (3.2 mmol) of sodium hydroxide, followed by stirring at room temperature for 2 hours. After removing the methanol under reduced pressure, 15 ml of water and 10 ml of 1N HCl were added to the residue, followed by extraction with chloroform. The organic layer was dried over anhydrous sodium sulfate, and the solvent was removed under reduced pressure. The residue thus obtained was purified by silica gel chromatography to give the title compound in a yield of 425 mg (75%).

| Elemental analysis for $C_{27}H_{24}N_2O_4S$: | | | | |
|---|---|---|---|---|
| | C % | H % | N % | S % |
| Calcd. | 68.63 | 5.12 | 5.93 | 6.78 |
| Found | 68.44 | 5.35 | 5.79 | 6.59 |

Property: oily
IR Spectrum ($\nu$KBr, $cm^{-1}$): 2568, 1620
NMR Spectrum ($\delta$, $CDCl_3$):
1.23-1.33 (1H, m), 2.51-2.72 (3H, m), 2.72-2.93 (3H, m), 4.04-4.18 (2H, m), 4.47-4.57 (1H, m), 7.16-7.32 (13H, m)

EXAMPLE 61

1-(carboxymethyl)-5,5-diphenyl-3-(1-mercapto-3-phenyl-2-propyl)hydantoin

The same procedure as in Example 60 was repeated except that diphenylglycine methyl ester was used as the amino acid derivative, thereby the title compound was obtained.

| Elemental analysis for $C_{26}H_{24}N_2O_4S$: | | | | |
|---|---|---|---|---|
| | C % | H % | N % | S % |
| Calcd. | 67.81 | 5.25 | 6.08 | 6.96 |
| Found | 67.77 | 5.44 | 6.01 | 6.88 |

Property: oily
IR Spectrum ($\nu$KBr, $cm^{-1}$): 2568, 1774, 1714
NMR Spectrum ($\delta$, $CDCl_3$):
1.35 (1H, dd, J=10Hz, J=8Hz), 2.92-3.00 (1H, m), 3.10-3.35 (3H, m), 3.87 (1H, d, J=18Hz), 4.13 (1H, d, J=18Hz), 4.42-4.53 (1H, m), 6.63 (2H, d, J=7Hz), 7.13-7.38 (13H, m)

EXAMPLE 62

1-(carboxymethyl)-3-(1-mercapto-2-propyl)hydantoin

The same procedure as in Example 26 was repeated except that 2-(acetylthiomethyl)propanoic acid was used as the carboxylic acid derivative and N-(ethoxycarbonylmethyl)glycine t-butyl ester was used as the amino acid derivative, thereby the title compound was obtained.

| Elemental analysis for $C_8H_{12}N_2O_4S$: | | | | |
|---|---|---|---|---|
| | C % | H % | N % | S % |
| Calcd. | 41.37 | 5.21 | 12.06 | 13.80 |
| Found | 41.55 | 5.13 | 12.31 | 13.74 |

Property: oily
IR Spectrum ($\nu$KBr, $cm^{-1}$): 2988, 1774, 1736
NMR Spectrum ($\delta$, $CDCl_3$):
1.38 (1H, dd, J=10Hz, 8Hz), 1.48 (3H, d, J=7Hz), 2.80-2.88 (1H, m), 3.07-3.19 (1H, m), 4.02 (2H, s), 4.12-4.23 (1H, m), 4.18 (1H, d, J=18Hz), 4.25 (1H, d, J=18Hz)

EXAMPLE 63

1-(carboxymethyl)-3-(1-mercapto-2-propyl)hydantoin-5-spirocyclopentane

The same procedure as in Example 26 was repeated except that 2-(acetylthiomethyl)propanoic acid was used as the carboxylic acid derivative and N-(1-ethoxycarbonylcyclopentyl)glycine t-butyl ester was used as the amino acid derivative, thereby the title compound was obtained.

| Elemental analysis for $C_{12}H_{18}N_2O_4S$: | | | | |
|---|---|---|---|---|
| | C % | H % | N % | S % |
| Calcd. | 50.33 | 6.34 | 9.78 | 11.20 |
| Found | 50.14 | 6.25 | 9.92 | 11.35 |

Melting point: 114.2-115.6 °C
IR Spectrum ($\nu$KBr, $cm^{-1}$): 2980, 1772, 1694
NMR Spectrum ($\delta$, $CDCl_3$):
1.35 (1H, dd, J=10Hz, 8Hz), 1.47 (3H, d, J=7Hz), 1.75-1.94 (6H, m), 2.07-2.16 (2H, m), 2.77-2.86 (1H, m), 3.08-3.19 (1H, m), 4.01 (1H, d, J=18Hz), 4.09 (1H, d, J=18Hz), 4.12-4.21 (1H, m)

EXAMPLE 64

1-(carboxymethyl)-3-(1-mercapto-3-methyl-2-butyl)-hydantoin

The same procedure as in Example 26 was repeated except that 2-(acetylthiomethyl)-3-methyl butanoic acid was used as the carboxylic acid derivative and N-(ethoxycarbonylmethyl)glycine t-butyl ester was used as the amino acid derivative, thereby the title compound was obtained.

| Elemental analysis for $C_{10}H_{16}N_2O_4S$: | | | | |
|---|---|---|---|---|
| | C % | H % | N % | S % |
| Calcd. | 46.14 | 6.20 | 10.76 | 12.32 |
| Found | 46.03 | 6.33 | 10.81 | 12.19 |

Property: oily
IR Spectrum ($\nu$KBr, $cm^{-1}$): 2972, 1770, 1710
NMR Spectrum ($\delta$, $CDCl_3$):
0.89 (3H, d, J=7Hz), 1.01 (3H, d, J=7Hz), 1.29 (1H, dd, J=10Hz, 8Hz), 2.22-2.38 (1H, m), 2.91-3.00 (1H, m), 3.17 (1H, dd, J=11Hz, 10Hz), 3.67-3.75 (1H, m), 4.06 (2H, s), 4.18 (1H, d, J=18Hz), 4.27 (1H, d, J=18Hz)

EXAMPLE 65

1-(carboxymethyl)-3-(1-mercapto-3-methyl-2-butyl)hydantoin-5-spirocyclopentane

The same procedure as in Example 26 was repeated except that 2-(acetylthiomethyl)-3-methylbutanoic acid was used as the carboxylic acid derivative and N-(1-ethoxycarbonylcyclohexyl)glycine t-butyl ester was used as the amino acid derivative, thereby the title compound was obtained.

| Elemental analysis for $C_{14}H_{22}N_2O_4S$: | | | | |
|---|---|---|---|---|
| | C % | H % | N % | S % |
| Calcd. | 53.48 | 7.05 | 8.91 | 10.20 |
| Found | 53.21 | 7.18 | 8.88 | 10.31 |

Property: oily
IR Spectrum ($\nu$KBr, $cm^{-1}$): 2968, 1780, 1692
NMR Spectrum ($\delta$, $CDCl_3$):
0.88 (3H, d, J=7Hz), 1.00 (3H, d, J=7Hz), 1.28 (1H, dd, J=10Hz, 7Hz), 1.77-1.96 (6H, m), 2.09-2.19 (2H, m), 2.22-2.36 (1H, m), 2.91-2.99 (1H, m), 3.12-3.24 (1H, m), 3.64-3.83 (1H, m), 4.02 (1H, d, J=18Hz), 4.10 (1H, d, J=18Hz)

EXAMPLE 66

1-(carboxymethyl)-3-(1-mercapto-4-methyl-2-pentyl)-hydantoin

The same procedure as in Example 26 was repeated except that 2-(acetylthiomethyl)-4-methylpentanoic acid was used as the carboxylic acid derivative and N-(ethoxycarbonylmethyl)glycine t-butyl ester was used as the amino acid derivative, thereby the title compound was obtained.

| Elemental analysis for $C_{11}H_{18}N_2O_4S$: | | | | |
|---|---|---|---|---|
| | C % | H % | N % | S % |
| Calcd. | 48.16 | 6.61 | 10.21 | 11.69 |
| Found | 48.09 | 6.55 | 10.08 | 11.51 |

Property: oily
IR Spectrum ($\nu$KBr, $cm^{-1}$): 2964, 1778, 1704
NMR Spectrum ($\delta$, $CDCl_3$):
0.90-0.93 (6H, m), 1.36 (1H, dd, J=10Hz, 7Hz), 1.44-1.55 (2H, m), 2.01-2.07 (1H, m), 2.75-2.84 (1H, m), 3.05-3.17 (1H, m), 4.03 (2H, s), 4.09-4.20 (1H, m), 4.17 (1H, d, J=18Hz), 4.26 (1H, d, J=18Hz)

EXAMPLE 67

1-(carboxymethyl)-3-(1-mercapto-4-methyl-2-pentyl)hydantoin-5-spirocyclopentane

The same procedure as in Example 26 was repeated except that 2-(acetylthiomethyl)-4-methylpropanoic acid was used as the carboxylic acid derivative and N-(1-ethoxycarbonylcyclopentyl)glycine t-butyl ester was used as the amino acid derivative, thereby the title compound was obtained.

| Elemental analysis for $C_{15}H_{24}N_2O_4S$: | | | | |
|---|---|---|---|---|
| | C % | H % | N % | S % |
| Calcd. | 54.86 | 7.37 | 8.53 | 9.76 |
| Found | 54.77 | 7.09 | 8.41 | 9.61 |

Property: oily
IR Spectrum ($\nu$KBr, $cm^{-1}$): 2964, 1770, 1704
NMR Spectrum ($\delta$, $CDCl_3$):
0.90-0.93 (6H, m), 1.33 (1H, dd, J=10Hz, 8Hz), 1.40-1.55 (2H, m), 1.76-2.15 (9H, m), 2.74-2.83 (1H, m), 3.06-3.14 (1H, m), 4.01 (1H, d, J=18Hz), 4.08 (1H, d, J=18Hz), 4.09-4.18 (1H, m)

EXAMPLE 68

1-(carboxymethyl)-3-[2-(mercaptomethyl)indan-2-yl]-hydantoin-5-spirocyclopentane

(1) 1-(ethoxycarbonylmethyl)-3-[2-(hydroxymethyl)indan-2-yl]hydantoin-5-spiropentane:

To a solution of 9.31 g (5 mmol) of 1-(ethoxycarbonyl)cyclopentane carboxylic acid and 1.38 g (5 mmol) of diphenylphosphorylazide (DPPA) in 30 ml of dry toluene was added 0.7 ml (5 mmol) of triethylamine under nitrogen atmosphere, followed by stirring at room temperature for 30 minutes and then at 80°C for 2 hours. The mixture was cooled to room temperature and 756 mg (5 mmol) of (2-aminoindan-2-yl)methanol was added thereto, followed by stirring for 18 hours. To the reaction mixture was added ethyl acetate, and the resulting mixture was washed with a saturated aqueous solution of sodium hydrogen carbonate and then with a saturated aqueous solution of sodium chloride. The organic layer was dried over anhydrous sodium sulfate, and the solvent was removed under reduced pressure. The residue was dissolved in 50 ml of anhydrous tetrahydrofuran and 66 mg (0.5 mmol) of potassium t-butoxide was added thereto, followed by stirring at room temperature for 2 hours. After removing the tetrahydrofuran under reduced pressure, the residue was dissolved in 50 ml of anhydrous acetonitrile and 835 mg (5 mmol) of ethyl bromoacetate and 829 mg (6 mmol) of potassium carbonate were added thereto, followed by reflux for 18 hours. After filtering off the insoluble materials, the acetonitrile was removed under reduced pressure and 30 ml of water and 10 ml of 1N HCl were added to the residue, followed by extraction with ethyl acetate. The organic layer was washed with a saturated aqueous solution of sodium chloride and dried over anhydrous sodium sulfate. After removing the solvent under reduced pressure, the residue was purified by silica gel chromatography to give the title compound in a yield of 1.02 g (52 %).
NMR Spectrum ($\delta$, $CDCl_3$):
1.29 (3H, t, J=7Hz), 1.69-2.17 (8H, m), 3.15 (1H, t, J=7Hz), 3.42 (2H, d, J=16Hz), 3.59 (2H, d, J=16Hz), 3.76 (2H, d, J=7Hz), 3.99 (2H, s), 4.23 (2H, q, J=7Hz), 7.12-7.24 (4H, m)

(2) 3-[2-(acetylthiomethyl)indan-2-yl]-1-(ethoxycarbonylmethyl)hydantoin-5-spirocyclopentane:

To a solution of 1.36 g (5.2 mmol) of triphenylphosphine in 30 ml of anhydrous tetrahydrofuran was added a solution of 1.05 g (5.2 mmol) of diisopropylazocarboxylate in 5 ml of anhydrous tetrahydrofuran at 0°C under nitrogen atmosphere and the resulting mixture was stirred at 0°C for 30 minutes. To this mixture was added a solution of 1.01 g (2.6 mmol) of 1-(ethoxycarbonylmethyl)3-[2-(hydroxymethyl)indan-2-yl]hydantoin-5-spiropentane and 0.37 ml (5.2 mmol) of thiolacetic acid in 10 ml of anhydrous tetrahydrofuran, followed by stirring at 0°C for 2 hours and then at room temperature for 18 hours. To the reaction mixture was added 100 ml of ethyl acetate, and the organic layer was washed with a saturated aqueous solution of sodium hydrogen carbonate and then a saturated aqueous solution of sodium chloride, and dried over anhydrous sodium sulfate. After removing the solvent under reduced pressure, the residue was purified by silica gel chromatography to give the title compound in a yield of 702 mg (61 %).
NMR Spectrum ($\delta$, $CDCl_3$):
1.29 (3H, t, J=7Hz), 1.67-2.18 (8H, m), 2.32 (3H, s), 3.36 (2H, s), 3.62 (2H, d, J=16Hz), 3.73 (2H, d, J=16Hz), 3.98 (2H, s), 4.23 (2H, q, J=7Hz), 7.15-7.26 (4H, m)

(3) 1-(carboxymethyl)-3-[2-(mercaptomethyl)indan-2-yl]hydantoin-5-spirocyclopentane:

To a solution of 702 mg (1.6 mmol) of 3-[2-(acetylthiomethyl)indan-2-yl]-1-(ethoxycarbonylmethyl)-hydantoin-5-spiropentane in 30 ml of methanol was added 5 ml of 1N NaOH while cooling with ice, followed by stirring at room temperature for 2 hours. After removing the methanol under reduced pressure, 20 ml of water and 10 ml of 1N HCl were added to the residue, followed by extraction with chloroform. The organic layer was washed with a saturated aqueous solution of sodium chloride and dried over anhydrous sodium sulfate. After removing the solvent under reduced pressure, the residue was purified by silica gel column chromatography to give the title compound in a yield of 525 mg (89 %).

| Elemental analysis for $C_{19}H_{22}N_2O_4S$: | | | | |
|---|---|---|---|---|
| | C % | H % | N % | S % |
| Calcd. | 60.94 | 5.92 | 7.48 | 8.56 |
| Found | 60.71 | 6.11 | 7.25 | 8.33 |

Melting point: 169.2-170.1 °C
IR Spectrum (νKBr, $cm^{-1}$): 2576, 1770, 1708
NMR Spectrum (δ, $CDCl_3$):
1.37 (1H, t, J = 9Hz), 1.72-2.00 (6H, m), 2.10-2.22 (2H, m), 2.94 (2H, d, J = 9Hz), 3.65 (2H, d, J = 17Hz), 3.73 (2H, d, J = 17Hz), 4.07 (2H, s), 7.13-7.26 (4H, m)

EXAMPLE 69

1-(carboxymethyl)-3-[2-(mercaptomethyl)indan-2-yl]hydantoin-5-spiro-2'-indane

The same procedure as in Example 68 was repeated except that 2-(ethoxycarbonyl)indane-2-carboxylic acid was used as the carboxylic acid derivative, thereby the title compound was obtained.

| Elemental analysis for $C_{23}H_{22}N_2O_4S$: | | | | |
|---|---|---|---|---|
| | C % | H % | N % | S % |
| Calcd. | 65.39 | 5.25 | 6.63 | 7.59 |
| Found | 65.29 | 5.41 | 6.84 | 7.63 |

Melting point: 173.8-174.8 °C
IR Spectrum (νKBr, $cm^{-1}$): 2576, 1770, 1712
NMR Spectrum (δ, $CDCl_3$):
1.41 (1H, t, J = 9Hz), 2.97 (2H, d, J = 9Hz), 3.25 (2H, d, J = 17Hz), 3.65 (2H, d, J = 17Hz), 3.68 (2H, d, J = 16Hz), 3.77 (2H, d, J = 16Hz), 3.85 (2H, s), 7.15-7.28 (8H, m)

EXAMPLE 70

4-(1-(acetylthio-3-phenyl-2-propyl)-1-cyclopentylurazole

(1) N-(1-acetylthio-3-phenyl-2-propyl)aminocarbonyl-N-cyclopentyl-N'-methoxycarbonylhydrazine:

The same procedure as in Example 1(1) was repeated except that N-cyclopentyl-N'-methoxycarbonylhydrazine was used as the amino acid derivative, thereby the title compound was obtained.
NMR Spectrum (δ, $CDCl_3$):
1.37-1.95 (8H, m), 2.33 (3H, s), 2.76 (1H, dd, J = 14Hz, 8Hz), 2.94-3.06 (3H, m), 3.75 (3H, s), 4.08-4.21 (1H, m), 4.54-4.68 (1H, m), 5.38 (1H, d, J = 6Hz), 6.10 (1H, s), 7.13-7.32 (5H, m)

(2) 4-(1-acetylthio-3-phenyl-2-propyl)-1-cyclopentylurazole:

In 4 ml of o-dichlorobenzene was dissolved 1.23 g (3.1 mmol) of N-(1-acetylthio-3-phenyl-2-propyl)-aminocarbonyl-N-cyclopentyl-N'-methoxycarbonylhydrazine, followed by reflux for 18 hours. After removing the o-dichlorobenzene, the residue was purified by silica gel column chromatography to give the title compound in a yield of 862 mg (76 %).

| Elemental analysis for $C_{18}H_{23}N_3O_3S$: | | | | |
|---|---|---|---|---|
| | C % | H % | N % | S % |
| Calcd. | 59.81 | 6.41 | 11.63 | 8.87 |
| Found | 59.76 | 6.61 | 11.61 | 8.63 |

Melting point: 95.3-97.5 °C
IR Spectrum ($\nu$KBr, $cm^{-1}$): 1766, 1706, 1692
NMR Spectrum ($\delta$, $CDCl_3$):
1.28-1.96 (8H, m), 2.32 (3H, s), 3.13 (1H, dd, J = 15Hz, 6Hz), 3.33 (1H, dd, J = 15Hz, 11Hz), 3.37 (1H, dd, J = 14Hz, 5Hz), 3.51 (1H, dd, J = 14Hz, 10Hz), 4.27-4.43 (2H, m), 7.07 (1H, brs), 7.16-7.39 (5H, m)

EXAMPLE 71

1-cyclopentyl-4-(1-mercapto-3-phenyl-2-propyl)urazole

The same procedure as in Example 15 was repeated using 4-(1-acetyl-3-phenyl-2-propyl)-1-cyclopentylurazole, thereby the title compound was obtained.

| Elemental analysis for $C_{16}H_{21}N_3O_2S$: | | | | |
|---|---|---|---|---|
| | C % | H % | N % | S % |
| Calcd. | 60.16 | 6.63 | 13.16 | 10.04 |
| Found | 60.08 | 6.90 | 12.91 | 9.89 |

Melting point: 116.8-119.0 °C
IR Spectrum ($\nu$KBr, $cm^{-1}$): 1754, 1694
NMR Spectrum ($\delta$, $CDCl_3$):
1.40 (1H, dd, J = 10Hz, 8Hz), 1.40-1.99 (8H, m), 2.87-2.99 (1H, m), 3.10-3.30 (3H, m), 4.20-4.44 (2H, m), 7.05-7.20 (5H, m), 7.63 (1H, brs)

EXAMPLE 72

1-(carboxymethyl)-2-cyclopentyl-4-(1-mercapto-3-phenyl-2-propyl)urazole

To a solution of 361 mg (1 mmol) of 4-(1-acetylthio-3-phenyl-2-propyl)-1-cyclopentylurazole and 166 mg (1.2 mmol) of sodium carbonate in 20 ml of acetonitrile was added 234 mg (1.2 mmol) of ethyl bromoacetate, followed by stirring at room temperature for 18 hours. After filtering off the insoluble materials, the acetonitrile was removed under reduced pressure. To the residue was added 20 ml of water and 2 ml of 1N HCl, followed by extraction with ethyl acetate. The organic layer was washed with a saturated aqueous solution of sodium chloride and then dried over anhydrous sodium sulfate. After removing the solvent under reduced pressure, the residue was dissolved in 20 ml of methanol and 4 ml of 1N NaOH was added thereto while cooling with ice, followed by stirring at room temperature for 2 hours. After removing the methanol under reduced pressure, 20 ml of water and 10 ml of 1N HCl were added to the residue, followed by extraction with chloroform. The organic layer was washed with a saturated aqueous solution of sodium chloride and dried over anhydrous sodium sulfate. After removing the solvent under reduced pressure, the residue was purified by silica gel column chromatography to give the title compound in a yield of 367 mg (97 %).

| Elemental analysis for $C_{18}H_{23}N_3O_4S$: | | | | |
|---|---|---|---|---|
| | C % | H % | N % | S % |
| Calcd. | 57.28 | 6.14 | 11.13 | 8.49 |
| Found | 57.09 | 6.42 | 11.17 | 8.55 |

Property: oily
IR Spectrum (νKBr, $cm^{-1}$): 2568, 1770, 1710
NMR Spectrum (δ, $CDCl_3$):
1.42-1.98 (8H, m), 1.59 (1H, t, J=9Hz), 2.75-2.90 (1H, m), 3.05-3.36 (3H, m), 3.87-4.40 (4H, m), 7.12-7.37 (5H, m)

EXAMPLE 73

4-(1-acetylthio-3-phenyl-2-propyl)-1-phenylurazole

(1) N-(1-acetylthio-3-phenyl-2-propyl)aminocarbonyl-N'-phenylhydrazine:

The same procedure as in Example 1(1) was repeated using phenylhydrazine, thereby the title compound was obtained.
NMR Spectrum (δ, $CDCl_3$):
2.13 (3H, s), 2.80 (1H, dd, J=14Hz, 7Hz), 2.90-3.08 (3H, m), 4.15-4.28 (1H, m), 5.92-6.08 (2H, m), 6.76 (2H, d, J=8Hz), 6.95 (1H, t, J=8Hz), 7.12-7.30 (7H, m)

(2) 4-(1-acetylthio-3-phenyl-2-propyl)-1-phenylurazole:

To a solution of 1.40 g (4 mmol) of N-(1-acetylthio-3-phenyl-2-propyl)aminocarbonyl-N'-phenylhydrazine, 1.55 g (12 mmol) of diisopropylethylamine and 489 mg (4 mmol) of 4-dimethylaminopyridine in 30 ml of toluene solution was added a solution of 1.25 g (8 mmol) phenyl chloroformate in 5 ml of toluene, followed by reflux for 18 hours. After cooling the reaction mixture to room temperature, 50 ml of 1N HCl was added thereto, followed by extraction with ethylacetate. The organic layer was washed with a saturated aqueous solution of sodium chloride and dried over anhydrous sodium sulfate. After removing the solvent under reduced pressure, the residue was purified by silica gel column chromatography to give the title compound in a yield of 423 mg (29 %).

| Elemental analysis for $C_{19}H_{19}N_3O_3S$: | | | | |
|---|---|---|---|---|
| | C % | H % | N % | S % |
| Calcd. | 61.77 | 5.18 | 11.37 | 8.68 |
| Found | 61.55 | 5.43 | 11.29 | 8.53 |

Melting point: 152.0-152.9 °C
IR Spectrum (νKBr, $cm^{-1}$): 1766, 1704, 1692
NMR Spectrum (δ, $CDCl_3$):
2.32 (3H, s), 3.20 (1H, dd, J=14Hz, 6Hz), 3.32-3.44 (2H, m), 3.56 (1H, dd, J=14Hz, 10Hz), 4.41-4.52 (1H, m), 7.17-7.48 (10H, m), 7.70 (1H, brs)

EXAMPLE 74

4-(1-mercapto-3-phenyl-2-propyl)-1-phenylurazole

The same procedure as in Example 15 was repeated using 4-(1-acetylthio-3-phenyl-2-propyl)-1-phenylurazole, thereby the title compound was obtained.

| Elemental analysis for $C_{17}H_{17}N_3O_2S$: | | | | |
|---|---|---|---|---|
| | C % | H % | N % | S % |
| Calcd. | 62.37 | 5.23 | 12.83 | 9.79 |
| Found | 62.21 | 5.44 | 12.76 | 9.71 |

Melting point: 150.1-150.8 °C
IR Spectrum (νKBr, $cm^{-1}$): 2564, 1756, 1686

NMR Spectrum (δ, $CDCl_3$):
1.45 (1H, dd, J=10Hz, 8Hz), 2.91-3.02 (1H, m), 3.17-3.36 (3H, m), 4.30-4.42 (1H, m), 7.17-7.50 (10H, m)

EXAMPLE 75

1-cyclopentyl-3-[1-(2,4,6-trimethylbenzoylthio)-3-phenyl 2-propyl]hydantoin

To a solution of 1.59 g (5 mmol) of 1-cyclopentyl-(1-mercapto-3-phenyl-2-propyl)hydantoin, 0.84 ml (6 mmol) of triethylamine and 61 mg (0.5 mmol) of 4-dimethylaminopyridine (DMAP) in 50 ml of ethyl acetate was added a solution of 931 mg (5 mmol) of 2,4,6-trimethylbenzoylchloride in 5 ml of ethyl acetate while cooling with ice, followed by stirring at room temperature for 2 hours. To the reaction mixture were added 50 ml of water and 10 ml of 1N HCl, followed by extraction with ethyl acetate. The organic layer was washed with a saturated aqueous solution of sodium chloride and dried over anhydrous sodium sulfate. After removing the solvent under reduced pressure, the residue was purified by silica gel column chromatography to give the title compound in a yield of 1.97 g (85 %).

| Elemental analysis for $C_{27}H_{32}N_2O_3S$: | | | | |
|---|---|---|---|---|
| | C % | H % | N % | S % |
| Calcd. | 69.80 | 6.94 | 6.03 | 6.90 |
| Found | 69.91 | 6.89 | 6.21 | 6.87 |

Melting point: 88.0-88.8 °C
IR Spectrum (νKBr, $cm^{-1}$): 1770, 1710, 1676
NMR Spectrum (δ, $CDCl_3$):
1.22-1.93 (8H, m), 2.25 (6H, s), 2.26 (3H, s), 3.21 (1H, dd, J=14Hz, 6Hz), 3.33 (1H, dd, J=14Hz, 10Hz), 3.47-3.63 (3H, m), 3.74 (1H, dd, J=14Hz, 10Hz), 4.26-4.38 (1H, m), 4.48-4.60 (1H, m), 6.82 (2H, s), 7.17-7.32 (5H, m)

EXAMPLE 76

5,5-bis(phenylmethyl)-1-cyclopentyl-3-(1-mercapto-3-phenyl-2-propyl)hydantoin

(1) 5,5-bis(phenylmethyl)-1-cyclopentyl-3-[1-(2,4,6-trimethylbenzoylthio)-3-phenyl-2-propyl]hydantoin:

Under nitrogen atmosphere, a solution of 465 mg (1 mmol) of 1-cyclopentyl-3-[1-(2,4,6-trimethylbenzoylthio)-3-phenyl-2-propyl]hydantoin in 5 ml of anhydrous dimethylformamide was added to a suspension of 100 mg (2.5 mmol) of a 60 % sodium hydride in 30 ml of anhydrous dimethylformamide while cooling with ice and the resulting mixture was stirred for 30 minutes. To this mixture was added 513 mg (3 mmol) of benzylbromide, followed by stirring at room temperature for 3 hours. After removing the dimethylformamide under reduced pressure, ethyl acetate was added to the residue and the organic layer was washed with 1N HCl and then a saturated aqueous solution of sodium chloride and dried over anhydrous sodium sulfate. After removing the solvent under reduced pressure, the residue was purified by silica gel column chromatography to give the title compound in a yield of 534 mg (83 %).
NMR Spectrum (δ, $CDCl_3$):
1.42-2.09 (8H, m), 2.25 (6H, s), 2.26 (3H, s), 2.53-2.84 (3H, m), 2.93-3.43 (5H, m), 3,51-3.64 (1H, m), 4.31-4.43 (1H, m), 6.81 (2H, s), 6.95-7.37 (15H, m)

(2) 5,5-bis(phenylmethyl)-1-cyclopentyl-3-(1-mercapto-3-phenyl-2-propyl)hydantoin:

To a solution of 322 mg (0.5 mmol) of 5,5-bis(phenylmethyl)-1-cyclopentyl-3-[1-(2,4,6-trimethylbenzoylthio)-3-phenyl-2-propyl]hydantoin in 15 ml of ethanol was added 2 ml of 10N NaOH, followed by reflux for 2 hours. After removing the ethanol under reduced pressure, 30 ml of 1N HCl was added to the residue, followed by extraction with ethyl acetate. The organic layer was washed with a saturated aqueous solution of sodium chloride and dried over anhydrous sodium sulfate. After removing the solvent under reduced pressure, the residue was purified by silica gel column chromatography to give the title compound in a yield of 127 mg (51 %).

| Elemental analysis for $C_{31}H_{34}N_2O_2S$: | | | | |
|---|---|---|---|---|
| | C % | H % | N % | S % |
| Calcd. | 74.66 | 6.87 | 5.62 | 6.43 |
| Found | 74.51 | 6.99 | 5.41 | 6.55 |

Melting point: 158.0-159.8 °C
IR Spectrum ($\nu$KBr, $cm^{-1}$): 2568, 1762, 1706
$^1$H-NMR Spectrum ($\delta$, $CDCl_3$):
0.76 (1H, t, J = 8Hz), 1.78-2.20 (8H, m), 2.42-2.49 (1H, m), 2.55 (1H, dd, J = 14Hz, 8Hz), 2.72 (1H, d, J = 14Hz), 2.76-2.87 (1H, m), 2.87 (1H, d, J = 14Hz), 2.94 (1H, dd, J = 14Hz, 8Hz), 3.08 (1H, d, J = 14Hz), 3.16 (1H, d, J = 14Hz), 3.61-3.71 (1H, m), 4.05-4.13 (1H, m), 7.06-7.28 (15H, m)

EXAMPLE 77

1-cyclopentyl-5,5-dimethyl-3-(1-mercapto-3-phenyl-2-propyl) hydantoin

The same procedure as in Example 76 was repeated using methyl iodide as the halogen compound, thereby the title compound was obtained.

| Elemental analysis for $C_{19}H_{26}N_2O_2S$: | | | | |
|---|---|---|---|---|
| | C % | H % | N % | S % |
| Calcd. | 65.86 | 7.56 | 8.09 | 9.25 |
| Found | 65.66 | 7.71 | 8.18 | 9.31 |

Property: oily
IR Spectrum ($\nu$KBr, $cm^{-1}$): 2568, 1764, 1696
$^1$H-NMR Spectrum ($\delta$, $CDCl_3$):
1.04 (3H, s), 1.20 (3H, s), 1.35 (1H, dd, J = 10, 8Hz), 1.48-2.09 (8H, m), 2.92 (1H, ddd, J = 14Hz, 8Hz, 6Hz), 3.10-3.26 (2H, m), 3.27-3.40 (1H, m), 4.26-4.38 (1H, m), 7.14-7.26 (5H, m)

EXAMPLE 78

1-cyclopentyl-3-(1-mercapto-3-phenyl-2-propyl)hydantoin-5-spirocyclopentane

The same procedure as in Example 76 was repeated using butane 1,4-diiodide as the halogen compound, thereby the title compound was obtained.

| Elemental analysis for $C_{21}H_{28}N_2O_2S$: | | | | |
|---|---|---|---|---|
| | C % | H % | N % | S % |
| Calcd. | 67.71 | 7.58 | 7.52 | 8.61 |
| Found | 67.68 | 7.60 | 7.29 | 8.69 |

Melting point: 104.7-106.2 °C
IR Spectrum ($\nu$KBr, $cm^{-1}$): 2568, 1762, 1696
$^1$H-NMR Spectrum ($\delta$, $CDCl_3$):
1.35 (1H, dd, J = 10Hz, 8Hz), 1.48-2.14 (16H, m), 2.93 (1H, ddd, J = 14Hz, 8Hz, 6Hz), 3.10-3.35 (4H, m), 4.22-4.33 (1H, m), 7.14-7.25 (5H, m)

TEST EXAMPLE 1

Measurement of *in vitro* inhibitory activity on NEP:

NEP was prepared as follows in accordance with the process as described in Peptide, 9, 173 (1988). Kidneys of male SD rats were homogenized with 10 times by volume of a tris/HCl buffer solution (pH 7.0) containing mannitol and magnesium chloride. The homogenate was centrifuged (700 × g) to give a supernatant liquid. To the supernatant liquid was added n-octyl-β-D-glucoside in an amount to give a final concentration of 1 %, followed by stirring at 4°C for 1 hour, thereby solubilizing the enzyme. The solution was dialyzed against 5 mM HEPES/0.1% Triton (pH 7.4). The inner liquid was applied to a DE-52 column (2.5 cm i.d. × 15 cm) equilibrated with 5 mM HEPES/0.1% Triton (pH 7.4). The column was eluted with 500 ml of a NaCl solution having a linear concentration gradient of 0-300 mM. The fractions with the NEP activity were collected, and was used as the enzyme source for the test.

The inhibitory activity on NEP was measured according to the method described in Biochemistry, 20, 4942 (1981). That is, 250 μl of a 50 mM tris/HCl buffer solution (pH 7.4) containing 0.4 mM of glutaryl-Ala-Ala-Phe-4-methoxy-β-naphthylamide, 0.25 unit/ml of leucine aminopeptidase, a predetermined amount of the sample compound and NEP was prepared and the reaction was carried out at 37°C for 1 hour. The amount of 4-methoxy-β-naphthylamide produced by the NEP activity was measured to determine the inhibitory activity. The results are shown in Table 1.

TABLE 1

| Example No. | Name of Compound | Inhibitory activity ($IC_{50}$, M) |
|---|---|---|
| 1 | 1-cyclopentyl-3-(1-mercapto-3-phenyl-2-propyl)hydantoin | $4.5 \times 10^{-7}$ |
| 3 | 1-cyclohexyl-3-(1-mercapto-3-phenyl-2-propyl)hydantoin | $2.5 \times 10^{-7}$ |
| 18 | 5-(carboxymethyl)-1-cyclopentyl-3-(1-mercapto)-3-phenyl-2-propyl)-hydantoin | $2.5 \times 10^{-7}$ |
| 19 | 5-(2-carboxyethyl)-1-cyclopentyl-3-(1-mercapto-3-phenyl-2-propyl)-hydantoin | $1.6 \times 10^{-7}$ |
| 20 | 1-(carboxymethyl)-3-(1-mercapto-3-phenyl-2-propyl)hydantoin | $2.3 \times 10^{-7}$ |
| 21 | 1-[(1S)-1-carboxy-2-methylpropyl]-3-(1-mercapto-3-phenyl-2-propyl)-hydantoin | $5.2 \times 10^{-8}$ |
| 26 | 1-[(1S)-1-carboxyethyl]-3-(1-mercapto-3-phenyl-2-propyl)-hydantoin | $1.7 \times 10^{-7}$ |
| 27 | 1-[(1S)-1-carboxy-2-phenylethyl]-3-(1-mercapto-3-phenyl-2-propyl)-hydantoin | $2.6 \times 10^{-7}$ |
| 28 | 1-[(1S)-1-carboxy-3-methylbutyl]-3-(1-mercapto-3-phenyl-2-propyl)-hydantoin | $2.2 \times 10^{-7}$ |
| 29 | 1-[(1S)-1-carboxy-2-methylbutyl]-3-(1-mercapto-3-phenyl-2-propyl)-hydantoin | $5.6 \times 10^{-8}$ |
| 41 | 1-(carboxymethyl)-3-(1-mercapto-3-phenyl-2-propyl)hydantoin-5-spiro-cyclopentane | $4.6 \times 10^{-7}$ |
| 44 | 1-(carboxymethyl)-3-(1-mercapto-3-phenyl-2-propyl)hydantoin-5-spiro-2'-indane | $9.9 \times 10^{-8}$ |

TABLE 1 (cont'd)

| Example No. | Name of Compound | Inhibitory activity ($IC_{50}$, M) |
|---|---|---|
| 45 | 3-(1-mercapto-3-phenyl-2-propyl)-1-piperizinylhydantoin | $5.9 \times 10^{-8}$ |
| 46 | 3-(1-mercapto-3-phenyl-2-propyl)-1-(4-morpholinyl)hydantoin | $1.1 \times 10^{-7}$ |
| 51 | 1-(carboxymethyl)-5-cyclopentyl-3-(1-mercapto-3-phenyl-2-propyl) hydantoin | $5.6 \times 10^{-7}$ |
| 52 | 1-(carboxymethyl)-5-phenyl-3-(1-mercapto-3-phenyl-2-propyl)-hydantoin | $6.1 \times 10^{-8}$ |
| 53 | 1-(carboxymethyl)-5-(diphenylmethyl)-3-(1-mercapto-3-phenyl-2-propyl)-hydantoin | $2.3 \times 10^{-7}$ |
| 55 | 1-(carboxymethyl)-5-(4-hydroxyphenyl)-3-(1-mercapto-3-phenyl-2-propyl)-hydantoin | $9.0 \times 10^{-8}$ |
| 56 | 1-(carboxymethyl)-3-(1-mercapto-3-phenyl-2-propyl)-5-(4-methoxyphenyl)-hydantoin | $6.5 \times 10^{-9}$ |
| 58 | 1-(carboxymethyl)-3-(1-mercapto-3-phenyl-2-propyl)-5-[4-(2-propyloxy)-phenyl]hydantoin | $2.8 \times 10^{-8}$ |
| 60 | 1-(carboxymethyl)-5-(9-fluoreny)-3-(1-mercapto-3-phenyl-2-propyl)-hydantoin | $1.8 \times 10^{-7}$ |
| 66 | 1-(carboxymethyl)-3-(1-mercapto-4-methyl-2-pentyl)hydantoin | $7.0 \times 10^{-7}$ |
| 67 | 1-(carboxymethyl)-3-(1-mercapto-4-methyl-2-pentyl)hydantoin-5-spirocyclopentane | $1.6 \times 10^{-7}$ |
| 72 | 1-(carboxymethyl)-2-cyclopentyl-4-(1-mercapto-3-phenyl-2-propyl)urazole | $4.0 \times 10^{-7}$ |

TEST EXAMPLE 2

Measurement of *in vitro* inhibitory activity on ACE:

The inhibitory activity on ACE was measured according to the method described in Biochem. Pharmacolo., 20, 1637 (1971). That is, a 100 mM boric acid/sodium carbonate buffer solution (pH 8.4)

containing 2.0 mM of hippuryl-L-histidyl-L-leucine, 0.016 unit/ml of ACE, 0.8 M of NaCl, and a predetermined amount of the sample compound was prepared and the reaction was carried out at 37°C for 30 minutes. The amount of hippuric acid produced by the ACE activity was measured to determine the inhibitory activity. The results are shown in Table 2.

TABLE 2

| Example No. | Name of Compound | Inhibitory activity ($IC_{50}$, M) |
|---|---|---|
| 20 | 1-(carboxymethyl)-3-(1-mercapto-3-phenyl-2-propyl)hydantoin | $5.4 \times 10^{-6}$ |
| 24 | 1-(1-carboxycyclohexyl)-3-(1-mercapto-3-phenyl-2-propyl)hydantoin | $5.2 \times 10^{-6}$ |
| 34 | 1-(1-carboxymethyl)-3-(1-mercapto-3-phenyl-2-propyl)hydantoin-5-spirocyclohexane | $2.0 \times 10^{-7}$ |
| 36 | 1-(carboxymethyl)-5-(1-methylethyl)-3-(1-mercapto-3-phenyl-2-propyl)hydantoin | $3.4 \times 10^{-6}$ |
| 40 | 1-(carboxymethyl)-5,5-dimethyl-3-(1-mercapto-3-phenyl-2-propyl)hydantoin | $2.4 \times 10^{-6}$ |
| 41 | 1-(carboxymethyl)-3-(1-mercapto-3-phenyl-2-propyl)hydantoin-5-spirocyclopentane | $4.6 \times 10^{-7}$ |
| 42 | 5-(2-butyl)-1-(carboxymethyl)-3-(1-mercapto-3-phenyl-2-propyl)hydantoin | $1.6 \times 10^{-6}$ |
| 43 | 1-(carboxymethyl)-5-(1-hydroxyethyl)-3-(1-mercapto-3-phenyl-2-propyl)hydantoin | $1.1 \times 10^{-6}$ |
| 44 | 1-(carboxymethyl)-3-(1-mercapto-3-phenyl-2-propyl)hydantoin-5-spiro-2'-indane | $6.8 \times 10^{-7}$ |
| 50 | 1-(carboxymethyl)-3-(1-mercapto-3-phenyl-2-propyl)hydantoin-5-spirocyclobutane | $3.0 \times 10^{-7}$ |
| 51 | 1-(carboxymethyl)-5-cyclopentyl-3-(1-mercapto-3-phenyl-2-propyl)hydantoin | $3.7 \times 10^{-7}$ |
| 52 | 1-(carboxymethyl)-5-phenyl-3-(1-mercapto-3-phenyl-2-propyl)hydantoin | $4.5 \times 10^{-7}$ |
| 63 | 1-(carboxymethyl)-3-(1-mercapto-2-propyl)hydantoin-5-spirocyclopentane | $1.8 \times 10^{-7}$ |
| 65 | 1-(carboxymethyl)-3-(1-mercapto-3-methyl-2-butyl)hydantoin-5-spirocyclopentane | $1.0 \times 10^{-6}$ |
| 67 | 1-(carboxymethyl)-3-(1-mercapto-4-methyl-2-pentyl)hydantoin-5-spirocyclopentane | $5.1 \times 10^{-7}$ |

While the invention has been described in detail and with reference to specific embodiments thereof, it will be apparent to one skilled in the art that various changes and modifications can be made therein without departing from the spirit and scope thereof.

## Claims

1. A hydantoin derivative represented by formula (I):

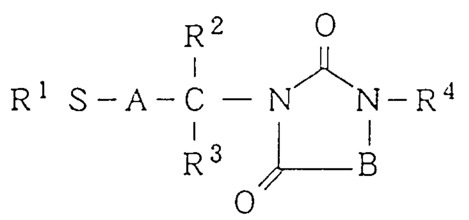

(I)

wherein A represents a $C_{1-10}$ divalent aliphatic hydrocarbon group which may be substituted by an aromatic hydrocarbon group or an aromatic heterocyclic group; B represents $-(CH_2)_n-N(R^5)-$ or $-(CH_2)_n-CR^6(R^7)-$ (in which $R^5$, $R^6$, and $R^7$ each represents a hydrogen atom, an aromatic hydrocarbon group, an aromatic heterocyclic group or a $C_{1-16}$ substituted or unsubstituted monovalent aliphatic hydrocarbon group ($R^6$ and $R^7$ may be combined with each other to form a 4- to 7-membered carbon ring)); n represents 0 or 1; $R^1$ represents a hydrogen atom or a protecting group of the mercapto group; $R^2$ and $R^3$ each represents a hydrogen atom, an aromatic hydrocarbon group, an aromatic heterocyclic group, or a $C_{1-10}$ substituted or unsubstituted monovalent aliphatic hydrocarbon group ($R^2$ and $R^3$ are not hydrogen atoms at the same time and may be combined with each other to form a 4- to 7-membered carbon ring); and $R^4$ represents an aromatic hydrocarbon group, an aromatic heterocyclic group, a $-NR^8R^9$ group (wherein $R^8$ and $R^9$ each represents a hydrogen atom, an aromatic hydrocarbon group, an aromatic heterocyclic group, or a $C_{1-16}$ substituted or unsubstituted monovalent aliphatic hydrocarbon group, or $R^8$ and $R^9$ may be combined with each other to form a 4- to 7-membered ring), or a $C_{1-16}$ substituted or unsubstituted monovalent aliphatic hydrocarbon group, or a pharmaceutically acceptable salt thereof.

2. The hydantoin derivative or the salt thereof of claim 1, wherein A represents a $C_{1-6}$ divalent aliphatic hydrocarbon group.

3. The hydantoin derivative or the salt thereof of claim 1, wherein $R^2$ and $R^3$ each represents a hydrogen atom, an aromatic hydrocarbon group or an aromatic heterocyclic group, provided that $R^2$ and $R^3$ are not hydrogen atoms at the same time.

4. The hydantoin derivative or the salt thereof of claim 3, wherein said aromatic hydrocarbon group is selected from phenyl group and naphthyl group, and said aromatic heterocyclic group is selected from furyl group, thienyl group, pyridyl group, indolyl group, quinolyl group, isoquinolyl group and imidazolyl group, each of which may have a substituent selected from the group consisting of a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ each represents a hydrogen atom, a $C_{1-4}$ alkyl group or an aromatic hydrocarbon group).

5. The hydantoin derivative or the salt thereof of claim 1 , wherein $R^2$ and $R^3$ each represents a hydrogen atom or a $C_{1-6}$ substituted or unsubstituted monovalent aliphatic hydrocarbon group, provided that $R^2$ and $R^3$ are not hydrogen atoms at the same time and may be combined with each other to form a 4- to 7-membered carbon ring.

6. The hydantoin derivative or the salt thereof of claim 5, wherein said monovalent aliphatic hydrocarbon group is substituted by an aromatic hydrocarbon group selected from phenyl group and naphthyl group, or an aromatic heterocyclic group selected from furyl group, thienyl group, pyridyl group, indolyl group, quinolyl group, isoquinoly, group and imidazolyl group, each of which may have a substituent

selected from the group consisting of a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ each represents a hydrogen atom, a $C_{1-4}$ alkyl group or an aromatic hydrocarbon group).

**7.** The hydantoin derivative or the salt thereof of claim 1, wherein $R^4$ represents a $-NR^8R^9$ group (wherein $R^8$ and $R^9$ each represents a hydrogen atom, an aromatic hydrocarbon group, an aromatic, heterocyclic group, or a $C_{1-6}$ substituted or unsubstituted monovalent aliphatic hydrocarbon group, or $R^8$ and $R^9$ may be combined with each other to form a 4- to 7-membered ring).

**8.** The hydantoin derivative or the salt thereof of claim 1, wherein $R^4$ represents an aromatic hydrocarbon group or an aromatic heterocyclic group.

**9.** The hydantoin derivative or the salt thereof of claim 8, wherein said aromatic hydrocarbon group is selected from phenyl group and naphthyl group, and said aromatic heterocyclic group is selected from furyl group, thienyl group, pyridyl group, indolyl group, quinolyl group, isoquinolyl group and imidazolyl group, each of which may have a substituent selected from the group consisting of a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ each represents a hydrogen atom, a $C_{1-4}$ alkyl group or an aromatic hydrocarbon group).

**10.** The hydantoin derivative or the salt thereof of claim 1, wherein $R^4$ represents a $C_{1-6}$ substituted or unsubstituted monovalent aliphatic hydrocarbon group.

**11.** The hydantoin derivative or the salt thereof of claim 10, wherein said monovalent aliphatic hydrocarbon group is substituted by an aromatic hydrocarbon group selected from phenyl group and naphthyl group, an aromatic heterocyclic group selected from furyl group, thienyl group, pyridyl group, indolyl group, quinolyl group, isoquinolyl group and imidazolyl group, hydroxyl group, a $C_{1-4}$ alkoxy group, mercapto group, a $C_{1-4}$ alkylthio group, carboxyl group, a $C_{2-5}$ alkoxycarbonyl group, sulfo group, cyano group, $CONR^{11}R^{12}$, or $SO_2NR^{13}N^{14}$ (wherein $R^{11}$, $R^{12}$, $R^{13}$, and $R^{14}$ each represents a hydrogen atom, a $C_{1-4}$ alkyl group, or an aromatic hydrocarbon group).

**12.** The hydantoin derivative or the salt thereof of claim 1, wherein $R^5$, $R^6$, and $R^7$ each represents a hydrogen atom, an aromatic hydrocarbon group or an aromatic heterocyclic group.

**13.** The hydantoin derivative or the salt thereof of claim 12, wherein said aromatic hydrocarbon group is selected from phenyl group and naphthyl group, and said aromatic heterocyclic group is selected from furyl group, thienyl group, pyridyl group, indolyl group, quinolyl group, isoquinolyl group and imidazolyl group, each of which may have a substituent selected from the group consisting of a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ each represents a hydrogen atom, a $C_{1-4}$ alkyl group or an aromatic hydrocarbon group).

**14.** The hydantoin derivative or the salt thereof of claim 1, wherein $R^5$, $R^6$, and $R^7$ each represents a hydrogen atom or a $C_{1-6}$ substituted or unsubstituted monovalent aliphatic hydrocarbon group, provided that $R^6$ and $R^7$ may be combined with each other to form a 4- to 7-membered carbon ring.

**15.** The hydantoin derivative or the salt thereof of claim 14, wherein said monovalent aliphatic hydrocarbon group is substituted by an aromatic hydrocarbon group selected from phenyl group and naphthyl group, an aromatic heterocyclic group selected from furyl group, thienyl group, pyridyl group, indolyl group, quinolyl group, isoquinolyl group and imidazolyl group, hydroxyl group, a $C_{1-4}$ alkoxy group, mercapto group, a $C_{1-4}$ alkylthio group, carboxyl group, a $C_{2-5}$ alkoxycarbonyl group, sulfo group, cyano group, $CONR^{11}R^{12}$, or $SO_2NR^{13}N^{14}$ (wherein $R^{11}$, $R^{12}$, $R^{13}$, and $R^{14}$ each represents a hydrogen atom, a $C_{1-4}$ alkyl group, or an aromatic hydrocarbon group).

**16.** The hydantoin derivative or the salt thereof of claim 2, wherein $R^2$ and $R^3$ each represents a hydrogen atom or a $C_{1-6}$ substituted or unsubstituted monovalent aliphatic hydrocarbon group, provided that $R^2$ and $R^3$ are not hydrogen atoms at the same tame and may be combined with each other to form a 4- to 7-membered carbon ring.

**17.** The hydantoin derivative or the salt thereof of claim 16, wherein said monovalent aliphatic hydrocarbon group is substituted by an aromatic hydrocarbon group selected from phenyl group and naphthyl group, or an aromatic heterocyclic group selected from fury group, thienyl group, pyridyl group, indolyl group, quinolyl group, isoquinolyl group and imidazolyl group, each of which may have a substituent selected from the group consisting of a halogen atom, a $C_{1-4}$ alkyl group, a $C_{2-8}$ alkoxyalkyl group, carboxyl group, a $C_{2-5}$ alkoxycarbonyl group, a trihalomethyl group, cyano group, nitro group, hydroxyl group, formyl group, phenyl group, benzyl group, phenoxy group, benzyloxy group, and $CONR^{11}R^{12}$ (wherein $R^{11}$ and $R^{12}$ each represents a hydrogen atom, a $C_{1-4}$ alkyl group or an aromatic hydrocarbon group).

**18.** The hydantoin derivative or the salt thereof of claim 16, wherein $R^4$ represents a $-NR^8R^9$ group (wherein $R^8$ and $R^9$ each represents a hydrogen atom, an aromatic hydrocarbon group, an aromatic heterocyclic group, or a $C_{1-6}$ substituted or unsubstituted monovalent aliphatic hydrocarbon group, or $R^8$ and $R^9$ may be combined with each other to form a 4- to 7-membered ring).

**19.** The hydantoin derivative or the salt thereof of claim 16, wherein $R^4$ represents a $C_{1-6}$ substituted or unsubstituted monovalent aliphatic hydrocarbon group.

**20.** The hydantoin derivative or the salt thereof of claim 19, wherein said monovalent aliphatic hydrocarbon group is substituted by an aromatic hydrocarbon group selected from phenyl group and naphthyl group, an aromatic heterocyclic group selected from furyl group, thienyl group, pyridyl group, indolyl group, quinolyl group, isoquinolyl group and imidazolyl group, hydroxyl group, a $C_{1-4}$ alkoxy group, mercapto group, a $C_{1-4}$ alkylthio group, carboxyl group, a $C_{2-5}$ alkoxycarbonyl group, sulfo group, cyano group, $CONR^{11}R^{12}$, or $SO_2NR^{13}N^{14}$ (wherein $R^{11}$, $R^{12}$, $R^{13}$, and $R^{14}$ each represents a hydrogen atom, a $C_{1-4}$ alkyl group, or an aromatic hydrocarbon group).

**21.** A pharmaceutical composition which comprises a pharmaceutically acceptable amount of a hydantoin derivative represented by formula (I):

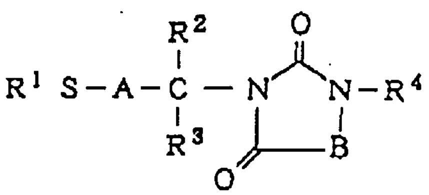

(I)

wherein A represents a $C_{1-10}$ divalent aliphatic hydrocarbon group which may be substituted by an aromatic hydrocarbon group or an aromatic heterocyclic group; B represents $-(CH_2)_n-N(R^5)-$ or $-(CH_2)_n-CR^6(R^7)-$ (in which $R^5$, $R^6$, and $R^7$ each represents a hydrogen atom, an aromatic hydrocarbon group, an aromatic heterocyclic group or a $C_{1-16}$ substituted or unsubstituted monovalent aliphatic hydrocarbon group ($R^6$ and $R^7$ may be combined with each other to form a 4- to 7-membered carbon ring)); n represents 0 or 1; $R^1$ represents a hydrogen atom or a protecting group Of the mercapto group; $R^2$ and $R^3$ each represents a hydrogen atom, an aromatic hydrocarbon group, an aromatic heterocyclic group, or a $C_{1-10}$ substituted or unsubstituted monovalent aliphatic hydrocarbon group ($R^2$ and $R^3$ are not hydrogen atoms at the same time and may be combined with each other to form a 4- to 7-membered carbon ring); and $R^4$ represents an aromatic hydrocarbon group, an aromatic heterocyclic group, a $-NR^8R^9$ group (wherein $R^8$ and $R^9$ each represents a hydrogen atom, an aromatic hydrocarbon group, an aromatic heterocyclic group, or a $C_{1-16}$ substituted or unsubstituted monovalent aliphatic hydrocarbon group, or $R^8$ and $R^9$ may be combined with each other to form a 4- to 7-membered ring), or a $C_{1-16}$ substituted or unsubstituted monovalent aliphatic hydrocarbon group, or a pharmaceutically acceptable salt thereof; and a pharmaceutical acceptable carrier or diluent.

**22.** Use of a hydantoin derivative or the salt thereof of claim 1 for the preparation of a pharmaceutical composition for the treatment of cardiovascular diseases or pain.

European Patent Office

# EUROPEAN SEARCH REPORT

Application Number
EP 94 11 3137

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO-A-92 03410 (SCHERING CORPORATION) 5 March 1992<br>* page 36 - page 40; claims 1-7,11,16 *<br>* page 20; example 2 *<br>--- | 1-22 | C07D233/72<br>A61K31/415<br>A61K31/505<br>A61K31/41<br>C07D233/76 |
| D,A | EP-A-0 052 696 (TANABE SEIYAKU CO., LTD.) 2 June 1982<br>* page 27 - page 29; claims 1-7,10 *<br>* page 2, line 6 - page 3, line 1 *<br>----- | 1-22 | C07D233/78<br>C07D233/80<br>C07D233/90<br>C07D235/02<br>C07D249/12<br>C07D239/22<br>C07D403/06 |

TECHNICAL FIELDS SEARCHED (Int.Cl.6)

C07D

The present search report has been drawn up for all claims

| Place of search | Date of completion of the search | Examiner |
|---|---|---|
| THE HAGUE | 24 October 1994 | Fink, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document